(19) Europäisches Patentamt
European Patent Office
Office européen des brevets

(11) Veröffentlichungsnummer: **0 328 024 B1**

(12) **EUROPÄISCHE PATENTSCHRIFT**

(45) Veröffentlichungstag der Patentschrift: **28.12.94**

(51) Int. Cl.5: **C07D 401/04**, C08K 5/18, G03C 1/06, G03C 1/815

(21) Anmeldenummer: **89102019.0**

(22) Anmeldetag: **06.02.89**

(54) **Tetramethylpiperidino-s-triazine.**

(30) Priorität: **08.02.88 CH 440/88**

(43) Veröffentlichungstag der Anmeldung:
**16.08.89 Patentblatt 89/33**

(45) Bekanntmachung des Hinweises auf die Patenterteilung:
**28.12.94 Patentblatt 94/52**

(84) Benannte Vertragsstaaten:
**AT BE CH DE FR GB IT LI NL SE**

(56) Entgegenhaltungen:
**EP-A- 0 165 608**
**DE-A- 2 025 080**
**DE-A- 2 456 735**
**DE-A- 3 208 570**
**US-A- 3 382 221**
**US-A- 4 629 752**
**US-A- 4 722 806**

**CHEMICAL ABSTRACTS, Band 99, Nr. 6, 8 August 1983, Columbus, Ohio, USA; ASAHI CHEMICAL INDUSTRY CO.,LTD. "S-Triazine compounds." Seite 37, Spalte 1, Zusammenfassung-Nr. 39335g.**

(73) Patentinhaber: **CIBA-GEIGY AG**
**Klybeckstrasse 141**
**CH-4002 Basel (CH)**

(72) Erfinder: **Rody, Jean, Dr.**
**Rütiring 82**
**CH-4125 Riehen (CH)**
Erfinder: **Rytz, Gerhard, Dr.**
**Am Bach 2**
**CH-3150 Schwarzenburg (CH)**
Erfinder: **Slongo, Mario, Dr.**
**Sägetrainweg 553**
**CH-1712 Tafers (CH)**

(74) Vertreter: **Zumstein, Fritz, Dr. et al**
**Patentanwälte,**
**Dr. F. Zumstein,**
**Dipl.-Ing. F. Klingseisen,**
**Bräuhausstrasse 4**
**D-80331 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 328 024 B1

## Beschreibung

Die Erfindung betrifft neue Triazinderivate, die mindestens eine Tetramethylpiperidinogruppe enthalten sowie deren Verwendung als Stabilisatoren für organische Materialien.

Verbindungen, die in ihrem Molekül mindestens eine Triazingruppe und eine 2,2,6,6-Tetramethyl-4-piperidinylgruppe enthalten, sind als Stabilisatoren für organische Materialen, bekannt. Solche Verbindungen enthalten entweder nur eine Triazingruppe, wie z.B. die in der US-A-3,925,376 beschriebenen Verbindungen, oder sie enthalten mehrere Triazingruppen, wie z.B. die in der US-A-4,108,829 beschriebenen Verbindungen. Sie können auch Oligomere oder Polymere mit wiederkehrender Triazingruppe sein, wie z.B. die in der US-A-4,086,204 beschriebenen Verbindungen. In allen diesen bekannten Verbindungen ist der Piperidinrest über seine 4-Stellung mit dem Triazinring verknüpft.

Es wurden nunmehr Verbindungen gefunden, in denen der 2,2,6,6-Tetramethylpiperidinrest über seine 1-Stellung (das Stickstoffatom) an den Triazinring gebunden ist. Zwei solcher Verbindungen sind in der DE-A-2,025,080 beschrieben und wurden dort als Arzneimittel vorgeschlagen. Eine Verwendung als Stabilisatoren ist nicht erwähnt.

Gegenstand der Erfindung sind Verbindungen der Formeln (II) bis (XII) mit Ausnahme von 2-(2,2,6,6-Tetramethyl-1-piperidino)-4,6-bis(2,4,4-trimethyl-2-pentylamino)-triazin und 2,4-Dichlor-6-(2,2,6,6-tetramethyl-1-piperidino)-triazin.

1) Verbindungen der Formel II,

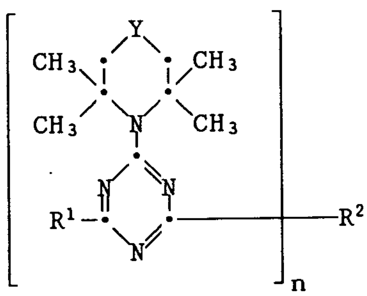

II

worin n eine ganze Zahl von 1 bis 6 bedeutet,
$R^1$ einen Rest der Formel

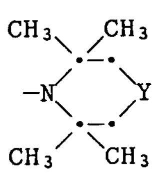

oder Cl, OH, $-OR^3$, $-SR^3$ oder $-NR^4R^5$ bedeutet, wobei $R^3$ $C_1$-$C_{18}$-Alkyl, Allyl, Cyclohexyl, Benzyl, Phenyl oder eine Gruppe der Formel A bedeutet,

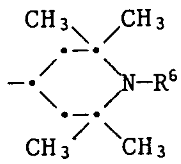

A

$R^4$ Wasserstoff, $C_1$-$C_{12}$-Alkyl, 2-Hydroxyethyl, Allyl, Cyclohexyl, Benzyl oder eine Gruppe der Formel A bedeutet,
$R^5$ $C_1$-$C_{12}$-Alkyl, 2-Hydroxyethyl, Allyl, Cyclohexyl, Benzyl, Phenyl, durch Halogen, $C_1$-$C_4$-Alkyl oder $C_1$-$C_4$-Alkoxy substituiertes Phenyl, eine Gruppe der Formel A oder eine Gruppe der Formel C bedeutet,

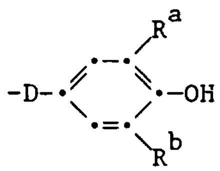

C

worin D durch

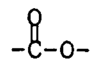

unterbrochenes $C_2$-$C_{20}$-Alkylen bedeutet, $R^a$ und $R^b$ $C_1$-$C_{12}$-Alkyl, $C_5$-$C_6$-Cycloalkyl, $C_6$-$C_{10}$-Aryl oder $C_7$-$C_9$-Phenylalkyl bedeuten und $R^a$ auch Wasserstoff bedeutet, oder
$R^4$ und $R^5$ zusammen $C_4$-$C_8$-Alkylen bedeuten, das durch -O- oder -N($R^8$)-unterbrochen sein kann und worin $R^8$ Wasserstoff, $C_1$-$C_4$-Alkyl oder Acetyl bedeutet,
$R^6$ Wasserstoff, $C_1$-$C_{12}$-Alkyl, $C_7$-$C_9$-Phenylalkyl, $C_3$-$C_5$-Alkenyl, $C_2$-$C_4$-Alkanoyl, $C_3$-$C_5$-Alkenoyl, -O•, -OH oder -O$R^7$ bedeutet und
$R^7$ $C_1$-$C_{18}$-Alkyl, $C_5$-$C_8$-Cycloalkyl, $C_7$-$C_9$-Phenylalkyl, Phenyl, $C_2$-$C_{18}$-Alkanoyl oder Benzoyl bedeutet,
$R^2$, wenn n = 1 ist, Cl, OH, -O$R^3$, -S$R^3$ oder -N$R^4R^5$ bedeutet,
wenn n = 2 ist, eine Gruppe -O-$R^9$-O-, -S-$R^9$-S-, -N($R^{10}$)-$R^9$-N($R^{10}$)-, -O-$R^9$-N($R^{10}$)-,

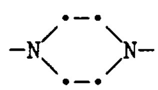

oder -NH-NH- bedeutet, wobei
$R^9$ $C_2$-$C_{20}$-Alkylen, das durch ein oder mehrere -O-, -N($R^8$)- oder -OOC-$R^{17}$-COO- unterbrochen sein kann, $C_4$-$C_8$-Alkenylen, $C_5$-$C_8$-Cycloalkylen, Xylylen, Phenylen oder Tolylen bedeutet,
$R^{10}$ Wasserstoff, $C_1$-$C_{12}$-Alkyl, Allyl, 2-Hydroxyethyl, Benzyl, Phenyl oder eine Gruppe der Formel A bedeutet,
wenn n = 3 ist, eine Gruppe

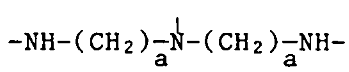

oder

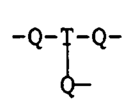

bedeutet, worin
a 2 oder 3 ist, Q -O-, -S- oder -N($R^{10}$)- ist und T $C_3$-$C_{20}$-Alkantriyl oder eine Gruppe

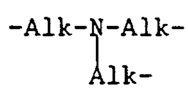

oder

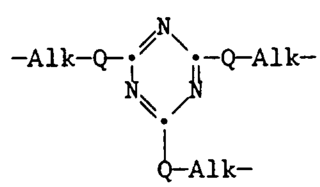

bedeutet, worin Alk eine $C_2$-$C_{12}$-Alkylengruppe ist,
wenn n = 4 ist, eine Gruppe $C(CH_2O-)_4$ oder

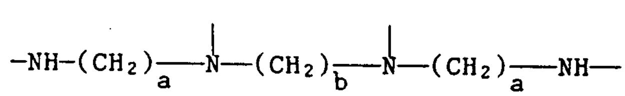

bedeutet, worin
a 2 oder 3 ist und b 2-12 ist,
wenn n = 5 ist, eine Gruppe

$$-NH(CH_2CH_2N)_3CH_2CH_2NH-$$

bedeutet, und wenn n = 6 ist, eine Gruppe

$$-NH(CH_2CH_2N)_4CH_2CH_2NH-$$

bedeutet,
Y eine Gruppe

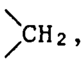 , 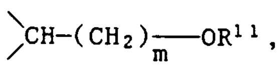 , 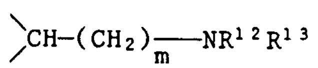 , 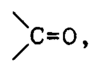 ,

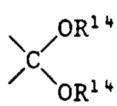 , 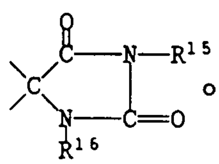 oder 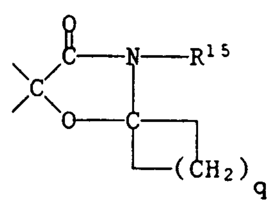

bedeutet,
worin m O, 1 oder 2 ist, q eine ganze Zahl von 5 - 11 ist,
$R^{11}$ Wasserstoff, $C_1$-$C_{18}$-Alkyl, $C_3$-$C_7$-Alkenyl, $C_5$-$C_8$-Cycloalkyl, $C_7$-$C_{11}$-Aralkyl oder eine Gruppe -CO-$R^{18}$ bedeutet,
$R^{12}$ Wasserstoff, $C_1$-$C_{12}$-Alkyl, $C_3$-$C_7$-Alkenyl, $C_5$-$C_8$-Cycloalkyl, $C_7$-$C_{11}$-Aralkyl, $C_2$-$C_4$-Hydroxyalkyl, $C_3$-$C_8$-Alkoxyalkyl, $C_4$-$C_{20}$-Dialkylaminoalkyl, $C_3$-$C_{14}$-Alkoxycarbonylalkyl oder eine Gruppe der Formel A bedeutet,
$R^{13}$ $C_1$-$C_{12}$-Alkyl, $C_2$-$C_4$-Hydroxyalkyl, $C_3$-$C_7$-Alkenyl, $C_5$-$C_8$-Cycloalkyl, Phenyl, durch Halogen, $C_1$-$C_4$-Alkyl oder $C_1$-$C_4$-Alkoxy substituiertes Phenyl, $C_2$-$C_{20}$-Alkanoyl, $C_3$-$C_8$-Alkenoyl, Benzoyl, Phenylacetyl oder einen Triazinylrest der Formel B

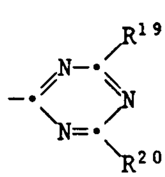 B

bedeutet, oder
$R^{12}$ und $R^{13}$ zusammen $C_4$-$C_8$-Alkylen bedeuten, das durch -O- oder -N($R^8$)-unterbrochen sein kann oder $R^{12}$ und $R^{13}$ zusammen einen Rest der Formel

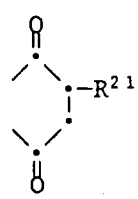

bedeuten, worin $R^{21}$ $C_1$-$C_{18}$-Alkyl bedeutet,

$R^{14}$ $C_1$-$C_{18}$-Alkyl, $C_5$-$C_8$-Cycloalkyl oder $C_7$-$C_9$-Phenylalkyl bedeutet oder beide Gruppen $R^{14}$ zusammen $C_2$-$C_6$-Alkylen, o-Phenylen oder o-Xylylen bedeuten,

$R^{15}$ Wasserstoff, $C_1$-$C_{12}$-Alkyl, $C_3$-$C_5$-Alkenyl, $C_7$-$C_9$-Phenylalkyl, $C_2$-$C_4$-Hydroxyalkyl, $C_3$-$C_8$-Alkoxyalkyl oder $C_3$-$C_{14}$-Alkoxycarbonylalkyl bedeutet,

$R^{16}$ Wasserstoff, $C_1$-$C_{12}$-Alkyl, Allyl oder Benzyl bedeutet,

$R^{17}$ $C_1$-$C_{12}$-Alkylen, Vinylen, Cyclohexylen, Xylylen oder $C_6$-$C_{12}$-Arylen bedeutet,

$R^{18}$ $C_1$-$C_{18}$-Alkyl, $C_2$-$C_6$-Alkenyl, $C_5$-$C_8$-Cycloalkyl, $C_7$-$C_9$-Phenylalkyl, Phenyl oder durch Halogen, Nitro, $C_1$-$C_4$-Alkyl, Hydroxy oder $C_1$-$C_4$-Alkoxy substituiertes Phenyl oder durch Hydroxy und $C_1$-$C_4$-Alkyl substituiertes $C_7$-$C_9$-Phenylalkyl bedeutet,

$R^{19}$ eine der für $R^1$ gegebenen Bedeutungen hat, und

$R^{20}$ Cl, -OH, -$OR^3$, -$SR^3$ oder -$NR^4R^5$ bedeutet.

Soweit irgendwelche Substituenten Alkyl bedeuten, kann dies gerade- oder verzweigtkettiges Alkyl sein. Beispiele hierfür sind Methyl, Ethyl, n-Propyl, iso-Propyl, n-Butyl, sec.Butyl, tert.Butyl, iso-Pentyl, n-Hexyl, n-Heptyl, n-Octyl, 2-Ethylhexyl, iso-Decyl, n-Dodecyl, n-Hexadecyl oder n-Octadecyl. $R^{12}$, $R^{13}$ und $R^{15}$ in der Bedeutung von Hydroxyalkyl können z.B. 2-Hydroxypropyl, 3-Hydroxypropyl oder 2-Hydroxybutyl, insbesondere aber 2-Hydroxyethyl sein.

$R^{12}$ und $R^{15}$ als Alkoxyalkyl können z.B. 2-Methoxyethyl, 2-Ethoxyethyl, 2-Methoxypropyl, 2-Butoxypropyl oder 2-Hexyloxyethyl sein. $R^{12}$ als Dialkylaminoalkyl kann z.B. 2-Dimethylaminoethyl, 2-Dibutylaminoethyl, 2-Diethylaminopropyl oder 2-Dihexylaminoethyl sein.

$R^{18}$ als $C_2$-$C_8$-Alkenyl kann insbesondere Vinyl oder 2-Propenyl sein. $R^6$, $R^{11}$, $R^{12}$, $R^{13}$ und $R^{15}$ als Alkenyl können insbesondere Allyl oder Methallyl sein.

$R^6$, $R^7$, $R^{14}$, $R^{15}$, $R^{17}$ und $R^{18}$ als $C_7$-$C_9$-Phenylalkyl können insbesondere Benzyl oder Phenylethyl sein. $R^{12}$ als $C_7$-$C_{11}$-Aralkyl kann z.B. Benzyl, Phenylethyl oder Naphthylmethyl sein.

$R^7$, $R^{11}$, $R^{12}$, $R^{13}$, $R^{14}$ und $R^{18}$ als Cycloalkyl können insbesondere Cyclohexyl sein.

$R^6$, $R^7$, $R^{13}$ und $R^{17}$ als Alkanoyl können gerade- oder verzweigtkettig sein. Beispiele hierfür sind Acetyl, Propionyl, Isobutyryl, Hexanoyl, Octanoyl, Lauroyl oder Stearoyl. $R^6$ als Alkenoyl kann insbesondere Acryloyl oder Methacryloyl sein.

$R^9$ als Alkylen kann gerade- oder verzweigtkettig oder durch Heteroatome unterbrochen sein. Beispiele hierfür sind Di-, Tri-, Tetra-, Hexa-, Octa-, Deca-oder Dodecamethylen, 2,2-Dimethyltrimethylen oder 2,2,4-Trimethyltetramethylen, 3-Oxapentamethylen, 3-Azapentamethylen, 3-Methylazapentamethylen, 4-Butylazaheptamethylen oder 3,6-Dioxaoctamethylen.

$R^4$ und $R^5$ Zusammen sowie $R^{12}$ und $R^{13}$ zusammen können $C_4$-$C_8$-Alkylen sein, das durch -O- oder -$N(R^8)$- unterbrochen sein kann. In diesem Fall bilden sie zusammen mit dem N-Atom, an das sie gebunden sind, einen heterocyclischen Ring, der vorzugsweise 5- oder 6-gliedrig ist. Beispiele hierfür sind Pyrrolidin, Piperidin, 2,6-Dimethylpiperidin, Morpholin, Piperazin, 4-Methylpiperazin oder 4-Acetylpiperazin.

$R^9$ als Alkenylen kann insbesondere 2-Butenylen-1,4 sein. $R^9$ als Cycloalkylen kann insbesondere Cyclohexylen-1,4 sein.

Wenn $R^2$ $C_3$-$C_{20}$-Alkantriyl ist, so kann dieser Rest gerade- oder verzweigtkettig sein. Beispielen hierfür sind Propan-1,2,3-triyl, Butan-1,3,4-triyl, Pentan-1,3,5-triyl oder 2-Methylpentan-1,3,5-triyl.

Bevorzugt sind solche Verbindungen der Formel II, worin n eine ganze Zahl von 1-4 ist

$R^1$ ein Rest Cl, -$OR^3$ oder -$NR^4R^5$ ist, worin

$R^3$, $R^4$ und $R^5$ die vorhin gegebenen Bedeutungen haben,

$R^2$, wenn n = 1 ist, Cl, -$OR^3$ oder -$NR^4R^5$ bedeutet,

wenn n = 2 ist, eine Gruppe -$N(R^{10})$-$R^9$-$N(R^{10})$- oder

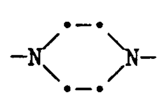

bedeutet, worin $R^9$ und $R^{10}$ die vorhin gegebenen Bedeutungen haben,

wenn n = 3 ist, eine Gruppe

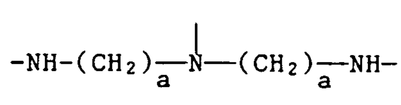

bedeutet und wenn n = 4 ist, eine Gruppe

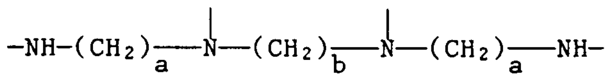

bedeutet, worin a 2 oder 3 und b 2 bis 8 bedeuten und
Y eine Gruppe
$>CH-OR^{11}$, $>CH-NR^{12}R^{13}$, $>C=O$ oder

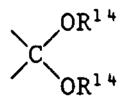

ist,
worin $R^{11}$, $R^{12}$, $R^{13}$ und $R^{14}$ die vorhin gegebenen Bedeutungen haben.

Bevorzugt sind darunter solche Verbindungen der Formel II, worin n = 1 ist und $R^1$ und $R^2$ identisch sind.

Besonders bevorzugt sind Verbindungen der Formel II, worin n = 1 ist, $R^1$ und $R^2$ unabhängig voneinander Cl oder $-NR^4R^5$ bedeuten, $R^4$ Wasserstoff oder $C_1$-$C_{12}$-Alkyl bedeutet, $R^5$ $C_1$-$C_{12}$-Alkyl bedeutet oder $R^4$ und $R^5$ Zusammen Pentamethylen oder 3-Oxapentamethylen bedeuten, und Y eine Gruppe
$>CH_2$, $>CH-OR^{11}$, $>C(OR^{14})_2$,

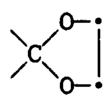 ,

$>C=O$ oder

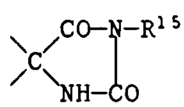

bedeutet,
worin $R^{11}$ Wasserstoff, $C_1$-$C_{12}$-Alkyl oder $-CO-R^{18}$ bedeutet und $R^{18}$ $C_1$-$C_{18}$-Alkyl oder Phenyl ist, $R^{14}$ $C_1$-$C_4$-Alkyl ist und $R^{15}$ Wasserstoff oder $C_1$-$C_{12}$-Alkyl bedeutet.

Zur Herstellung der Verbindungen der Formel II geht man vorteilhaft von Cyanursäurechlorid aus und setzt dieses mit einem Aequivalent eines Tetramethylpiperidins der Formel XIII um.

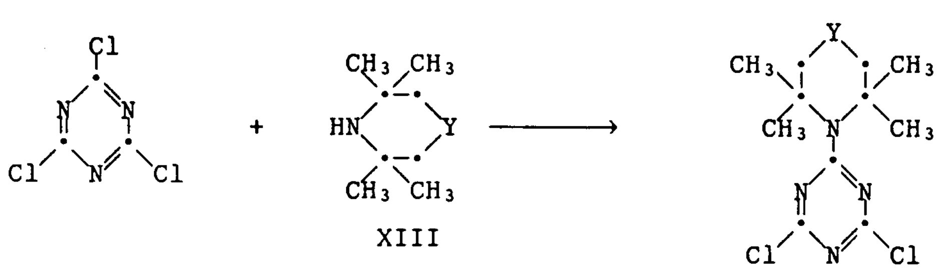

XIII

XIV

Das erhaltene Dichlortriazin XIV kann in einer zweiten Reaktionsstufe mit einem Mol einer Verbindung $R^1H$ umgesetzt werden, wobei man ein Monochlortriazin XV erhält.

XIV + $R^1H$

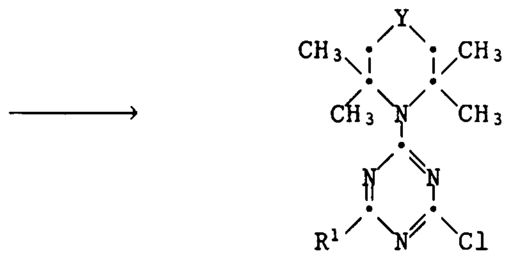

XV

Durch Umsetzung von n Aequivalenten XV mit einer Verbindung $R^2$-$(H)_n$ erhält man in einer dritten Reaktionsstufe die gewünschte Verbindung der Formel II.

Die einzelnen Reaktionsstufen können ohne Isolierung der Zwischenprodukte XIV und XV durchgeführt werden. Alle drei Reaktionsstufen werden bevorzugt in einem inerten Lösungsmittel unter Zusatz von Basen als HCl-Abfänger durchgeführt.

Beispiele für geeignete Lösungsmittel sind Benzol, Toluol oder Xylol. Beispiele für geeignete Basen sind tertiäre Amine wie Tributylamin oder Dimethylanilin oder Alkalimetallhydroxide wie NaOH oder KOH oder Alkalimetallcarbonate wie $Na_2CO_3$ oder $K_2CO_3$. Es kann auch ein Ueberschuss des Piperidins XIII als HCl-Abfänger verwendet werden.

Die Umsetzungen geschehen vorzugsweise unter Erwärmen der Reaktionsgemische. Die Base wird vorzugsweise sukzessive zugegeben in dem Masse wie die Reaktion fortschreitet. Der Fortschritt der Reaktion kann z.B. durch Analyse des gebundenen und/oder des ionisierten Chlor verfolgt werden. Eine andere Möglichkeit der Reaktionskontrolle ist eine chromatographische Analyse des Reaktionsgemisches.

Zur Isolierung des Produktes filtriert man zweckmässig die Basensalze ab oder extrahiert sie mit Wasser und dampft die organische Lösung ein. Will man die Zwischenprodukte isolieren, so geht man in derselben Weise vor.

In alternativer Weise kann das Zwischenprodukt XIV zuerst mit $R^2(H)_n$ und anschliessend mit $R^1H$ umgesetzt werden. Wenn $R^1$ und $R^2$ identisch sind, können die zweite und dritte Reaktionsstufe vereinigt werden, indem man XIV mit 2 Aequivalenten $R^1H$ umsetzt.

Es versteht sich von selbst, dass man in der ersten Stufe eine Verbindung XIII verwendet, deren Gruppe Y gegenüber Cyanursäurechlorid unter den Reaktionsbedingungen inert ist. Vor allem soll Y keine freie OH-, NH- oder SH-Gruppe enthalten. Im Anschluss an die dreistufige Synthese von II kann in einer weiteren Reaktion oder Reaktionssequenz die ursprüngliche (inerte) Gruppe Y gegen eine andere Gruppe Y ausgetauscht werden.

Beispielsweise kann eine ursprüngliche Ketalgruppe

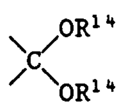

zur Ketogruppe $>C=O$ hydrolisiert werden. Die Ketogruppe lässt sich zur Gruppe $>CH\text{-}OH$ reduzieren, die sich wiederum verethern oder verestern lässt unter Bildung von $>CH\text{-}OR^{11}$.
Die Ketogruppe $>C=O$ kann durch reduktive Aminierung in eine Aminogruppe $>CH\text{-}NHR^{12}$ umgewandelt werden, die anschliessend durch entsprechende N-Substitution in eine Gruppe $>CH\text{-}NR^{12}R^{13}$ übergeführt werden kann.

Die Ketogruppe $>C=O$ kann in die entsprechenden $\alpha$-Hydroxy- oder $\alpha$-Aminonitrile übergeführt werden, die ihrerseits wieder durch Addition von Isocyanaten oder Ketonen in die entsprechenden Spirohydantoine und Spirooxazolidone überführt werden können.

In analoger Weise können andere bekannte Reaktionen zur Umwandlung der Gruppe Y benutzt werden.

Beispiele für einzelne Verbindungen der Formel II sind die folgenden Verbindungen.

| n | $R^1$ | $R^2$ | Y |
|---|---|---|---|
| 1 | 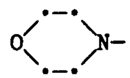 | $-N(C_2H_5)_2$ | $>CH_2$ |
| 1 | $(C_4H_9)_2N-$ | $-N(CH_2CH_2OH)_2$ | $>CH_2$ |
| 1 | 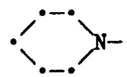 | $= R^1$ | $>CH_2$ |
| 1 | $HOCH_2CH_2NH-$ | $= R^1$ | $>CH_2$ |
| 2 | $C_4H_9NH-$ | $-NH-(CH_2)_6-NH-$ | $>CH_2$ |
| 2 | $HOCH_2CH_2NH-$ | $-NH-(CH_2)_6-NH-$ | $>CH_2$ |
| 2 | $(C_4H_9)_2N-$ | $-NH-CH_2CH_2-NH-$ | $>CH_2$ |
| 3 | $C_4H_9NH-$ | $-NH-CH_2CH_2-\overset{\vert}{N}-CH_2CH_2-NH-$ | $>CH_2$ |
| 1 | $(C_2H_5)_2N-$ | $-NH-$ 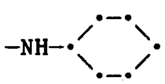 | $>CH-OH$ |
| 1 | $HOCH_2CH_2NH-$ | $= R^1$ | $>CH-OH$ |
| 1 | $(C_4H_9)_2N-$ | $= R^1$ | $>CH-OCOCH_3$ |

| n | $R^1$ | $R^2$ | Y |
|---|---|---|---|
| 1 | $(C_2H_9)(CH_3)N-$ | $= R^1$ | $>CH-OCOC_{11}H_{23}$ |
| 1 | $(C_4H_9)(CH_3)N-$ | $= R^1$ | $>CH-OCH_2CH=CH_2$ |
| 1 | 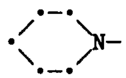 | $-NHC_8H_{17}$ | $>CH-OCH_2$Phenyl |
| 1 | $(CH_3)_2N-$ | $= R^1$ | $>CH-O-C_{12}H_{25}$ |
| 1 | $C_4H_9NH-$ | $= R^1$ | 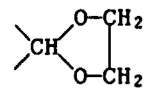 |
| 1 | $(CH_3)_2N-$ | $= R^1$ | 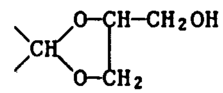 |
| 1 | $C_4H_9NH-$ | $= R^1$ | 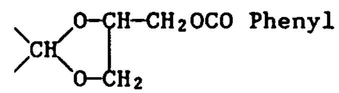 |
| 1 | $(C_2H_5)_2N-$ | $= R^1$ | $>CH-N(C_4H_9)-COCH_3$ |
| 1 | $(CH_3)_2N-$ | $= R^1$ | $>CH-N(C_4H_9)-COOC_2H_5$ |
| 1 | $(C_4H_9)_2N-$ | $= R^1$ | 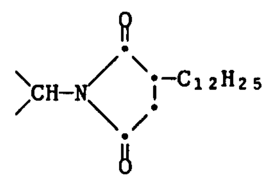 |

| n | $R^1$ | $R^2$ | Y |
|---|---|---|---|
| 1 | $(C_4H_9)(CH_3)N-$ | $= R^1$ | $>CH-N(CH_3)-COC_{11}H_{23}$ |
| 1 | $(C_4H_9)_2N-$ | $= R^1$ | 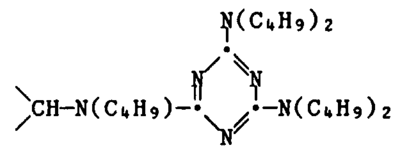 |
| 1 | $C_8H_{17}NH-$ | $= R^1$ | 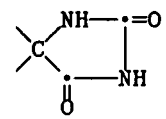 |
| 1 | $(CH_3)_2N-$ | $= R^1$ | 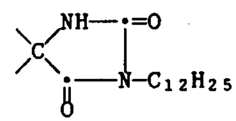 |
| 1 | $(C_4H_9)_2N-$ | $= R^1$ | 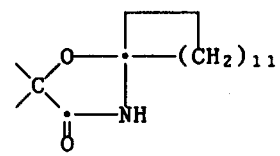 |
| 1 | 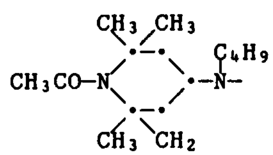 | $= R^1$ | $>CH_2$ |
| 2 | $C_4H_9NH-$ | $-NHCH_2CH_2OOC-(CH_2)_4-COOCH_2CH_2NH-$ | $>CH_2$ |

| n | $R^1$ | $R^2$ | Y |
|---|---|---|---|
| 2 | $(C_4H_9)(CH_3)N-$ | $-NH(CH_2)_3OOC-(CH_2)_8-COO(CH_2)_3NH-$ | 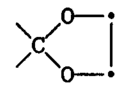 |
| 4 | $C_4H_9NH-$ | $-NH(CH_2)_3-N-CH_2CH_2-N-(CH_2)_3NH-$ | 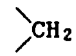 |
| 1 | 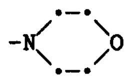 | $= R_1$ | 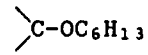 |
| 1 | 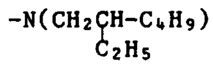 | $= R_1$ | 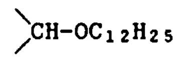 |
| 1 | 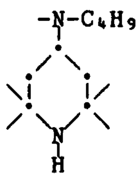 | $= R_1$ | 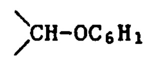 |
| 1 | 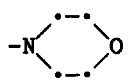 | $= R^1$ | 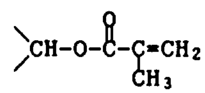 |
| 1 | 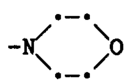 | 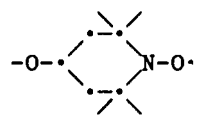 | 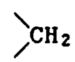 |

| n | $R^1$ | $R^2$ | Y |
|---|---|---|---|
| 2 | 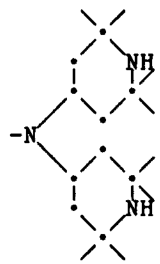 | $-NH-(CH_2)_6-NH-$ | $>CH_2$ |
| 1 | 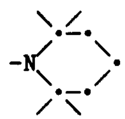 | Cl | $>CH_2$ |

2) Verbindungen der Formel III,

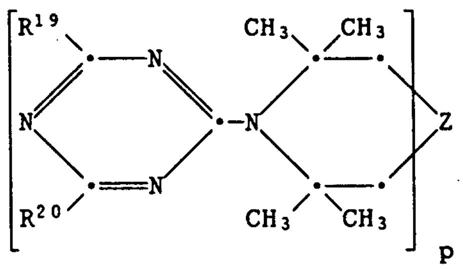

III

worin p 2, 3 oder 4 ist, $R^{19}$ und $R^{20}$ die vorhin gegebenen Bedeutungen haben und Z, wenn p = 2 ist, eine der Gruppen

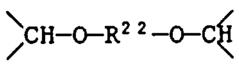 , 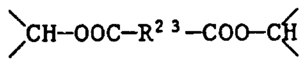 , 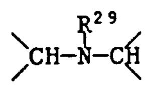 ,

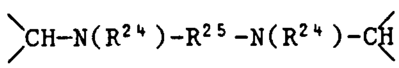 , 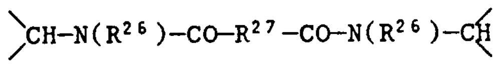 ,

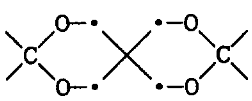 , 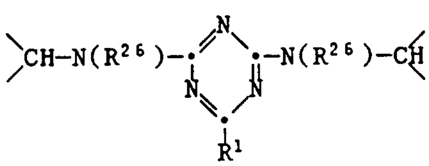 , 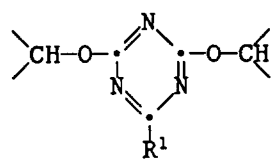 ,

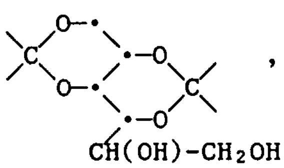 , 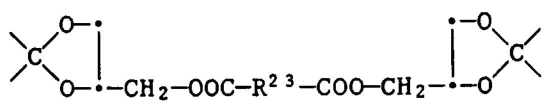

bedeutet, worin
$R^{22}$ $C_2$-$C_{12}$-Alkylen, $C_4$-$C_8$-Alkenylen, Xylylen oder -CO- bedeutet,
$R^{23}$ $C_1$-$C_{12}$-Alkylen, Vinylen, Cyclohexylen, Xylylen, $C_6$-$C_{12}$-Arylen oder durch Halogen, Nitro oder $C_1$-$C_4$-Alkyl substituiertes Phenylen oder eine direkte Bindung bedeutet,
$R^{24}$ Wasserstoff, $C_1$-$C_{12}$-Alkyl, $C_5$-$C_8$-Cycloalkyl, $C_7$-$C_9$-Phenylalkyl, $C_3$-$C_7$-Alkenyl, $C_2$-$C_{18}$-Alkanoyl, $C_3$-$C_7$-Alkenoyl, oder Benzoyl bedeutet,
$R^{25}$ $C_2$-$C_{12}$-Alkylen, durch NH oder O unterbrochenes $C_4$-$C_{16}$-Alkylen, $C_4$-$C_8$-Alkenylen, Xylylen oder Cyclohexylen bedeutet,
$R^{26}$ Wasserstoff, $C_1$-$C_{12}$-Alkyl, $C_5$-$C_8$-Cycloalkyl oder eine Gruppe der Formel A bedeutet,
$R^{27}$ eine der für $R^{23}$ gegebenen Bedeutungen hat oder eine Gruppe -NH-$R^{28}$-NH- ist,
$R^{28}$ $C_2$-$C_{12}$-Alkylen oder $C_6$-$C_{12}$-Arylen bedeutet, das durch $C_1$-$C_4$-Alkyl substituiert sein kann,
$R^{29}$ Wasserstoff, $C_1$-$C_{12}$-Alkyl, $C_2$-$C_{18}$-Alkanoyl oder einen Triazinylrest der Formel B bedeutet, und $R^1$ die eingangs gegebene Bedeutung hat,
und wenn p = 3 ist, eine der Gruppen

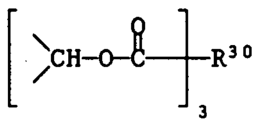 , 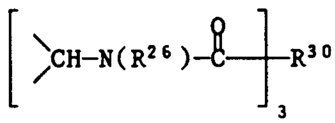 ,

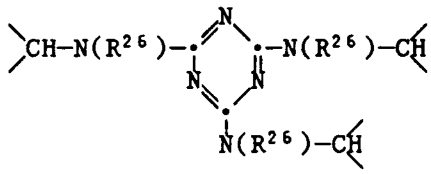

bedeutet, worin $R^{30}$ $C_3$-$C_{18}$-Alkantriyl oder $C_6$-$C_{12}$-Arentriyl bedeutet, und wenn p = 4 ist, eine der Gruppen

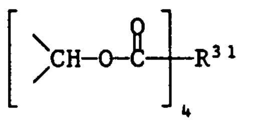 , 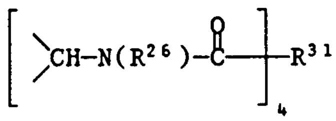

bedeutet, worin $R^{31}$ $C_4$-$C_{16}$-Alkantetrayl oder $C_6$-$C_{12}$-Arentetrayl bedeutet.

Wenn $R^{24}$ , $R^{26}$ oder $R^{29}$ $C_1$-$C_{12}$-Alkyl bedeutet, so kann dies gerade- oder verzweigtkettiges Alkyl sein, wie z.B. Methyl, Ethyl, iso-Propyl, n-Butyl, sec.Butyl, iso-Amyl, n-Hexyl, n-Octyl, 2-Ethylhexyl, n-Decyl oder n-Dodecyl. $R^{24}$ als $C_3$-$C_7$-Alkenyl kann insbesondere Allyl sein.

$R^{24}$ und $R^{26}$ als $C_5$-$C_8$-Cycloalkyl können insbesondere Cyclohexyl sein. $R^{24}$ als $C_7$-$C_9$-Phenylalkyl kann insbesondere Benzyl sein.

$R^{24}$ und $R^{29}$ als $C_2$-$C_{18}$-Alkanoyl können gerade- oder verzweigtkettig sein. Beispiele hierfür sind Acetyl, Propionyl, Butyroyl, Octanoyl, Lauroyl oder Stearoyl. $R^{24}$ als $C_3$-$C_7$-Alkenoyl kann insbesondere Acryloyl oder Nethacryloyl sein.

$R^{22}$, $R^{25}$ und $R^{28}$ als $C_2$-$C_{12}$-Alkylen können gerade- oder verzweigtkettig sein. Beispiele hierfür sind Di-, Tri-, Tetra-, Hexa-, Octa-, Deca- oder Dodecamethylen, 2,2-Dimethyltrimethylen, Diethylmethylen oder 2,2,4-Trimethyl-tetramethylen. $R^{23}$ als $C_1$-$C_{14}$-Alkylen kann darüber hinaus auch z.B. Methylen oder Tetradecamethylen sein. $R^{25}$ als unterbrochenes Alkylen kann z.B. 3-Oxapentamethylen, 3-Azapentamethylen, 3-Methylazapentamethylen oder 4-Oxaheptamethylen sein. $R^{22}$ als $C_4$-$C_8$-Alkenylen kann insbesondere 2-Butenylen-1,4 sein.

$R^{23}$ und $R^{28}$ als $C_6$-$C_{12}$-Arylen können z.B. Phenylen, Diphenylen oder Naphthylen sein.

$R^{30}$ als dreiwertiger Rest kann z.B. Propan-1,2,3-triyl, Butan-1,2,4-triyl, Benzol-1,2,4-triyl oder Naphthalin-1,4,6-triyl sein.

$R^{31}$ als vierwertiger Rest kann z.B. Butan-1,2,3,4-tetrayl, Benzol-1,2,4,5-tetrayl oder Naphthalin-1,4,5,8-tetrayl sein.

Bevorzugt sind Verbindungen der Formel III, worin p 2,3 oder 4 ist, $R^{19}$ und $R^{20}$ unabhängig voneinander, $-OR^3$ oder $-NR^4R^5$ bedeuten, worin $R^3$, $R^4$ und $R^5$ die vorhin gegebenen Bedeutungen haben, Z, wenn p = 2 ist, eine der Gruppen

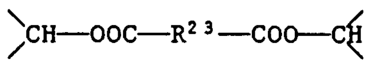 , 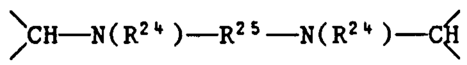 ,

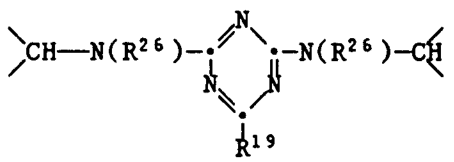 , 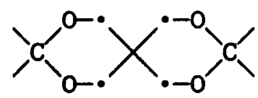 ,

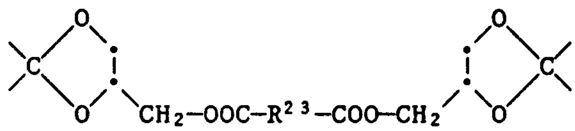

bedeutet, worin $R^{23}$, $R^{24}$, $R^{25}$ und $R^{26}$ die vorhin gegebenen Bedeutungen haben,
wenn p = 3 ist, eine der Gruppen

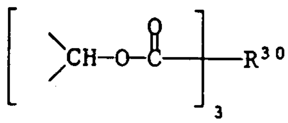 oder 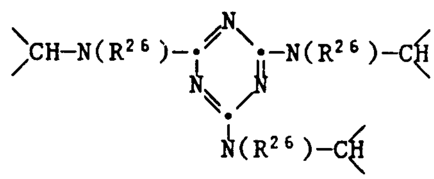

bedeutet, worin $R^{30}$ $C_3$-$C_8$-Alkantriyl oder $C_6$-$C_{12}$-Arentriyl bedeutet und $R^{26}$ die vorhin gegebene Bedeutung hat, und
wenn p = 4 ist, eine Gruppe

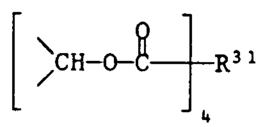

ist, worin $R^{31}$ $C_4$-$C_{12}$-Alkantetrayl oder $C_6$-$C_{12}$-Arentetrayl bedeuten.

Bevorzugt sind darunter solche Verbindungen der Formel III, worin $R^{19}$ und $R^{20}$ identisch sind.

Zur Herstellung der Verbindungen der Formel III kann man in einem ersten Schritt p Aequivalente Cyanursäurechlorid mit einer Bis-, Tris- oder Tetrakispiperidinverbindung XVI umsetzen.

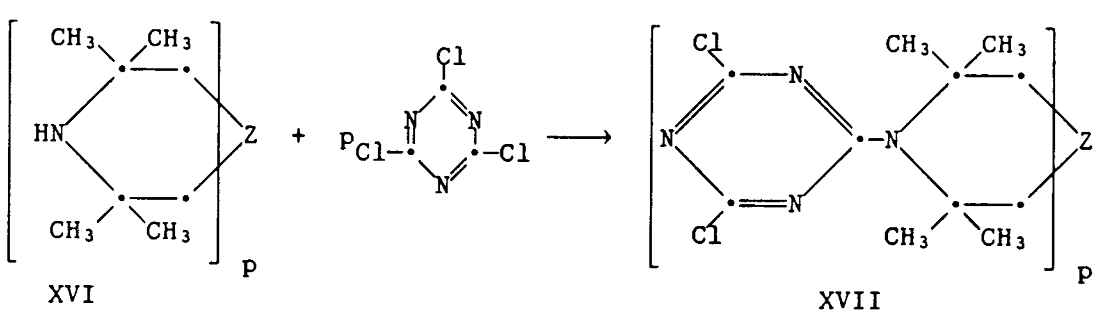

XVI XVII

Das Zwischenprodukt XVII kann dann in zwei weiteren Reaktionsschritten mit $R^{19}H$ und $R^{20}H$ umgesetzt werden. Sind $R^{19}$ und $R^{20}$ identisch, so können sie in einem Schritt eingeführt werden.

Die einzelnen Reaktionsschritte bedeuten eine stufenweise Substitution der drei Chloratome am Triazinring. Sie können ausgeführt werden wie es vorhin für die Synthese von II beschrieben wurde.

Alternativ kann man zuerst eine Verbindung der Formel XVIII herstellen, wie es für die Herstellung von II beschrieben ist, und diese mit zwei-, drei- oder vierwertigen Reagenzien XIX umsetzen.

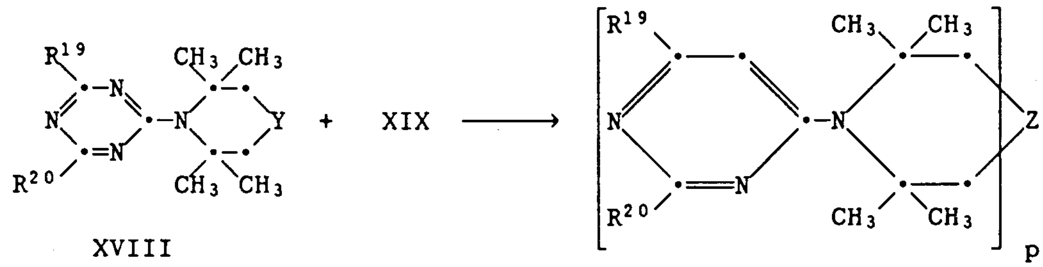


XVIII

III

Ist z.B. Y eine Gruppe >CH-OH, so kann als zweiwertiges XIX eine Verbindung Hal-$R^{22}$-Hal, ClCO-$R^{23}$-COCl, AlkOOC-$R^{23}$-COOAlk, oder

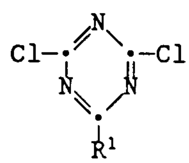

verwendet werden. Als dreiwertiges Reagens XIX kann eine Verbindung $(ClCO)_3R^{30}$, $(AlkOOC)_3R^{30}$ oder Cyanurchlorid verwendet werden, als vierwertiges XIX eine Verbindung $(ClCO)_4$ $R^{31}$ oder $(AlkOOC)_4$ $R^{31}$. Hierin bedeutet Hal ein Halogenatom und Alk eine $C_1$-$C_4$-Alkylgruppe.

Ist Y eine Gruppe >CH-NHR$^{26}$, so kann analog vorgegangen werden. Ist Y eine Gruppe >C=O, so kann diese z.B. mit einem Tetraol unter Bildung eines Bis-ketals umgesetzt werden.

Beispiele für Verbindungen der Formel III sind die folgenden Verbindungen:

| p | $R^{19}$ | $R^{20}$ | Z |
|---|---|---|---|
| 2 | iso-$C_3H_7NH$- | = $R^{19}$ | 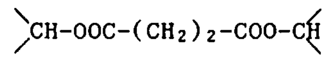 |
| 2 | $C_4H_9NH$- | = $R^{19}$ | 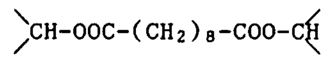 |
| 2 | $(C_2H_5)_2N$- | = $R^{19}$ | 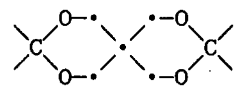 |
| 2 | $(CH_3)_2N$- | = $R^{19}$ | 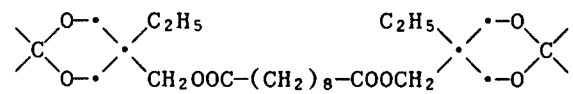 |
| 2 | 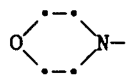 | -$NHC_4H_9$ | 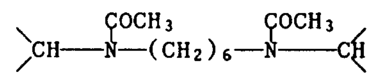 |
| 2 | $C_8H_{17}NH$- | = $R^{19}$ | 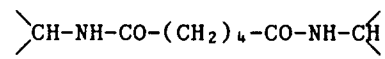 |
| 2 | $C_4H_9NH$- | = $R^{19}$ | 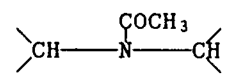 |
| 2 | $(C_2H_5)_2N$- | = $R^{19}$ | 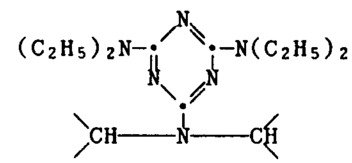 |
| 2 | $(C_4H_9)(CH_3)N$- | = $R^{19}$ | 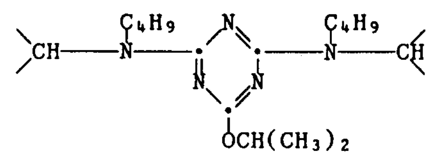 |

| p | $R^{19}$ | $R^{20}$ | Z |
|---|---|---|---|
| 2 | $(C_4H_9)(CH_3)N-$ | $= R^{19}$ | 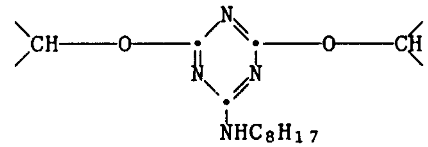 |
| 3 | $(C_4H_9)_2N-$ | $= R^1$ | 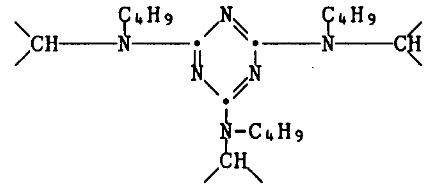 |
| 4 | $C_4H_9NH-$ | $-N(CH_3)_2$ | 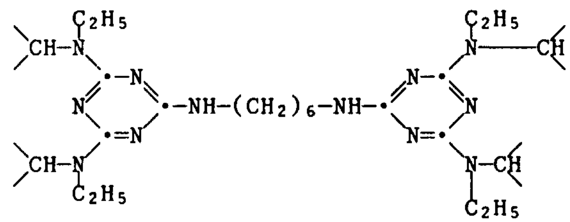 |
| 2 | $(CH_3)_2CHNH-$ | $= R^{19}$ | 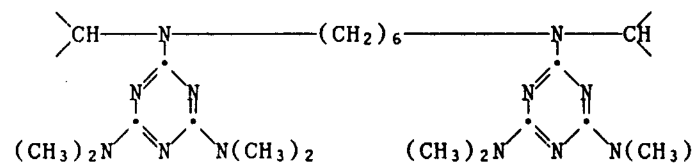 |
| 4 | $C_4H_9O-$ | $= R^{19}$ | 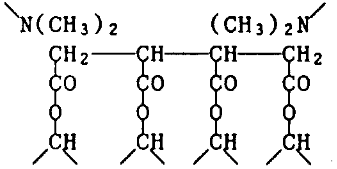 |
| 2 | 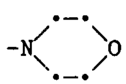 | $= R^{19}$ | 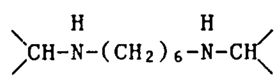 |

3) Verbindungen der Formel IV,

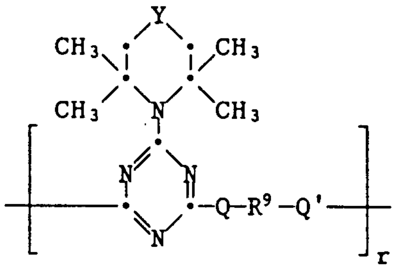

IV

worin r ein Wert von 3 bis 50 ist,
Q und Q' unabhängig voneinander -O-, -S- oder -N($R^{10}$)- bedeuten und Y, $R^9$ und $R^{10}$ die vorhin gegebenen Bedeutungen haben, oder worin -Q-$R^9$-Q'- eine Gruppe -NHNH-,

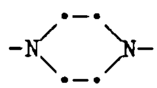

oder

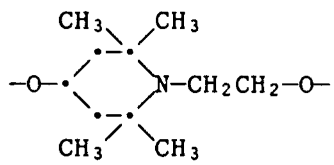

ist.

Bevorzugt ist r ein Wert von 3 bis 25 und Q und Q' -O- oder -N($R^{10}$).

Diese Verbindungen können hergestellt werden durch Umsetzung eines Dichlortriazins XIV mit einer Verbindung HQ-$R^9$-Q'H. Letztere Verbindung kann z.B. ein Diol, ein Dithiol, ein Diamin oder ein Hydroxyamin sein. Je nach dem Molverhältnis der beiden Edukte erhält man Produkte mit hohem oder niedrigem Polykondensationsgrad r. Die Polykondensation wird in Gegenwart von Basen ausgeführt, die das entstehende HCl binden.

Beispiele für Verbindungen der Formel IV sind die folgenden Verbindungen:

| Y | Q | Q' | $R^9$ |
|---|---|---|---|
| $>CH_2$ | NH | NH | $-(CH_2)_6-$ |
| $>CH-OCOCH_3$ | O | O | $-(CH_2)_4-$ |
| 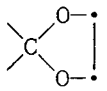 | NH | NH | $-(CH_2)_6-$ |
| $>CH_2$ | NH | NH | $-CH_2CH_2OCC(CH_2)_2COOCH_2CH_2-$ |
| 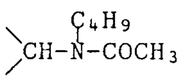 | O | O | 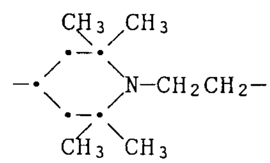 |
| $>CH-OC_4H_9$ | NH | NH | $-CH_2CH_2NHCH_2CH_2-$ |
| 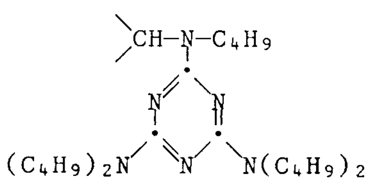 | NH | NH | $-(CH_2)_3-O-(CH_2)_3-$ |

4) Verbindungen der Formel V,

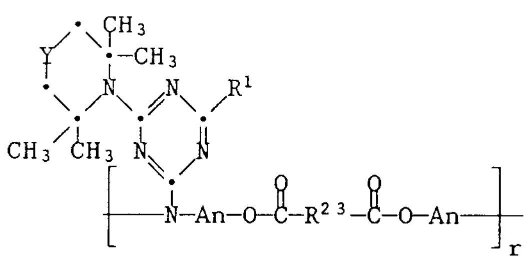

V

worin r ein Wert von 3 bis 50 ist, Y und $R^1$ die vorhin gegebene Bedeutungen haben, An eine $C_2$-$C_4$-Alkylengruppe ist und $R^{23}$ die vorhin gegebene Bedeutung hat.

Diese Verbindungen können hergestellt werden aus einem Monochlortriazin der Formel XV durch Umsetzung mit einer Verbindung $HN(AnOH)_2$ und anschliessende Polykondensation mit einem Dicarbonsäuredialkylester

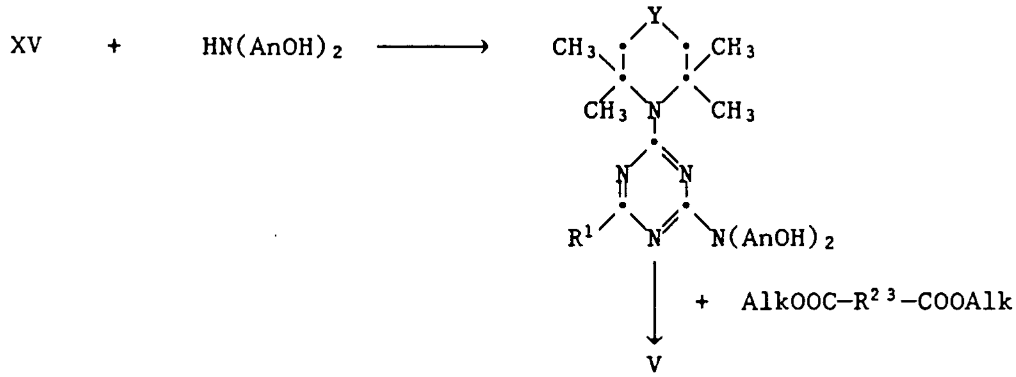


Hierin bedeutet Alk $C_1$-$C_4$-Alkyl. An ist vorzugsweise $-CH_2CH_2-$. Auch hier kann der Polykondensationsgrad r durch Variation des molaren Verhältnisses der Edukte variiert werden. Beispiele für verwendbare Dicarbonsäuredialkylester sind Dimethylsuccinat, Diethyladipat, Dimethylsebacat, Dimethylterephthalat oder Diethylisophthalat.

Beispiele für Verbindungen der Formel V sind die folgenden Verbindungen:

| Y | $R^1$ | An | $R^{23}$ |
|---|---|---|---|
| $>CH_2$ | $-N(C_4H_9)_2$ | $-CH_2CH_2-$ | $-CH_2CH_2-$ |
| $>CH_2$ | 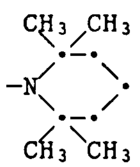 | $-CH_2CH_2-$ | 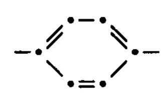 |
| $>C=O$ | $-N(C_2H_5)_2$ | $-CH_2CH_2-$ | $-(CH_2)_4-$ |
| $>CH-OCH_2CH=CH_2$ | $-NHC_4H_9$ | $-CH_2-CH(CH_3)-$ | $-(CH_2)_8$ |
| 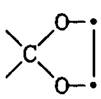 | 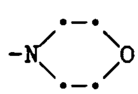 | $-CH_2CH_2-$ | $-CH_2CH_2-$ |

5) Verbindungen der Formel VI,

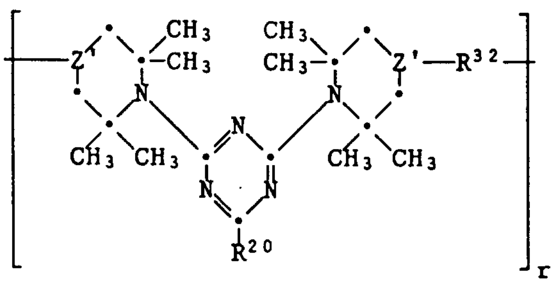

VI

worin r ein Wert von 3 bis 50 ist,

$R^{20}$ die vorhin gegebene Bedeutung hat und

$>Z'-R^{32}-Z'<$ eine der folgenden Gruppen bedeutet

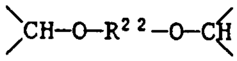 , 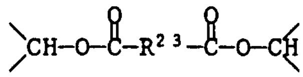 , 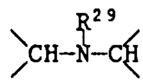 ,

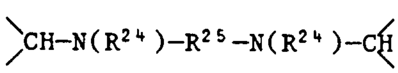 , 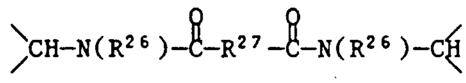

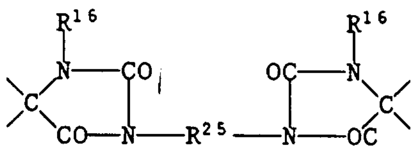

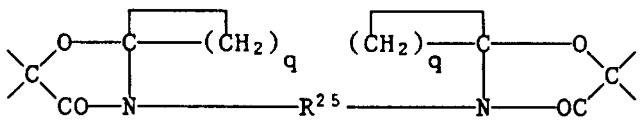

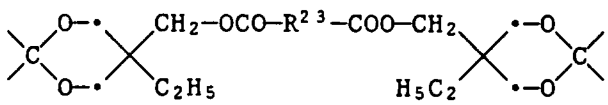

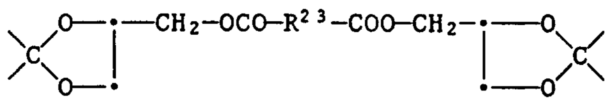

worin q, $R^{16}$, $R^{22}$, $R^{23}$, $R^{24}$, $R^{25}$, $R^{26}$, $R^{27}$ und $R^{29}$ die vorhin gegebene Bedeutung haben.

Diese Verbindungen können hergestellt werden, indem man zuerst durch stufenweise Substitution von Cyanursäurechlorid wie unter (1) beschrieben eine Verbindung der Formel XX herstellt, in der Y eine OH oder NH-Gruppe enthält,

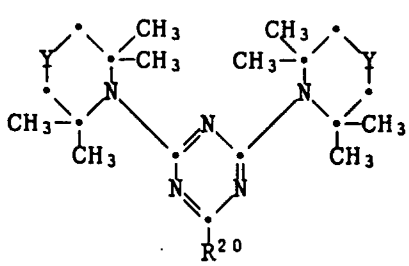

XX

und dieses mit einem difunktionellen Reagens umsetzt. Wenn z.B. Y eine Gruppe $>$CH-OH ist, so kann als difunktionelles Reagens eine Verbindung Hal-$R^{22}$-Hal oder AlkOOC-$R^{23}$-COOAlk verwendet werden.

Alternativ kann man eine Bis-piperidinverbindung der Formel XXI

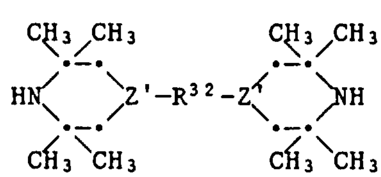

XXI

mit einer Verbindung der Formel XXII

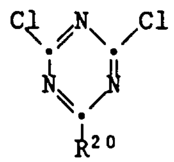

XXII

umsetzen. Oder man setzt eine Verbindung XXI mit einem Aequivalent Cyanursäurechlorid um und führt zum Schluss $R^{20}$ durch Umsetzung mit $R^{20}H$ ein.

Beispiele für Verbindungen der Formel VI sind

| $R^{20}$ | 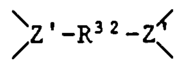 |
|---|---|
| $-N(C_4H_9)_2$ | 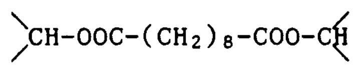 |
| $-OCH(CH_3)_2$ | 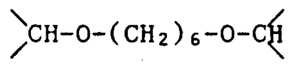 |
| 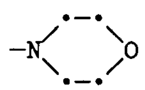 | 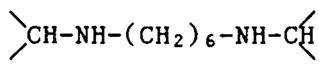 |
| $-N(C_4H_9)_2$ | 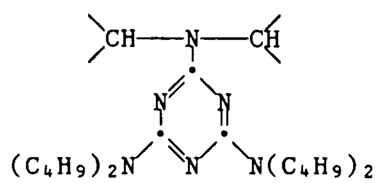 |
| 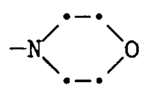 | 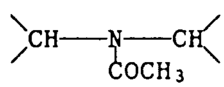 |

6) Verbindungen der Formel VII

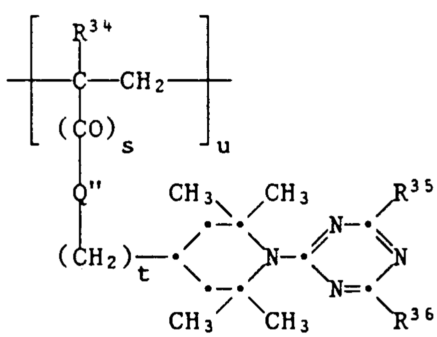

VII

worin s 0 oder 1, t 0 oder 2 und u ein Wert von 5 bis 100 bedeutet, Q'' -O-, -NH- oder -N($C_1$-$C_4$-Alkyl)- bedeutet, $R^{34}$ Wasserstoff oder Methyl ist, $R^{35}$ und $R^{36}$ unabhängig voneinander -$OR^3$, -$SR^3$ oder -$NR^4R^5$ bedeuten, sowie Copolymere einer solchen Verbindung mit (Meth)acrylsäure, Alkyl(meth)acrylaten, Hydroxyalkyl(meth)acrylaten oder Maleinsäureanhydrid.

Solche Verbindungen können durch Polymerisation eines Monomeren der Formel XXIII

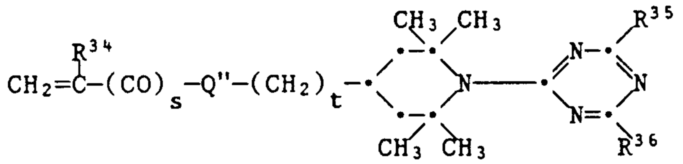

XXIII

oder durch Copolymerisation von XXIII mit (Meth)acrylsäure, einem Alkyl(meth)acrylat, einem Hydroxyalkyl(meth)acrylat oder Maleinsäureanhydrid hergestellt werden. Die Polymerisation geschieht unter Verwendung von radikalischen Polymerisationsinitiatoren, wie z.B. von organi-Peroxiden oder Azoverbindungen. Die Monomeren XXIII können durch stufenweise Substituenten von Cyanurchlorid - wie unter (1) beschrieben - hergestellt werden.

Bevorzugt sind Verbindungen der Formel VII, worin s = 1 ist, t = O ist und Q'' -O- ist.

Beispiele für Verbindungen der Formel VII sind:

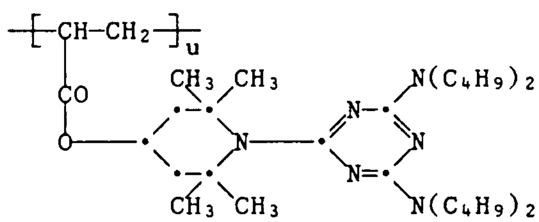

und dessen 1:1-Copolymerisat mit Methylacrylat,

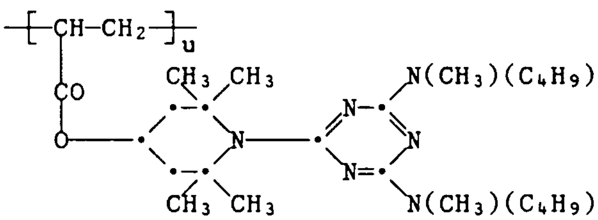

und dessen 1:1-Copolymerisat mit Butylacrylat,

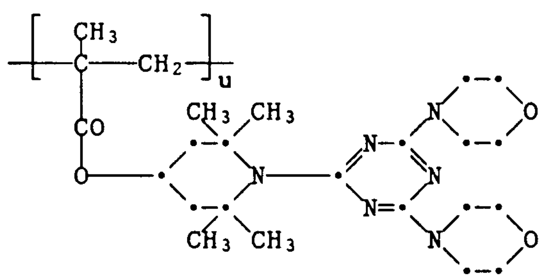

und dessen 1:1-Copolymerisat mit Methylmethacrylat,

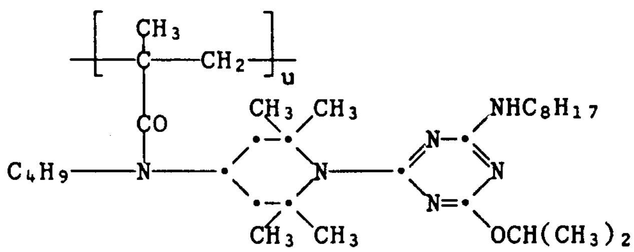

und dessen 1:1-Copolymerisat mit Hydroxyethylacrylat.

7) Verbindungen der Formel VIII,

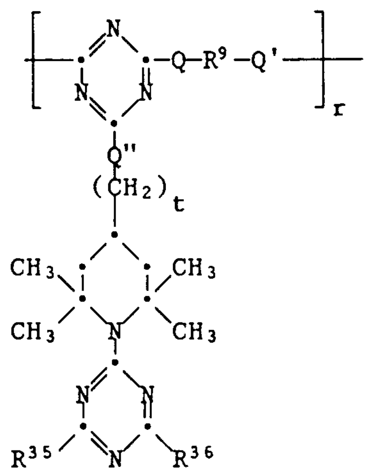

VIII

worin r ein Wert von 3 bis 50 ist, t 0 oder 2 ist,
Q und Q' die vorhin gegebenen Bedeutungen haben,
Q'' -O-, -NH- oder -N($C_1$-$C_4$-Alkyl) bedeutet und
$R^9$, $R^{35}$ und $R^{36}$ die vorhin gegebenen Bedeutungen haben.

Diese Verbindungen können hergestellt werden durch Polykondensation einer Verbindung der Formel XXIV,

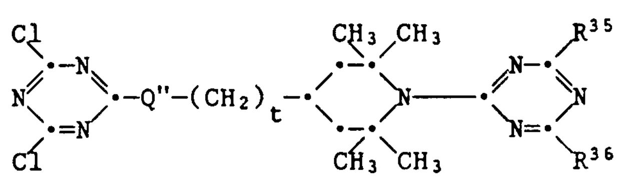

XXIV

mit einer difunktionellen Verbindung HQ-$R^9$-Q'H in Gegenwart von zwei Aequivalenten einer Base. Die Edukte XXIV können durch stufenweise Substitution von Cyanurchlorid - wie unter (1) beschrieben -hergestellt werden.

Bevorzugt sind Verbindungen der Formel VIII, worin t = 0 ist.

Beispiele für Verbindungen der Formel VIII sind die folgenden Verbindungen:

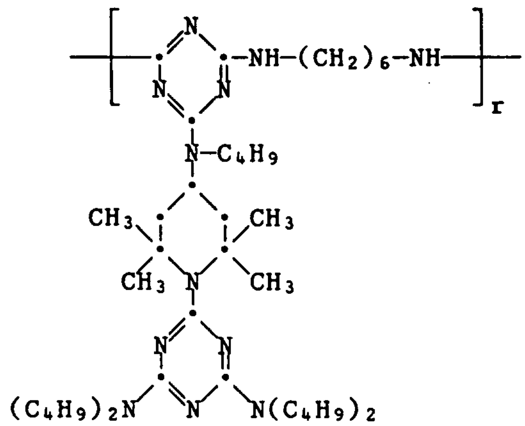

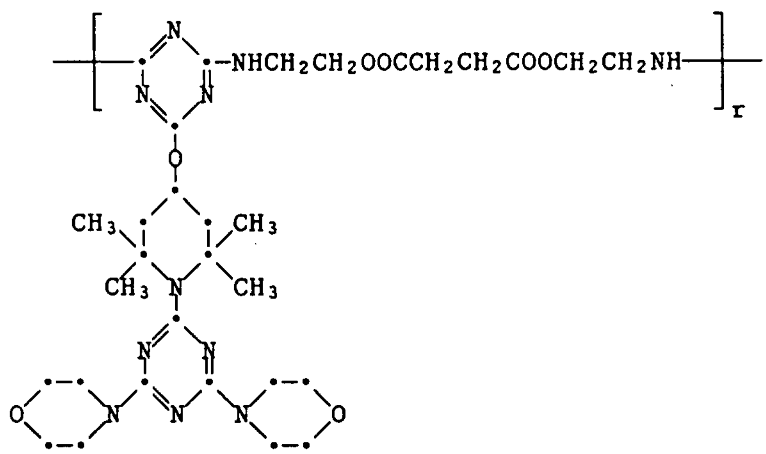

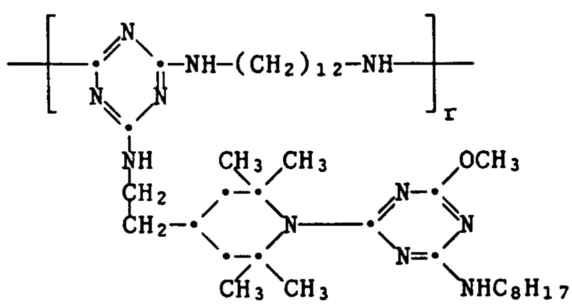

8) Verbindungen der Formel IX,

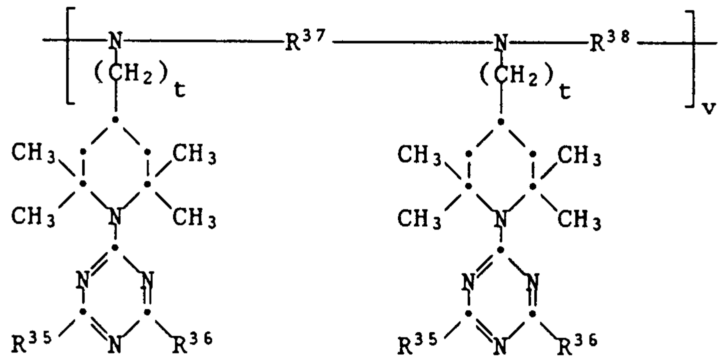

IX

worin v ein Wert von 2 bis 30 ist, t 0 oder 2 ist, $R^{35}$ und $R^{36}$ die vorhin gegebenen Bedeutungen haben, $R^{37}$ $C_2$-$C_8$-Alkylen, $C_4$-$C_8$-Alkenylen, Xylylen, $-CH_2-CH(OH)-CH_2-$ oder $-CH_2CH(OH)CH_2-O-R^{39}-O-CH_2CH-(CH)CH_2-$ bedeutet,
$R^{38}$ eine der für $R^{37}$ gegebenen Bedeutungen hat oder

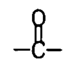

oder

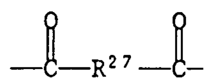

ist,
$R^{39}$ $C_2$-$C_8$-Alkylen, Phenylen oder

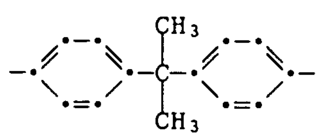

bedeutet
und $R^{27}$ die vorhin gegebene Bedeutung hat.

Diese Verbindungen können hergestellt werden aus einer Verbindung der Formel XXV

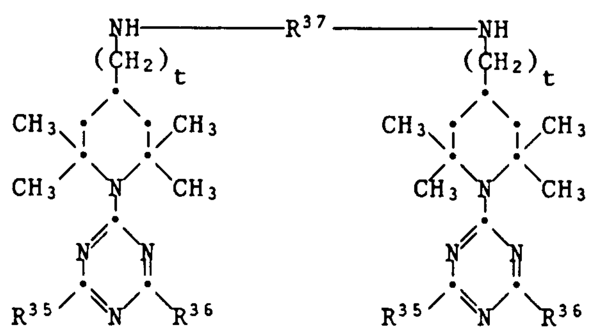

XXV

durch Umsetzung mit einer difunktionellen Verbindung, deren funktionelle Gruppen mit sekundären Aminen reagieren können. Beispiele hierfür sind die Dihalogenide Hal-$R^{38}$-Hal oder Epichlorhydrin oder Diglycidylether der Formel XXVa

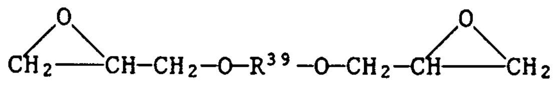

XXVa

Wenn $R^{38}$ gleich $R^{37}$ ist, so können die Verbindungen der Formel IX aus einem primären Amin der Formel XXVI

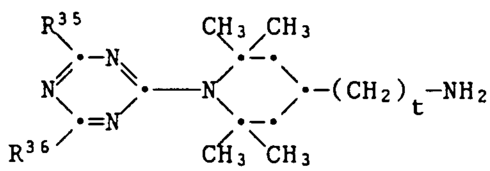

XXVI

durch Umsetzung mit Hal-$R^{37}$-Hal, mit Epichlorhydrin oder mit einem Diglycidylether XXVa hergestellt werden. Die Edukte XXV und XXVI können durch stufenweise Substitution von Cyanurchlorid - wie unter (1) oder (2) beschrieben - hergestellt werden.

Beispiele für Verbindungen der Formel IX sind:

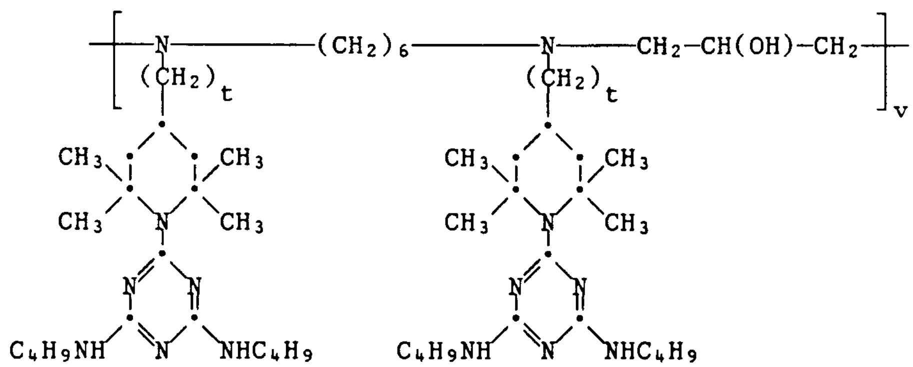

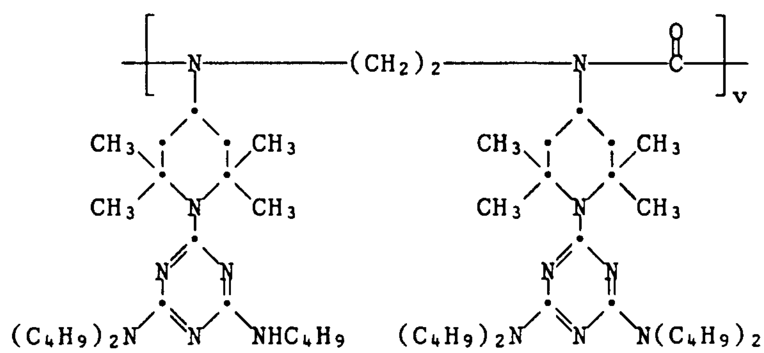

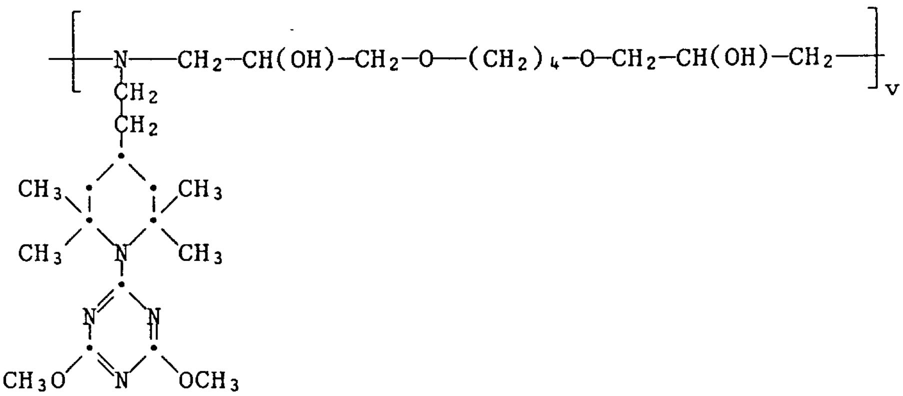

9) Verbindungen der Formel X,

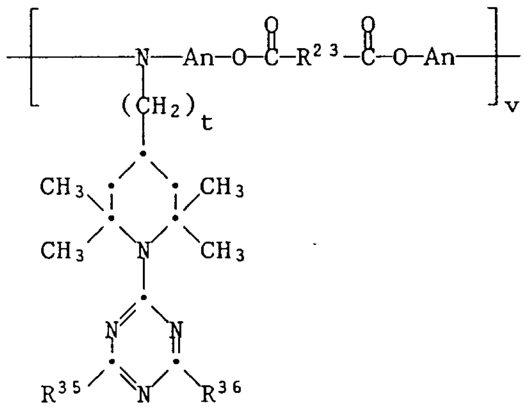

X

worin t 0 oder 2 ist, v ein Wert von 2 bis 30 ist,
An $C_2$-$C_4$-Alkylen bedeutet, $R^{35}$, $R^{36}$ und $R^{23}$ die vorhin gegebenen Bedeutungen haben.

Diese Verbindungen können durch Polykondensation von Verbindungen der Formel XXVII

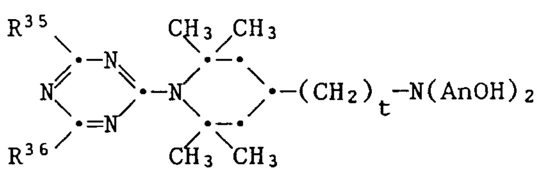

XXVII

mit Dicarbonsäuredialkylestern AlkOOC-$R^{23}$-COOAlk hergestellt werden. Bevorzugt sind die Verbindungen der Formel X, in denen t null ist und An -$CH_2CH_2$- ist. Die Edukte XXVII können durch stufenweise Substitution von Cyanurchlorid - wie in (1) beschrieben - hergestellt werden.

Beispiele für Verbindungen der Formel X sind:

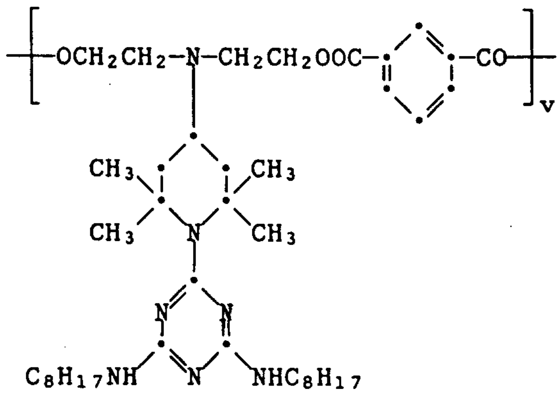

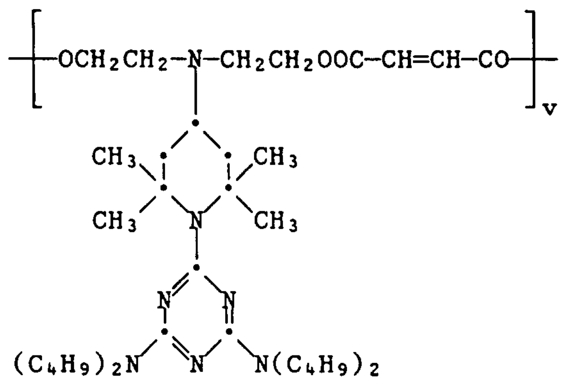

10) Verbindungen der Formel XI,

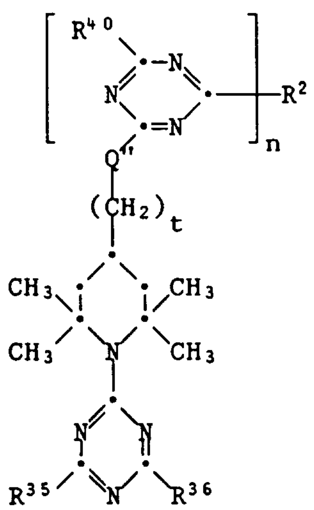

XI

worin n eine ganze Zahl von 1 bis 6 bedeutet, t 0 oder 2 ist, $R^2$, Q'', $R^{35}$ und $R^{36}$ die vorhin gegebenen Bedeutungen haben und $R^{40}$ entweder eine der für $R^1$ gegebenen Bedeutungen hat oder eine Gruppe

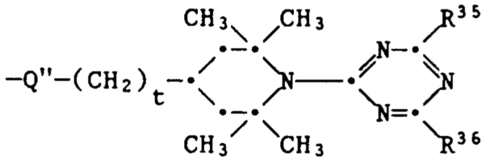

bedeutet.

Zur Herstellung dieser Verbindungen kann man n Aequivalente einer Verbindung der Formel XXVIII

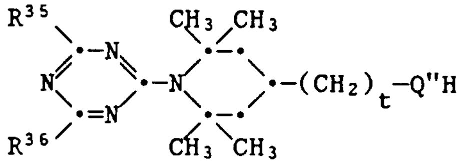

XXVIII

mit einem Aequivalent einer Verbindung der Formel

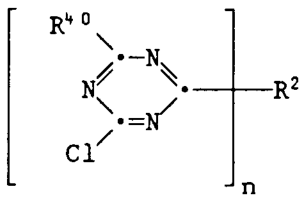

in Gegenwart von n Aequivalenten einer Base umsetzen.

Man kann aber auch zuerst eine Verbindung der Formel XXVIIIa

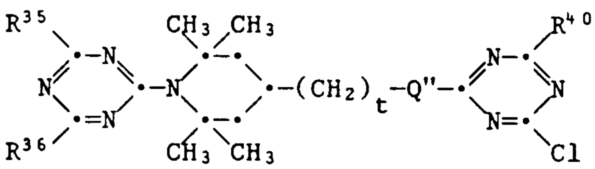

XXVIIIa

herstellen und anschliessend n Aequivalente von XXVIIIa mit einem Aequivalent $R^2(H)_n$ umsetzen.

Die Edukte XXVIII und XXVIIIa können durch stufenweise Substitution von Cyanurchlorid - wie unter (1) beschrieben - hergestellt werden.

Beispiele für Verbindungen der Formel XI sind folgende Verbindungen:

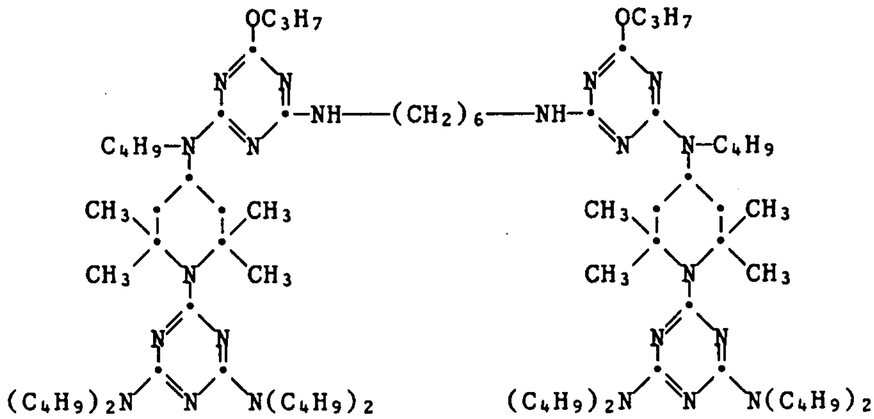

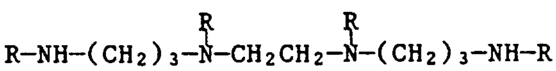

R =

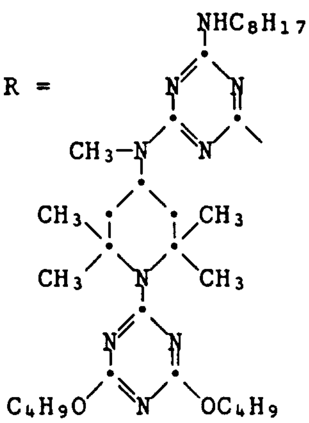

11) Verbindungen der Formel XII,

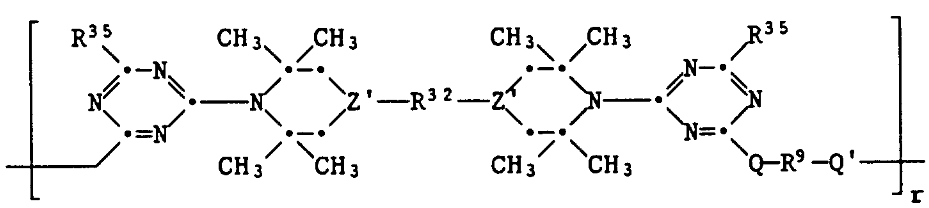

XII

worin r ein Wert von 3 bis 50 ist, $R^9$ und $R^{35}$ die vorhin gegebenen Bedeutungen haben, die Gruppe

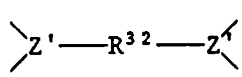

eine der für Klasse 5 gegebenen Bedeutungen hat und Q und Q' unabhängig voneinander -O-, -S- oder -N-($R^{10}$)- bedeuten.

Zur Herstellung dieser Verbindungen setzt man zuerst eine Bis-piperidinverbindung der Formel XXIX

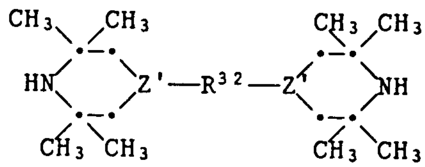

XXIX

mit 2 Aequivalenten Cyanursäurechlorid um, führt dann den Rest $R^{35}$ durch Umsetzung mit 2 Aequivalenten $R^{35}H$ ein und setzt das Produkt mit einer difunktionellen Verbindung $HQ\text{-}R^9\text{-}Q'H$ um. Diese kann z.B. ein Diol, ein Dithiol, ein Diamin oder ein Hydroxyamin sein.

Beispiele für Verbindungen der Formel XII sind:

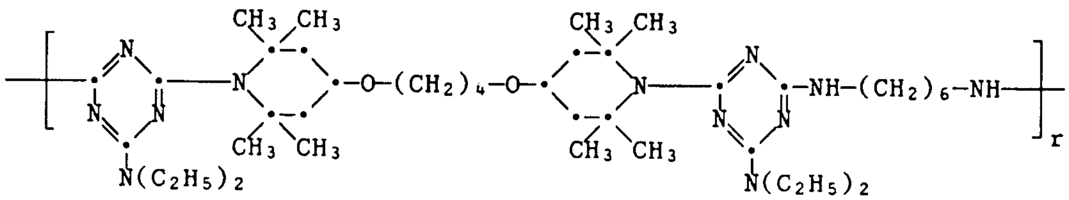

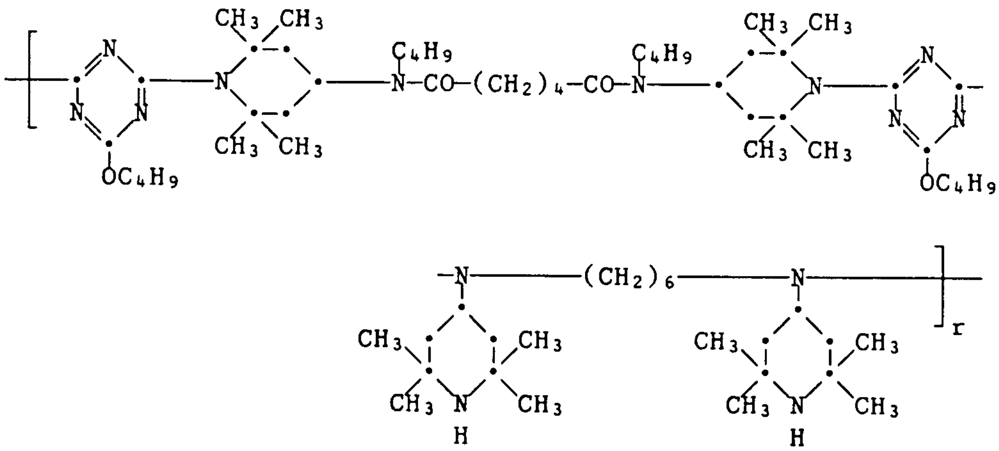

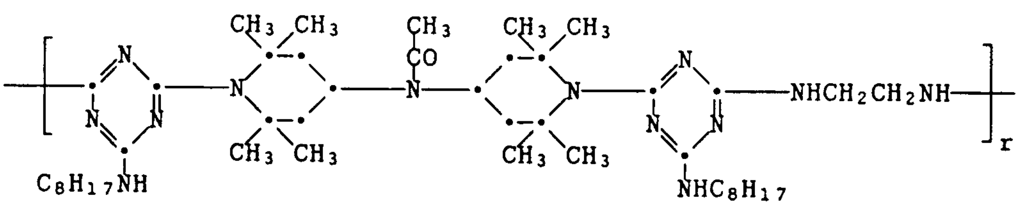

Bei den Verbindungen der Formeln IV bis X und XII handelt es sich um polymere Verbindungen, wobei die Formel die wiederkehrende molekulare Einheit darstellt. Die Endgruppen dieser polymeren Produkte können entsprechende Gruppen der Edukte oder des Polymerisationskatalysators sein. Eine gewünschte Begrenzung des Molekulargewichtes der polymeren Produkte kann durch Zusatz von monofunktionellen Verbindungen oder von Kettenabbrechern bei der Herstellung (Polymerisation) erreicht werden. In diesem Fall entstehen auch Endgruppen: die diesen Zusätzen entsprechen.

Die erfindungsgemässen Verbindungen sind verwendbar als Stabilisatoren für organische Materialien gegen Schädigung durch Licht, Sauerstoff und Hitze. Solche zu stabilisierende Materialien können z.B. Oele, Fette, Wachse, Kosmetika, Biocide oder photographische oder reprographische Materialien sein. Von besonderem Interesse ist die Verwendung in polymeren Materialien, wie sie in Kunststoffen, Kautschuken, Anstrichstoffen oder Klebstoffen vorliegen. Beispiele für Polymere, die auf diese Weise stabilisiert werden können, sind die folgenden:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.
3a. Kohlenwasserstoffharze (z.B. $C_5$-$C_9$) inklusive hydrierte Modifikationen davon (z.B. Klebrigmacherharze).
4. Polystyrol, Poly-(p-methylstyrol), Poly-($\alpha$-methylstyrol).
5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
6. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.
7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
8. Polymere, die sich von $\alpha,\beta$-ungesättigten Säuren und deren Derivaten ableiten, wie Polyacrylate und Polymethacrylate, Polyacrylamide und Polyacrylnitrile.
9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
11. Homo- und Copolymere von cyclischen Ethern, wie Polyalkylenglykole, Polyethylenoxyd, Polypropylenoxyd oder deren Copolymere mit Bisglycidylethern.
12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.
13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Von besonderer Bedeutung ist die Stabilisierung von Polyolefinen sowie von Bindemitteln für Anstrichstoffe.

Die Stabilisatoren werden den organischen Materialien zweckmässig in einer Menge von 0,01 bis 5 Gew.-%, berechnet auf das zu stabilisierende Material, zugesetzt. Vorzugsweise verwendet man 0,1 bis 2 Gew.-%. Der Zusatz zu polymeren Materialien kann bereits bei deren Herstellung (Polymerisation) erfolgen. Vorzugsweise erfolgt er vor oder während der Formgebung des Polymeren.

In gewissen Fällen kann es von Vorteil sein, Gemische von zwei oder mehreren der erfindungsgemässen Stabilisatoren zu verwenden.

Zusammen mit den erfindungsgemässen Stabilisatoren können dem organischen Material noch andere Stabilisatoren oder sonstige übliche Zusätze zugegeben werden. Beispiele hierfür sind die folgenden Zusätze:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-($\alpha$-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Di-phenyl-4-octadecyloxyphenol.

1.3. Hydroxylierte Thiodiphenylether, z.B. 2,2'-Thio-bis-(6-tert.butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert.butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.butyl-4'-hydroxyphenyl)butyrat],Bis-(3-tert.butyl-4-hydroxy-5-methylphenyl)-dicyclopentadien, Bis-[2-(3'-tert.butyl-2'-hydroxy-5'-methyl-benzyl)-6-tert.butyl-4-methyl-phenyl]-terephthalat.

1.5 Benzylverbindungen, z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiolterephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino)-s-triazin, N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.9. Ester der β-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.10. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.butyl-4-hydroxyphenylpropionyl)-trimethylendiamin, N,N'-Bis-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl-, 3',5'-Di-tert.butyl-, 5'-tert.Butyl-, 5'-(1,1,3,3-Tetramethylbutyl)-,5-Chlor-3',5'-di-tert.butyl-, 5-Chlor-3'-tert.butyl-5'-methyl, 3'-sec.-Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o-und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazin, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrittetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Bei der Mitverwendung solcher Costabilisatoren können synergistische Effekte auftreten, was insbesondere bei der Mitverwendung von UV-Absorbern der Fall ist.

Bei der Verwendung der erfindungsgemässen Verbindungen als Stabilisatoren für photographische Materialien ist vor allem die Verwendung in photographischen Schichten, beispielsweise auf Filmen oder Photopapieren, von Interesse.

Ein Teil der erfindungsgemässen Verbindungen lässt sich auch als Zwischenprodukt bei der Herstellung anderer erfindungsgemässer Verbindungen verwenden. Dies trifft vor allem für Verbindungen zu, die am Triazinring Chloratome tragen. Als Stabilisatoren bevorzugt sind solche Verbindungen, die an den Triazinresten keine Chloratome enthalten.

Die folgenden Beispiele illustrieren die Herstellung und Verwendung der erfindungsgemässen Verbindungen näher. Darin bedeuten Teile und % Gewichtsteile und Gewichts-%. Die Temperaturen sind in Celsius-Graden angegeben.

Beispiel 1: 2,4-Dichlor-6-(2,2,6,6-tetramethylpiperidin-1-yl)-1,3,5-triazin.

92,2 g Cyanurchlorid und 142,6 g 2,2,6,6-Tetramethylpiperidin werden in 400 ml Xylol 10 Stunden bei 120° gerührt. Nach dem Abkühlen auf Raumtemperatur wird vom gebildeten 2,2,6,6-Tetramethylpiperidinhydrochlorid abfiltriert und dieses mit 100 ml Xylol gewaschen. Die gelb bis bräunliche Xylollösung wird dreimal mit je 100 ml Wasser gewaschen, über Natriumsulfat getrocknet, mit 5 g Tonsil optimum (Bleicherde) und 5 g Tierkohle 10 Minuten gerührt, geklärt und im Vakuum eingedampft. Der

erhaltene Rückstand wird aus 300 ml Hexan unter Zugabe von 3 g Tonsil optimum umkristallisiert. Man erhält das 2,4-Dichlor-6-(2,2,6,6-tetramethylpiperidin-1-yl)-1,3,5-triazin als farblose Kristalle mit einem Schmelzpunkt von 129°.

Beispiel 2: 2,4-Dichlor-6-(2,2,6,6-tetramethyl-4-benzoyloxy-piperidin-1-yl)-1,3, 5-triazin.

46,1 g Cyanurchlorid und 136,0 g 2,2,6,6-Tetramethyl-4-benzoyloxypiperidin werden in 300 ml Xylol wie in Beispiel 1 beschrieben umgesetzt und aufgearbeitet. Nach einer Umkristallisation aus Isopropanol erhält man das 2,4-Dichlor-6-(2,2,6,6-tetramethyl-4-benzoyloxy-piperidin-1-yl)-1,3,5-triazin als farblose Kristalle mit einem Schmelzpunkt von 145°.

Beispiel 3: 2-Chlor-4-ethylamino-6-(2,2,6,6-tetramethylpiperidin-1-yl)-1,3,5-triazin.

28,9 g 2,4-Dichlor-6-(2,2,6,6-tetramethylpiperidin-1-yl)-1,3,5-triazin (hergestellt nach Beispiel 1) werden in 250 ml Ethanol bei Raumtemperatur mit 15 g einer 70%igen, wässrigen Ethylaminlösung versetzt. Die Temperatur steigt rasch bis auf ca. 35°, Anschliessend wird 12 Stunden bei 55° gerührt, 25 ml Wasser zugegeben und auf 5° abgekühlt. Der entstandene Niederschlag wird abfiltriert, mit 200 ml Wasser gewaschen und getrocknet. Durch Kristallisation aus Acetonitril erhält man das 2-Chlor-4-ethylamino-6-(2,2,6,6-tetramethylpiperidin-1-yl)-1,3,5-triazin als farblose Kristalle mit einem Schmelzpunkt von 148°.

Beispiel 4: 2-Chlor-4-diethylamino-6-(2,2,6,6-tetramethylpiperidin-1-yl)-1,3,5-triazin.

Verwendet man anstelle der Ethylaminlösung 15 g Diethylamin und verfährt man im übrigen wie in Beispiel 3 beschrieben, so erhält man das 2-Chlor-4-diethylamino-6-(2,2,6,6-tetramethylpiperidin-1-yl)-1,3,5-triazin als farblose Substanz mit einem Schmelzpunkt von 77°.

Beispiel 5: 2,4-Bis-isopropylamino-6-(2,2,6,6-tetramethylpiperidin-1-yl)-1,3,5-triazin.

43,4 g 2,4-Dichlor-6-(2,2,6,6-tetramethylpiperidin-1-yl)-1,3,5-triazin werden mit 39,0 g Isopropylamin in 200 ml Xylol 8 Stunden in einem Autoklav auf 160° erhitzt. Nach dem Abkühlen auf Raumtemperatur wird der Autoklaveninhalt dreimal mit je 100 ml Wasser gewaschen, die gelbliche Xylollösung über Natriumsulfat getrocknet, 10 Minuten mit 5 g Tonsil optimum (Bleicherde) verrührt, filtriert und eingedampft. Das erhaltene 2,4-Bis-isopropylamino-6-(2,2,6,6-tetramethylpiperidin-1-yl)-1,3,5-triazin ist ein schwach gelbliches Harz, das nicht kristallisiert werden konnte.

Beispiel 6: 2,4-Bis-dibutylamino-6-(2,2,6,6-tetramethylpiperidin-1-yl)-1,3,5-triazin.

57,8 g 2,4-Dichlor-6-(2,2,6,6-tetramethylpiperidin-1-yl)-1,3,5-triazin werden in 200 ml Xylol bei Raumtemperatur suspendiert. Dazu tropft man in 15 Minuten 25,8 g Dibutylamin, wobei die Temperatur auf 40° steigt. Man gibt zum Reaktionsgemisch in ca. 15 Minuten eine Lösung von 8,8 g Natriumhydroxid in 40 ml Wasser und rührt anschliessend während 2 Stunden bei 60°. Die wässrige Phase wird nun abgetrennt und zur klaren, organischen Phase gibt man 28,4 g Dibutylamin und eine Lösung 9,6 g Natriumhydroxid in 20 ml Wasser. Man erwärmt nun am Wasserabscheider bis eine Innentemperatur von ca. 135° erreicht ist und rührt dann 12 Stunden bei dieser Temperatur. Der Kolbeninhalt wird auf 90° abgekühlt, mit einer Lösung von 3 g Natriumhydroxid in 100 ml Wasser versetzt und 30 Minuten bei 90° kräftig gerührt. Man trennt nun die wässrige Phase ab, wäscht die Xylollösung fünfmal mit je 100 ml Wasser und dampft sie im Vakuum ein. Der gelbliche, ölige Rückstand wird im Hochvakuum destilliert. Man erhält das 2,4-Bis-dibutylamino-6-(2,2,6,6-tetramethylpiperidin-1-yl)-1,3,5-triazin als farbloses Oel mit einem Siedepunkt von 173° bei 6,5 Pa.

Beispiel 7: 2,4-Dimorpholino-6-(2,2,6,6-tetramethylpiperidin-1-yl)-1,3,5-triazin.

Verwendet man anstelle von Dibutylamin 36,6 g Morpholin (1. Portion: 17,4 g; 2. Portion: 19,2 g) und verfährt man im übrigen wie in Beispiel 6 beschrieben, so erhält man, nach Kristallisation aus Ethanol, das 2,4-Dimorpholino-6-(2,2,6,6-tetramethylpiperidin-1-yl)-1,3,5-triazin mit einem Schmelzpunkt von 147-48°.

Beispiel 8: 2,4-Bis-butylamino-6-(2,2,6,6-tetramethyl-4-benzoyloxypiperidin-1-yl)-1,3,5-triazin.

20,5 g 2,4-Dichlor-6-(2,2,6,6-tetramethyl-4-benzoyloxypiperidin-1-yl)-1,3,5-triazin (hergestellt nach Beispiel 2) werden in 200 ml Xylol mit 8,0 g n-Butylamin 4 Stunden am Rückfluss gerührt. Man gibt 4,4 g pulverisiertes Natriumhydroxid zum Reaktionsgemisch und rührt weitere 15 Stunden am Rückfluss. Der Kolbeninhalt wird auf Raumtemperatur abgekühlt und nach der Zugabe von 100 ml Wasser kräftig gerührt bis das ausgefallene Salz vollständig in Lösung gegangen ist. Die wässrige Phase wird abgetrennt, die Xylollösung dreimal mit je 100 ml Wasser gewaschen und im Vakuum eingedampft. Der ölige Rückstand wird bei 100° und 13 Pa. getrocknet. Man erhält das 2,4-Bis-butylamino-6-(2,2,6,6-tetramethyl-4-benzoylpiperidin-1-yl)-1,3,5-triazin als gelbliche viskose Masse.

Beispiel 9: 2,4-Bis-butylamino-6-(2,2,6,6-tetramethyl-4-hydroxypiperidin-1-yl)-1,3,5-triazin,

10 g 2,4-Bis-butylamino-6-(2,2,6,6-tetramethyl-4-benzoyloxypiperidin-1-yl)-1,3,5-triazin (hergestellt nach Beispiel 8) werden mit 50 ml Methanol und 50 ml zwanzigprozentiger Natronlauge 6 Stunden am Rückfluss erhitzt. Aus dem Reaktionsgemisch wird das Methanol im Vakuum abdestilliert. Zum Rückstand gibt man 100 ml Toluol und 50 ml Wasser, schüttelt kräftig durch, trennt die wässrige Phase ab und wäscht die Toluollösung dreimal mit je 50 ml Wasser. Nach dem Eindampfen der Toluollösung erhält man das 2,4-Bis-butylamino-6-(2,2,6,6-tetramethyl-4-hydroxypiperidin-1-yl)-1,3,5-triazin als gelbliche viskose Masse. Durch Kristallisation aus Acetonitril erhält man farblose Kristalle, die bei 90°C schmelzen.

Beispiel 10: 2,4-Dichlor-6-(2,2,6,6-tetramethyl-4-hexyloxypiperidin-1-yl)-1,3,5-triazin.

Zu einer Lösung von 23,9 g Cyanurchlorid in 100 ml Toluol werden unter Rühren 62,7 g 4-Hexyloxy-2,2,6,6-tetramethylpiperidin zugegeben. Anschliessend wird 24 Stunden auf 80° erwärmt. Dabei bildet sich ein weisser Niederschlag des Piperidin-Hydrochlorides. Nach dem Erkalten filtriert man den Niederschlag ab, wäscht die Toluollösung mehrmals mit 2N-Salzsäure, trocknet sie über $Na_2SO_4$ und dampft sie ein. Der Rückstand wird aus Acetonitril umkristallisiert. Das erhaltene Produkt schmilzt bei 49-51°.

Beispiel 11: 2,4-Dimorpholino-6-(2,2,6,6-tetramethyl-4-hexyloxypiperidin-1-yl)-1,3,5-triazin.

9,7 g des Produktes von Beispiel 10 werden mit 50 ml Morpholin 3 Stunden unter Rückfluss erwärmt. Das orange Reaktionsgemisch wird auf Wasser gegossen. Das dabei ausfallende Rohprodukt wird in Ethylacetat gelöst und an einer $SiO_2$-Säule chromatographisch gereinigt. Das gereinigte Produkt ist eine viskose Masse.

| | | | | |
|---|---|---|---|---|
| Analyse: | Ber.: | C = 63,64 % | H = 9,44 % | N = 17,12 % |
| | Gef.: | C = 63,64 % | H = 9,29 % | N = 17,08 % |

Beispiel 12: Verwendet man im Beispiel 11 statt des Morpholins Diisobutylamin und arbeitet sonst gleich wie in Beispiel 11, so erhält man das 2,4-Bis(diisobutylamino)-6-(2,2,6,6-tetramethyl-4-hexyloxypiperidin-1-yl)-1,3,5-triazin als viskose Flüssigkeit.

| | | | | |
|---|---|---|---|---|
| Analyse: | Ber.: | C = 75,12 % | H = 12,35 % | N = 10,51 % |
| | Gef.: | C = 75,36 % | H = 12,08 % | N = 10,54 % |

Beispiel 13: Verwendet man im Beispiel 11 statt des Morpholins Dibutylamin und arbeitet sonst wie in Beispiel 11 beschrieben, so erhält man das 2,4-Bis(dibutylamino)-6-(2,2,6,6-tetramethyl-4-hexyloxypiperidin-1-yl)-1,3,5-triazin als viskose Masse.

| | | | | |
|---|---|---|---|---|
| Analyse: | Ber.: | C = 71,02 % | H = 11,57 % | N = 14,61 % |
| | Gef.: | C = 71,09 % | H = 10,96 % | N = 14,69 % |

Beispiel 14: Polykondensat aus 2,4-Dichlor-6-(2,2,6,6-tetramethylpiperidin-1-yl)-1,3,5-triazin und Hexamethylendiamin.

Zu einer Lösung von 12,4 g (43 mmol) 2,4-Dichlor-6-(2,2,6,6-tetramethylpiperidin-1-yl)-1,3,5-triazin in 50 ml Xylol wird bei 100° unter Rühren eine Lösung von 10,5 g (90 mmol) Hexamethylendiamin in 50 ml Xylol langsam zugetropft und das Reaktionsgemisch 22 Stunden unter Rückfluss gerührt. Nach dem Abkühlen wird filtriert, das Filtrat zweimal mit 50 ml Wasser gewaschen und mit soviel Hexan versetzt bis kein Niederschlag mehr ausfällt. Der Niederschlag wird abfiltriert, das Filtrat dreimal mit 50 ml Wasser gewaschen, über $Na_2SO_4$ getrocknet und eingedampft. Der Rückstand wird im Vakuum bei 50° getrocknet. Man erhält ein harzartiges Polymer vom Molekulargewicht $\overline{M}_n$ = 1029/$\overline{M}_w$ = 1545 - (Gelpermeationschromatographie).

Beispiel 15: 2,4-Dichlor-6-(1,3,8-triaza-2,4-dioxo-3,7,7,9,9-pentamethyl-spiro[4,5]dec-1-yl)-1,3,5-triazin.

Zu einer Lösung von 90,9 g (0,38 mol) 1,3,8-Triaza-2,4-dioxo-3,7,7,9,9-pentamethyl-spiro[4,5]decan in 500 ml Xylol wird bei 0-5° unter Rühren eine Lösung von 35,1 g (0,19 mol) Cyanurchlorid in 200 ml Xylol zugetropft. Anschliessend wird 24 Stunden zum Rückfluss erwärmt. Nach dem Abkühlen filtriert man vom Niederschlag ab und wäscht das Filtrat zuerst mit Wasser, das mit Essigsäure auf pH 5 gestellt wurde, dann mit einer $Na_2CO_3$-Lösung und schliesslich mit Wasser. Die Xylol-Lösung wird filtriert, über $Na_2SO_4$ getrocknet und im Vakuum eingedampft. Der Rückstand wird aus 70 ml Acetonitril umkristallisiert. Man erhält ein bräunliches Pulver, das bei 237-242° schmilzt.

| | | | | |
|---|---|---|---|---|
| Analyse: | Ber.: | C = 46,52 % | H = 21,70 % | N = 5,21 % |
| | Gef.: | C = 46,40 % | H = 21,85 % | N = 5,22 % |

Beispiel 16: 2,4-Dimorpholino-6-(1,3,8-triaza-2,4-dioxo-3,7,7,9,9-pentamethyl-spiro[4,5]dec-1-yl)-1,3,5-triazin.

Zu 6 g des Produktes von Beispiel 15 werden unter Eiskühlung langsam 5,4 g Morpholin zugegeben. Dann werden weitere 30 ml Morpholin zugegeben und das Reaktionsgemisch bis zum Rückfluss erwärmt. Nach 38 Stunden Rückflusskochen wird abgekühlt und mit 50 ml Wasser versetzt. Der dabei ausfallende Niederschlag wird abfiltriert, mit Wasser gewaschen und getrocknet. Das Produkt wird aus Methylenchlorid/Hexan umkristallisiert. Man erhält ein weisses Pulver, das bei 319-321° unter Zersetzung schmilzt.

| | | | | |
|---|---|---|---|---|
| Analyse: | Ber.: | C = 56,54 % | H = 22,93 % | N = 7,43 % |
| | Gef.: | C = 56,55 % | H = 22,88 % | N = 7,53 % |

Beispiel 17: 2,4-Dichlor-6-(4,4-ethylendioxy-2,2,6,6-tetramethylpiperidin-1-yl)-1,3,5-triazin.

Zu einer Lösung von 119,6 g (0,6 mol) 4,4-Ethylendioxy-2,2,6,6-tetramethylpiperidin in 100 ml Xylol wird unter Rühren und unter Kühlung auf 0-5° eine Lösung von 55,3 g (0,3 mol) Cyanurchlorid in 300 ml Xylol zugetropft. Anschliessend erwärmt man zum Rückfluss und hält 26 Stunden bei Siedetemperatur. Nach Zugabe von 150 ml Xylol lässt man abkühlen und filtriert vom Niederschlag ab. Das Filtrat wird im Vakuum eingedampft. Der Rückstand wird aus 300 ml Acetonitril umkristallisiert. Das erhaltene Produkt ist ein bräunliches Pulver, das bei 169-172° schmilzt.

| | | | | |
|---|---|---|---|---|
| Analyse: | Ber.: | C = 48,43 % | H = 16,41 % | N = 5,81 % |
| | Gef.: | C = 48,49 % | H = 16,20 % | N = 5,66 % |

Beispiel 18: 2-Chlor-4-diisopropylamino-6-(4,4-ethylendioxy-2,2,6,6-tetramethylpiperidin-1-yl)-1,3,5-triazin.

Zu einer Lösung von 29,9 g (86 mmol) des Produktes von Beispiel 17 in 100 ml Toluol werden unter Rühren 26,1 g (258 mmol) Diisopropylamin zugegeben. Das Gemisch wird zum Rückfluss erwärmt und 24 Stunden bei dieser Temperatur gehalten. Nach dem Abkühlen wird eine Lösung von 4,1 g NaOH in 21 ml Wasser zugegeben. Das feste Produkt wird abfiltriert und aus 25 ml Toluol umkristallisiert. Man erhält ein gelbliches Pulver, das bei 179-184° schmilzt.

| | | | | |
|---|---|---|---|---|
| Analyse: | Ber.: | C = 58,31 % | H = 17,00 % | N = 8,32 % |
| | Gef.: | C = 58,34 % | H = 16,97 % | N = 8,42 % |

Beispiel 19: 2-Octylamino-4-diisopropylamino-6-(4,4-ethylendioxy-2,2,6,6-tetramethylpiperidin-1-yl)-1,3,5-triazin.

15 g (36,4 mmol) des Produktes von Beispiel 18 werden zusammen mit 30 ml Octylamin 3 Stunden auf 120° erwärmt. Nach dem Abkühlen werden 40 ml Wasser zugegeben und dreimal mit 30 ml Methylenchlorid extrahiert. Die $CH_2Cl_2$-Lösung wird mit Wasser gewaschen, über $Na_2SO_4$ getrocknet und eingedampft. Der ölige Rückstand wird in Hexan/Aceton gelöst und an einer $SiO_2$-Säule chromatographisch gereinigt. Die Hauptfraktion ist eine viskose Masse.

| | | | | |
|---|---|---|---|---|
| Analyse: | Ber.: | C = 66,63 % | H = 16,65 % | N = 10,38 % |
| | Gef.: | C = 66,76 % | H = 16,48 % | N = 10,30 % |

Beispiel 20: 2,4-Dimorpholino-6-(4,4-ethylendioxy-2,2,6,6-tetramethylpiperidin-1-yl)-1,3,5-triazin.

Zu 40 g des Produktes aus Beispiel 17 werden unter Eiskühlung 40 g Morpholin zugegeben. Nach Zugabe von weiteren 100 g Morpholin wird das Gemisch unter Rühren langsam auf 130° erwärmt und 6 Stunden unter Rückfluss gekocht. Nach Abkühlen auf Raumtemperatur werden 150 ml Wasser zugegeben. Der gebildete Niederschlag wird abfiltriert, mit Wasser gewaschen und aus Acetonitril umkristallisiert. Das erhaltene Produkt schmilzt bei 216-221°.

| | | | | |
|---|---|---|---|---|
| Analyse: | Ber.: | C = 58,91 % | H = 18,74 % | N = 8,09 % |
| | Gef.: | C = 58,93 % | H = 18,88 % | N = 8,01 % |

Beispiel 21: 2,4-Dimorpholino-6-(4-oxo-2,2,6,6-tetramethylpiperidin-1-yl)-1,3,5-triazin.

Unter Rühren werden 29,6 g des Produktes von Beispiel 20 in 200 ml einer Lösung von 1,26 g p-Toluolsulfonsäure in einer 1:1-Mischung von Tetrahydrofuran und Wasser eingetragen. Nach Zugabe von weiteren 60 ml Tetrahydrofuran wird 5 Stunden auf 50° erwärmt. Dann werden nochmals 1,26 g Toluolsulfonsäure zugegeben und weitere 14 Stunden bei 50° gerührt. Nach dem Abkühlen wird fünfmal mit 50 ml Methylenchlorid extrahiert. Die vereinigten $CH_2Cl_2$-Lösungen werden mit Wasser gewaschen, über $Na_2SO_4$ getrocknet und eingedampft. Der Rückstand wird aus 80 ml Acetonitril umkristallisiert. Die erhaltenen weissen Kristalle schmelzen bei 188-192°.

| | | | | |
|---|---|---|---|---|
| Analyse: | Ber.: | C = 59,38 % | H = 20,78 % | N = 7,97 % |
| | Gef.: | C = 59,58 % | H = 20,57 % | N = 7,93 % |

Beispiel 22: 2,4-Dichlor-6-(2,2,6,6-tetramethyl-4-dodecyloxypiperidin-1-yl)-1,3,5-triazin.

Verwendet man an Stelle des in Beispiel 10 beschriebenen 4-Hexyloxy-2,2,6,6-tetramethylpiperidins eine gleiche moläquivalente Menge 4-Dodecyloxy-2,2,6,6-tetramethylpiperidin und verfährt im übrigen genau gleich, wie in Beispiel 10 beschrieben, so erhält man obige Verbindung als leicht gelbliches Oel

| | | | | | |
|---|---|---|---|---|---|
| Analyse: | Ber.: | C = 60,88 % | H = 8,94 % | N = 11,83 % | Cl = 14,97 % |
| | Gef.: | C = 61,14 % | H = 8,69 % | N = 11,62 % | Cl = 14,66 % |

Beispiel 23: 2,4-Dichlor-6-(2,2,6,6-tetramethyl-4-allyloxypiperidin-1-yl)-1,3,5-triazin.

Verwendet man an Stelle des in Beispiel 10 beschriebenen 4-Hexyloxy-2,2,6,6-tetramethylpiperidins eine gleiche moläquivalente Menge 4-Allyloxy-2,2,6,6-tetramethylpiperidin und verfährt im übrigen genau gleich, wie in Beispiel 10 beschrieben, so erhält man obige Verbindung als kristallines Produkt, das aus Ethanol umkristallisiert werden kann. Das erhaltene Produkt schmilzt bei 53-55°C.

Beispiel 24: Verwendet man in Beispiel 11 statt des Morpholins Di(-2-ethyl-hexyl)-amin und arbeitet sonst genau gleich, wie in Beispiel 11 beschrieben, so erhält man 2,4-Bis[di(-2-ethyl-hexyl)-amino]-6-(2,2,6,6-tetramethyl-4-hexyloxypiperidin-1-yl)-1,3,5-triazin als gelbliches Oel.

| | | | | |
|---|---|---|---|---|
| Analyse: | Ber.: | C = 75,12 % | H = 12,35 % | N = 10,51 % |
| | Gef.: | C = 75,36 % | H = 12,08 % | N = 10,54 % |

Beispiel 25: Verwendet man in Beispiel 11 anstelle des Morpholins 4-Butylamino-2,2,6,6-tetramethylpiperidin und verfährt im übrigen genau gleich, wie in Beispiel 11 beschrieben, so erhält man 2,4-Bis-[N-(2,2,6,6-tetramethylpiperidin-4-yl)-butylamino]-6-(2,2,6,6-tetramethyl-4-hexyloxypiperidin-1-yl)-1,3,5-triazin als weisses Kristallisat, das aus Acetonitril umkristallisiert werden kann. Smp.: 135-137°C.

Beispiel 26: 2-Chlor-4-diisopropylamino-6-(2,2,6,6-tetramethylpiperidin-4-hexyloxypiperidin-1-yl)-1,3,5-triazin

Verwendet man in Beispiel 3 anstelle von 2,4-Dichlor-6-(2,2,6,6-tetramethylpiperidin-1-yl)-1,3,5-triazin die gleiche moläquivalente Menge der in Beispiel 10 hergestellten Verbindung und anstelle einer Ethylaminlösung, eine moläquivalente Menge Diisopropylamin und verfährt im übrigen genau gleich, wie in Beispiel 3 beschrieben, so erhält man obige Verbindung als farbloses Oel.

| | | | | | |
|---|---|---|---|---|---|
| Analyse: | Ber.: | C = 63,47 % | H = 9,76 % | N = 15,42 % | Cl = 7,80 % |
| | Gef.: | C = 63,64 % | H = 9,73 % | N = 15,47 % | Cl = 7,80 % |

Beispiel 27: N,N'-Bis-[4-diisopropylamino-6-(4,4-ethylendioxy-2,2,6,6-tetramethylpiperidin-1-yl)-triazin-2-yl]-hexamethylendiamin

Zu 14 mg (34 mmol) des Produktes von Beispiel 18 in 40 ml Xylol werden bei 100° 2,07 g (17 mmol) 1,6-Diamino-hexan und eine Lösung von 1,36 g (34 mmol) Natriumhydroxid in 4 ml Wasser zugegeben.

Unter Abdestillieren von Wasser wird die Innentemperatur bis auf 135°C erhöht. Nach 21 Stunden werden 0,1 g 1,6-Diamino-hexan zugegeben und es wird während 6 Stunden bei derselben Temperatur weiterreagieren gelassen. Man lässt auf 70C° abkühlen, gibt 0,26 g Natriumhydroxyd, gelöst in 7,4 ml Wasser, zu, lässt 30 Minuten rühren und trennt die beiden Phasen. Die organische Phase wird dreimal mit Wasser gewaschen, über $Na_2SO_4$ getrocknet und eingedampft. Der Rückstand wird in Chloroform gelöst und an einer $SiO_2$-Säule chromatographisch gereinigt. Als Hauptfraktion erhält man das obige Produkt, das nach gutem Trocknen bei 145-147° schmilzt.

| | | | | |
|---|---|---|---|---|
| Analyse: | Ber.:<br>Gef.: | C = 63,71 %<br>C = 64,13 % | H = 9,53 %<br>H = 9,52 % | N = 19,38 %<br>N = 19,01 % |

Beispiel 28: 2,4-Dichlor-6-(1,3,8-triaza-2,4-dioxo-3-dodecyl-7,7,9,9-tetramethyl-spiro[4,5]dec-1-yl)-1,3,5-triazin

Verwendet man an Stelle des im Beispiel 15 beschriebenen 1,3,8-Triaza-2,4-dioxo-3,7,7,9,9-pentamethyl-spiro[4,5]decans eine moläquivalente Menge 1,3,8-Triaza-2,4-dioxo-3-dodecyl-7,7,9,9-tetramethyl-spiro-[4,5]decan und verfährt im übrigen gleich wie unter Beispiel 15 angegeben, so erhält man, nachdem das angefallene Produkt in Hexan/Aceton gelöst und an einer $SiO_2$-Säule chromatographisch gereinigt worden war, das obige Produkt, das bei 109-115° schmilzt.

Beispiel 29: 2,4-Dimorpholino-6-(1,3,8-triaza-2,4-dioxo-3-dodecyl-7,7,9,9-tetramethyl-spiro[4,5]dec-1-yl)-1,3,5-triazin

Verwendet man an Stelle des im Beispiel 15 beschriebenen 2,4-Dichlor-6-(1,3,8-triaza-2,4-dioxo-3,7,7,9,9-pentamethyl-spiro[4,5]dec-1-yl)1,3,5-triazins eine moläquivalente Menge des unter Beispiel 28 beschriebenen 2,4-Dichlor-6-(1,3,8-triaza-2,4-dioxo-3-dodecyl-7,7,9,9-tetramethyl-spiro[4,5]dec-1-yl)-1,3,5-triazins und verfährt im übrigen gleich wie unter Beispiel 16 angegeben, so erhält man das obige Produkt, das bei 185-188° schmilzt.

| | | | | |
|---|---|---|---|---|
| Analyse: | Ber.:<br>Gef.: | C = 63,52 %<br>C = 63,39 % | H = 9,09 %<br>H = 9,20 % | H = 17,43 %<br>H = 17,38 % |

Beispiel 30: 2,4-Bis-N-butylmethylamino-6-(1,3,8-triaza-2,4-dioxo-3-dodecyl-7,7,9,9-tetramethyl-spiro[4,5]-dec-1-yl)-1,3,5-triazin

Verfährt man gleich wie unter Beispiel 29 angegeben, verwendet jedoch an Stelle von Morpholin eine moläquivalente Menge N-Butylmethylamin, so erhält man obige Verbindung, die bei 93-96° schmilzt.

| | | | | |
|---|---|---|---|---|
| Analyse: | Ber.:<br>Gef.: | C = 67,25 %<br>C = 67,05 % | H = 10,35 %<br>H = 10,26 % | H = 17,43 %<br>H = 17,55 % |

Beispiel 31: 2,4-Bis-N-butylmethylamino-6-(4,4-ethylendioxy-2,2,6,6-tetramethylpiperidin-1-yl)-1,3,5-triazin

Zu 15 g des Produktes aus Beispiel 17 werden unter Eiskühlung 24 ml N-Butylmethylamin zugegeben. Nach Zugabe von weiteren 50 ml N-Butylmethylamin wird das Gemisch unter Rühren langsam auf 88° erwärmt und 24 Stunden unter Rückfluss gekocht. Nach Abkühlen auf Raumtemperatur werden 150 ml Wasser zugegeben und etwas HCl bis pH 2. Dann wird mit Methylenchlorid ausgeschüttelt; die organische Phase wird über $Na_2SO_4$ getrocknet, eingedampft und getrocknet. Das Produkt der obigen Formel fällt dabei als leicht gelbes Oel an.

| | | | | |
|---|---|---|---|---|
| Analyse: | Ber.:<br>Gef.: | C = 64,25 %<br>C = 64,58 % | H = 9,89 %<br>H = 10,17 % | N = 18,73 %<br>N = 18,51 % |

Beispiel 32: 2,4-Bis-dibutylamino-6-(4,4-ethylendioxy-2,2,6,6-tetramethylpiperidin-1-yl)-1,3,5-triazin

Verwendet man an Stelle des im Beispiel 31 beschriebenen N-Butylmethylamins eine moläquivalente Menge Dibutylamin und verfährt im übrigen gleich wie im Beispiel 32 angegeben, wobei das Produkt am Schluss mit Toluol/Hexan gelöst und an einer $SiO_2$-Säule chromatographisch gereinigt wird, so erhält man obiges Produkt als farbloses Oel.

| Analyse: | Ber.:<br>Gef.: | C = 67,63 %<br>C = 67,27 % | H = 10,59 %<br>H = 10,37 % | N = 15,77 %<br>N = 15,45 % |
|---|---|---|---|---|

Beispiel 33: 2,4-Bis-N-butylmethylamino-6-(4-oxo-2,2,6,6-tetramethylpiperidin-1-yl)-1,3,5-triazin
Verwendet man an Stelle des im Beispiel 21 beschriebenen 2,4-Dimorpholino-6-(4,4-ethylendioxy-2,2,6,6-tetramethylpiperidin-1-yl)-1,3,5-triazins eine moläquivalente Menge des im Beispiel 31 beschriebenen 2,4-Bis-N-butylmethylamino-6-(4,4-ethylendioxy-2,2,6,6-tetramethylpiperidin-1-yl)-1,3,5-triazin und verfährt im übrigen gleich wie im Beispiel 21 angegeben, wobei das Produkt am Schluss in Hexan/Aceton gelöst und an einer $SiO_2$-Säule chromatographisch gereinigt wird, so erhält man obiges Produkt als farbloses Oel.

| Analyse: | Ber.:<br>Gef.: | C = 65,31 %<br>C = 65,11 % | H = 9,97 %<br>H = 9,86 % | N = 20,77 %<br>N = 20,53 % |
|---|---|---|---|---|

Beispiel 34: 2,4-Dimorpholino-6-(4-hydroxy-2,2,6,6-tetramethylpiperidin-1-yl)-1,3,5-triazin
5 g 2,4-Dimorpholino-6-(4-oxo-2,2,6,6-tetramethylpiperidin-1-yl)-1,3,5-triazin (Produkt aus Beispiel 21) werden in 100 ml Tetrahydrofuran bei 60° und einem Druck von 100 bar mit Raney-Nickel als Katalysator bis zum Stillstand der Reaktion hydriert. Das Reaktionsgemisch wird filtriert, eingedampft und aus 20 ml Toluol umkristallisiert. Die obige Substanz fällt als weisse Kristalle an, die bei 202-204° schmelzen.

| Analyse: | Ber.:<br>Gef.: | C = 59,09 %<br>C = 59,27 % | H = 8,43 %<br>H = 8,35 % | N = 20,67 %<br>N = 20,54 % |
|---|---|---|---|---|

Beispiel 35: 2-Chlor-4-(1-acetyl-2,2,6,6-tetramethylpiperidin-4-oxy)-6-(2,2,6,6-tetramethylpiperidin-1-yl)-1,3,5-triazin
43,4 g 2,4-Dichlor-6-(2,2,6,6-tetramethylpiperidin-1-yl)-1,3,5-triazin (Produkt aus Beispiel 1) werden in 175 ml Toluol gelöst, worauf 42,1 g pulverisiertes KOH, 1 g Kaliumcarbonat und 3,4 g Tetrabutylammoniumhydrogensulfat zugegeben werden. Dann wird eine Lösung von 29,9 g 1-Acetyl-2,2,6,6-tetramethyl-4-hydroxy-piperidin in 110 ml Toluol zugetropft, wobei die Innentemperatur mit einem Eisbad auf 10° gehalten wird. Nach 3,5 Stunden Rühren werden 100 ml Wasser zugegeben. Die Phasen werden getrennt, die organische Phase wird mit Wasser gewaschen, über $Na_2SO_4$ getrocknet und eingedampft. Nach Umkristallisieren aus Hexan fällt obiges Produkt als weisses Pulver an, das bei 114-116°C schmilzt.

| Analyse: | Ber.:<br>Gef.: | C = 61,11 %<br>C = 61,46 % | H = 8,47 %<br>H = 8,50 % | N = 15,49 %<br>N = 15,27 % |
|---|---|---|---|---|

Beispiel 36: 2-Chlor-4-(1-oxyl-2,2,6,6-tetramethylpiperidin-4-oxy)-6-(2,2,6,6-tetramethylpiperidin-1-yl)-1,3,5-triazin
Verwendet man an Stelle des im Beispiel 35 beschriebenen 1-Acetyl-2,2,6,6-tetramethyl-4-hydroxy-piperidins eine moläquivalente Menge 1-Oxyl-2,2,6,6-tetramethyl-4-hydroxy-piperidin und verfährt im übrigen gleich wie im Beispiel 35 angegeben, wobei das Produkt am Schluss aus Acetonitril umkristallisiert wird, so erhält man obiges Produkt als rötliches Pulver, das bei 152-153° schmilzt.

| Analyse: | Ber.:<br>Gef.: | C = 59,35 %<br>C = 59,08 % | H = 8,30 %<br>H = 8,29 % | N = 16,48 %<br>N = 16,65 % |
|---|---|---|---|---|

Beispiel 37: N,N'-Bis-(4-(1-acetyl-2,2,6,6-tetramethylpiperidin-4-oxy)-6-(2,2,6,6-tetramethylpiperidin-1-yl)-triazin-2-yl]-hexamethylendiamin
Zu 20 g 2-Chlor-4-(1-acetyl-2,2,6,6-tetramethylpiperidin-4-oxy)-6-(2,2,6,6-tetramethylpiperidin-1-yl)-1,3,5-triazin (Produkt aus Beispiel 35) werden 60 ml Xylol, 2,6 g 1,6-Diaminohexan und eine Lösung von 1,8 g Natriumhydroxid in 5 ml Wasser gegeben. Dann wird das Gemisch erhitzt, wobei das Wasser abdestilliert wird. Wenn die Temperatur konstant bleibt, wird noch während 24 Stunden am Rückfluss gerührt. Nach dem Abkühlen wird etwas Natronlauge zugegeben, worauf die Phasen getrennt werden. Die organische Phase wird über $Na_2SO_4$ getrocknet und eingedampft. Nach Umkristallisation aus Chloroform/Hexan, Lösen des Produkts in Chloroform/Methanol und chromatographischer Reinigung an einer $SiO_2$-Säule erhält man obiges Produkt als weisses Pulver, das bei 201-202° schmilzt.

| | | | | |
|---|---|---|---|---|
| Analyse: | Ber.:<br>Gef.: | C = 65,93 %<br>C = 65,78 % | H = 9,58 %<br>H = 9,73 % | N = 17,74 %<br>N = 17,67 % |

Beispiel 38: 2-Morpholino-4-(1-oxyl-2,2,6,6-tetramethylpiperidin-4-oxy)-6-(2,2,6,6-tetramethylpiperidin-1-yl)-1,3,5-triazin

5 g 2-Chlor-4-(1-oxyl-2,2,6,6-tetramethylpiperidin-4-oxy)-6-(2,2,6,6-tetramethylpiperidin-1-yl)-1,3,5-triazin (Produkt aus Beispiel 36) werden mit 50 ml Morpholin während 3 Stunden am Rückfluss gerührt. Nach Abkühlen auf 20° werden 200 ml Wasser zugegeben, und der Niederschlag wird abfiltriert. Nach Umkristallisation aus Petroläther erhält man obiges Produkt als rötliche Kristalle, die bei 153-157° schmelzen.

| | | | | |
|---|---|---|---|---|
| Analyse: | Ber.:<br>Gef.: | C = 63,13 %<br>C = 62,94 % | H = 9,11 %<br>H = 9,27 % | N = 17,67 %<br>N = 17,42 % |

Beispiel 39: 2-(1-Acetyl-2,2,6,6-tetramethylpiperidin-4-oxy)-4-(1-oxyl-2,2,6,6-tetramethylpiperidin-4-oxy)-6-(2,2,6,6-tetramethylpiperidin-1-yl)-1,3,5-triazin

4 g 2-Chlor-4-(1-oxyl-2,2,6,6-tetramethylpiperidin-4-oxy)-6-(2,2,6,6-tetramethylpiperidin-1-yl)-1,3,5-triazin (Produkt aus Beispiel 36) werden in 30 ml Toluol gelöst, worauf 2,6 g pulverisiertes KOH, 1 g Kaliumcarbonat und 0,15 g Tetrabutylammoniumhydrogensulfat zugegeben werden. Unter Rühren werden 1,95 g 1-Acetyl-2,2,6,6-tetramethyl-4-hydroxypiperidinzugegeben. Während 17 Stunden wird das Gemisch auf 60° erwärmt. Nach Abkühlen werden 30 ml Wasser zugegeben. Die Phasen werden getrennt. Die organische Phase wird über $Na_2SO_4$ getrocknet und eingedampft. Nach Umkristallisieren aus Methylenchlorid/Hexan, Lösen des Produkts in Hexan/Essigsäure und chromatographischer Reinigung an einer $SiO_2$-Säule erhält man obiges Produkt, das bei 175-176° schmilzt.

| | | | | |
|---|---|---|---|---|
| Analyse: | Ber.:<br>Gef.: | C = 65,38 %<br>C = 65,42 % | H = 9,43 %<br>H = 9,45 % | N = 14,30 %<br>N = 14,04 % |

Beispiel 40: 2-chlor-4-[N-bis(2,2,6,6-tetramethylpiperidin-4-yl)-amino]-6-(2,2,6,6-tetramethylpiperidin-1-yl)-1,3,5-triazin

Zu 28,9 g 2,4-Dichlor-6-(2,2,6,6-tetramethylpiperidin-1-yl)-1,3,5-triazin (Produkt aus Beispiel 1), gelöst in 130 ml Toluol, werden 29,5 g Bis-(2,2,6,6-tetramethylpiperidin-4-yl)-amin, gelöst in 60 ml Toluol, zugegeben. Nach 26 Stunden Rühren am Rückfluss und abkühlen lassen werden 5,6 g pulverisiertes KOH und 30 ml Wasser zugegeben, worauf die Phasen getrennt werden. Die organische Phase wird über $Na_2SO_4$ getrocknet und eingedampft. Nach Umkristallisation aus Acetonitril erhält man obiges Produkt als weisses Pulver, das bei 240-242° schmilzt.

| | | | | |
|---|---|---|---|---|
| Analyse: | Ber.:<br>Gef.: | C = 65,72 %<br>C = 65,69 % | H = 9,93 %<br>H = 9,89 % | N = 17,88 %<br>N = 17,69 % |

Beispiel 41: N,N'-Bis-{4-[N-bis(2,2,6,6-tetramethylpiperidin-4-yl)-amino]-6-(2,2,6,6-tetramethylpiperidin-1-yl)-triazin-2-yl}-hexamethylendiamin

Verwendet man an Stelle des im Beispiel 37 beschriebenen 2-Chlor-4-(1-acetyl-2,2,6,6-tetramethylpiperidin-4-oxy)-6-(2,2,6,6-tetramethylpiperidin-1-yl)-1,3,5-triazins eine moläquivalente Menge des unter Beispiel 40 beschriebenen Produktes und verfährt im übrigen gleich wie im Beispiel 37 beschrieben, so erhält man obiges Produkt als weisses Pulver, das bei 216-218° schmilzt.

| Analyse: | Ber.:<br>Gef.: | C = 69,55 %<br>C = 69,43 % | H = 10,79 %<br>H = 10,67 % | N = 19,66 %<br>N = 19,44 % |
|---|---|---|---|---|

Beispiel 42: Verbindung der Formel

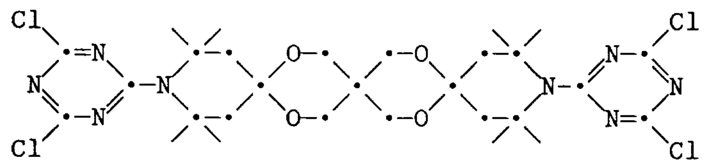

Zu 150 g 2,2,4,4,14,14,16,16-Octamethyl-7,11,18,21-tetraoxa-3,15-diazatrispiro[5,2 ,2,5,2,2]heneicosan, in 300 ml Xylol suspendiert, werden bei 0° 66 g Cyanurchlorid, gelöst in 500 ml Xylol, zugegeben. Nach 51 Stunden Rühren am Rückfluss lässt man etwas abkühlen, filtriert und dampft die flüssige Phase ein. Nach Lösung in Aceton/Chloroform/Methylenchlorid, chromatographischer Reinigung an einer $SiO_2$-Säule und Umkristallisation aus Toluol erhält man die Verbindung obiger Struktur, die bei 277-278° schmilzt.

| Analyse: | Ber.:<br>Gef.: | C = 49,30 %<br>C = 49,16 % | H = 5,71 %<br>H = 5,68 % | N = 15,86 %<br>N = 15,70 % |
|---|---|---|---|---|

Beispiel 43: Verbindung der Formel

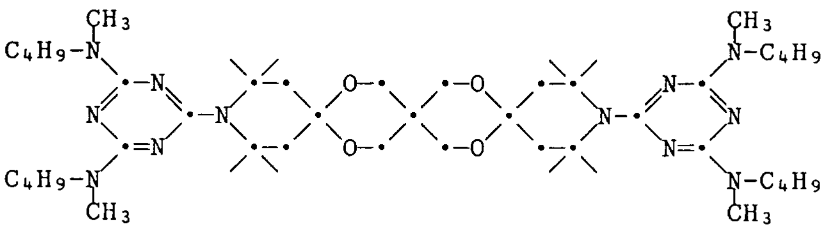

Verwendet man an Stelle des im Beispiel 31 eingesetzten 2,4-Dichlor-6-(4,4-ethylendioxy-2,2,6,6-tetramethylpiperidin-1-yl)-1,3,5-triazins eine moläquivalente Menge der im Beispiel 42 beschriebenen Tetrachlorverbindung und verfährt im übrigen gleich wie im Beispiel 31 angegeben, so erhält man nach Umkristallisieren aus Aceton die Verbindung obiger Struktur, die bei 141-143° schmilzt.

| Analyse: | Ber.:<br>Gef.: | C = 64,72 %<br>C = 64,66 % | H = 9,75 %<br>H = 9,79 % | N = 18,48 %<br>N = 18,37 % |
|---|---|---|---|---|

Beispiel 44: 2-Chlor-4-morpholino-6-(4,4-ethylendioxy-2,2,6,6-tetramethylpiperidin-1-yl)-1,3,5-triazin
Zu einer Lösung von 30 g 2,4-Dichlor-6-(4,4-ethylendioxy-2,2,6,6-tetramethylpiperidin-1-yl)-1,3,5-triazin (Produkt aus Beispiel 17) in 100 ml Toluol werden 3,8 g Morpholin und 15 Minuten später eine Lösung von 1,7 g NaOH in 5 ml Wasser zugegeben. Nun wird auf 60° erwärmt und nach 45 Minuten werden weitere 3,8 g Morpholin und 1,7 g NaOH in 5 ml Wasser zugegeben. Nach weiteren 1,5 Stunden ist die Reaktion beendet. Nach Abkühlen und Zufügen von 200 ml Toluol werden die Phasen getrennt. Die organische Phase wird über $Na_2SO_4$ getrocknet und eingedampft. Umkristallisieren aus Acetonitril führt zur Verbindung obiger Struktur, die bei 154-156° schmilzt (weisses Pulver).

| Analyse: | Ber.:<br>Gef.: | C = 54,33 %<br>C = 54,58 % | H = 7,09 %<br>H = 7,18 % | N = 17,60 %<br>N = 17,70 % |
|---|---|---|---|---|

Beispiel 45: 2-[Bis-(2-hydroxyethyl)-amino]-4-morpholino-6-(4,4-ethylendioxy-2,2,6,6-tetramethylpiperidin-1-yl)-1,3,5-triazin

Zu einer Lösung von 20 g 2-Chlor-4-morpholino-6-(4,4-ethylendioxy-2,2,6,6-tetramethylpiperidin-1-yl)-1,3,5-triazin (Produkt aus Beispiel 44) in 50 ml Xylol werden 5,3 g Diethanolamin und eine Lösung von 1 g NaOH in 3 ml Wasser gegeben. Unter Rühren am Rückfluss wird nach 1,75 Stunden nochmals 1 g NaOH in 3 ml Wasser zugegeben. Nach 14 Stunden werden 7,9 g Diethanolamin zugegeben, worauf noch während 28 Stunden am Rückfluss gerührt wird. Es wird abkühlen gelassen, mit Wasser gewaschen und die organische Phase abgetrennt. Nach Trocknen über $Na_2SO_4$, Eindampfen und zweimaligem Umkristallisieren aus Toluol erhält man obiges Produkt als weisses Pulver, das bei 135-140° schmilzt.

| | | | | |
|---|---|---|---|---|
| Analyse: | Ber.:<br>Gef.: | C = 56,63 %<br>C = 56,67 % | H = 8,21 %<br>H = 8,13 % | N = 18,01 %<br>N = 17,90 % |

Beispiel 46: Polykondensat aus 2-[Bis-(2-hydroxyethyl)-amino-4-morpholino-6-(4,4-ethylendioxy-2,2,6,6-tetramethylpiperidin-1-yl)-1,3,5-triazin und Diethylsuccinat

Zu einer Lösung von 9,8 g 2-[Bis-(2-hydroxyethyl)-amino]-4-morpholino-6-(4,4-ethylendioxy-2,2,6,6-tetramethylpiperidin-1-yl)-1,3,5-triazin (Produkt aus Beispiel 45) in 50 ml Toluol werden 3,5 ml Diethylsuccinat und 0,4 g Tetrabutyl-orthotitanat (monomer) gegeben. Das Gemisch wird während 24 Stunden so erhitzt, dass ganz langsam Toluol abdestilliert. Nach etwas abkühlen wird durch Bleicherde filtriert und eingedampft. Der Rückstand wird im Vakuum bei 80° getrocknet. Man erhält ein harzartiges Polymer vom Molekulargewicht $\overline{M}_n$ = 966/$\overline{M}_w$ = 1409 (Gelpermeationschromatographie).

Beispiel 47: 2,4-Bis-morpholino-6-(4-butylamino-2,2,6,6-tetramethylpiperidin-1-yl)-1,3,5-triazin

12 g 2,4-Bis-morpholino-6-(4-oxo-2,2,6,6-tetramethylpiperidin-1-yl)-1,3,5-triazin (Produkt aus Beispiel 21) werden in 120 ml Methanol und 60 ml Ethylacetat bei 40° und einem Druck von 80 bar mit 5 % Platin auf Kohle als Katalysator in Gegenwart von 6 g Butylamin und 0,25 g p-Toluolsulfonsäure bis zum Stillstand der Reaktion hydriert. Das Reaktionsgemisch wird filtriert und eingedampft. Nach Lösen in Methylenchlorid und Waschen mit Wasser wird die organische Phase über $Na_2SO_4$ getrocknet und eingedampft. Der Rückstand wird in Toluol/Aceton gelöst und an einer $SiO_2$-Säule chromatographisch gereinigt. Das ölige Produkt kristallisiert nach einigen Tagen und schmilzt bei 82-87°.

| | | | | |
|---|---|---|---|---|
| Analyse: | Ber.:<br>Gef.: | C = 62,44 %<br>C = 62,88 % | H = 9,39 %<br>H = 9,39 % | N = 21,24 %<br>N = 20,45 % |

Beispiel 48: Verbindung der Formel

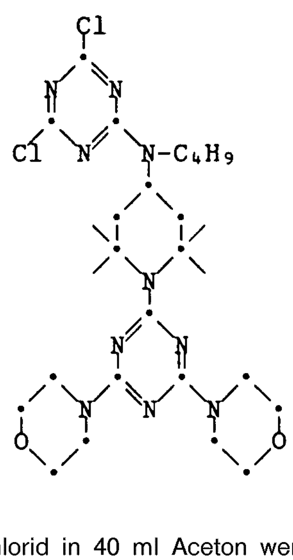

Zu einer Lösung von 3,2 g Cyanurchlorid in 40 ml Aceton werden unter Kühlen auf 0° 8 g 2,4-Bis-morpholino-6-(4-butylamino-2,2,6,6-tetramethylpiperidin-1-yl)-1,3,5-triazin (Produkt aus Beispiel 47) in 30 ml Aceton zugetropft. Nach Zugeben von 0,8 g NaOH in 2 ml Wasser wird 3 Stunden bei 0° gerührt,

worauf 60 ml Wasser zugegeben werden. Das dadurch ausgefällte Produkt wird abfiltriert und aus Acetonitril umkristallisiert. Die so erhaltenen weissen Kristalle obiger Struktur schmelzen bei 198-203 °C.

| Analyse: | Ber.:<br>Gef. : | C = 53,20 %<br>C = 53,32 % | H = 6,94 %<br>H = 6,93 % | N = 22,98 %<br>N = 22,92 % |
|---|---|---|---|---|

Beispiel 49: Verbindung der Formel

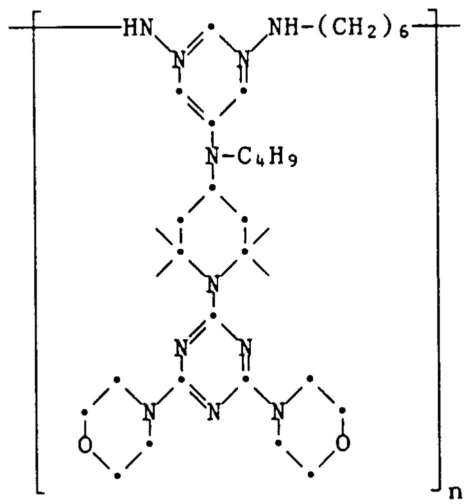

Polykondensat aus Verbindung aus Beispiel 48 und Hexamethylendiamin Verwendet man an Stelle des im Beispiel 14 beschriebenen 2,4-Dichlor-6-(2,2,6,6-tetramethylpiperidin-1-yl)-1,3,5-triazins die in Beispiel 48 beschriebene Dichlorverbindung in moläquivalenter Menge und verfährt im übrigen gleich wie unter Beispiel 14 beschrieben, so erhält man ein pulverisierbares Polymer vom Molekulargewicht $\overline{M}_n$ = 1520/$\overline{M}_w$ = 1985 (Gelpermeationschromatographie).

Beispiel 50: 2,4-Bis-morpholino-6-(4-methacryloyloxy-2,2,6,6-tetramethylpiperidin-1-yl)-1,3,5-triazin

10,2 g 2,4-Dimorpholino-6-(4-hydroxy-2,2,6,6-tetramethylpiperidin-1-yl)-1,3,5-triazin (Produkt aus Beispiel 34) werden zusammen mit 6,9 ml Methacrylsäureethylester, 0,04 g 2,6-Bis-tert.butyl-p-kresol und 0,04 ml Tetrabutyl-orthotitanat (monomer) auf 120° erhitzt, wobei langsam etwas Flüssigkeit abdestilliert wird. Es wird während 50 Stunden reagieren gelassen, wobei zeitweise etwas Methacrylsäureethylester und Katalysator nachgegeben werden. Nach beendeter Reaktion wird durch Bleicherde filtriert und eingedampft. Der Rückstand wird in Toluol/Aceton gelöst und an einer $SiO_2$-Säule chromatographisch gereinigt, wobei obiges Produkt als farbloses Harz erhalten wird.

| Analyse: | Ber.:<br>Gef.: | C = 60,74 %<br>C = 60,60 % | H = 8,07 %<br>H = 8,10 % | N = 17,71 %<br>N = 17,10 % |
|---|---|---|---|---|

Beispiel 51: Homopolymer von 2,4-Bis-morpholino-6-(4-methacryloyloxy-2,2,6,6-tetramethylpiperidin-1-yl)-1,3,5-triazin

6 g 2,4-Bis-morpholino-6-(4-methacryloyloxy-2,2,6,6-tetramethylpiperidin-1-yl)-1,3,5-triazin (Produkt aus Beispiel 50) werden zusammen mit 0,13 g Dodecylmercaptan und 0,1 g $\alpha,\alpha'$-Azo-isobutyronitril in 25 ml Isopropylmethylketon unter Stickstoff während 12 Stunden auf 75° erhitzt. Nach Eindampfen und Trocknen während 72 Stunden bei 60° unter Vakuum erhält man ein weisses, pulverisierbares Polymer vom Molekulargewicht $\overline{M}_n$ = 3188/$\overline{M}_w$ = 12064 (Gelpermeationschromatographie).

Beispiel 52: N,N'-Bis[1-(2,4-dimorpholino-1,3,5-triazin-6-yl)-2,2,6,6-tetramethylpiperidin-4-yl]-hexamethylendiamin

Verwendet man an Stelle des im Beispiel 47 eingesetzten Butylamins eine moläquivalente Menge 1,6-Diaminohexan und verfährt im übrigen gleich wie im Beispiel 47 angegeben, so erhält man die obige Verbindung als weisses Pulver, das bei 161-163° schmilzt.

| Analyse: | Ber.: | C = 61.85 % | H = 9.03 % | N = 21.95 % |
|---|---|---|---|---|
| | Gef.: | C = 61.68 % | H = 8.81 % | N = 21.43 % |

Beispiel 53: 2-Chlor-4,6-bis-(2,2,6,6-tetramethylpiperidin-1-yl)-1,3,5-triazin

18,4 g Cyanurchlorid und 113 g 2,2,6,6-Tetramethylpiperidin werden in einem 300 ml Autoklaven 10 Stunden bei 180° und dann 10 Stunden bei 210° erhitzt. Der Autoklaveninhalt wird in 500 ml Wasser aufgenommen, der unlösliche Rückstand abgenutscht, mit Wasser gewaschen und getrocknet. Der bräunliche Rückstand wird aus Ligroin kristallisiert. Man erhält das 2-Chlor-4,6-bis-(2,2,6,6-tetramethylpiperidin-1-yl)-1,3,5-triazin als farblose Kristalle mit einem Schmelzpunkt von 188°.

Beispiel 54: 2,4-Dichlor-6-[2,2,6,6-tetramethyl-4-(N-acetyl-butylamino)-piperidin-1-yl)-1,3,5-triazin

9,2 g Cyanurchlorid und 26,7 g 2,2,6,6-Tetramethyl-4-(N-acetyl-butylamino)-piperidin werden in 100 ml Xylol wie in Beispiel 1 beschrieben umgesetzt und aufgearbeitet. Man erhält das 2,4-Dichlor-6-[2,2,6,6-tetramethyl-4-(N-acetyl-butylamino)-piperidin-1-yl)-1,3,5-triazin als farblose Kristalle mit einem Schmelzpunkt von 131-133°.

Beispiel 55: 2-Chlor-4-isopropyloxy-6-(2,2,6,6-tetramethylpiperidin-1-yl)-1,3,5-triazin

104,0 g 2,4-Dichlor-6-isopropyloxy-1,3,5-triazin und 148,3 g 2,2,6,6-Tetramethylpiperidin werden in 300 ml Xylol wie in Beispiel 1 beschrieben umgesetzt und aufgearbeitet. Durch Kristallisation aus Hexan erhält man das 2-Chlor-4-isopropyloxy-6-(2,2,6,6-tetramethylpiperidin-1-yl)-1,3,5-triazin als farblose Kristalle mit einem Schmelzpunkt von 111-112°.

Beispiel 56: 2,4-Bis-isopropyloxy-6-(2,2,6,6-tetramethylpiperidin-1-yl)-1,3,5-triazin

12,5 g 2-Chlor-4-isoproplyoxy-6-(2,2,6,6-tetramethylpiperidin-1-yl)-1,3,5-triazin (Produkt aus Beispiel 55) 11,2 g fein pulverisiertes Kaliumhydroxid und 0,7 g Tetrabutylammoniumhydrogensulfat werden in 60 ml Toluol vorgelegt. Zu dieser orange gefärbten Suspension tropft man in 15 Minuten 2,6 g Isopropanol: schwache Exothermie bis ca. 30°. Der hellbraune Kolbeninhalt wird 8 Stunden bei 60° gerührt, auf 0-5° abgekühlt, mit 80 ml Wasser und dann mit 40 ml Toluol verdünnt. Die braune, wässrige Phase wird von der farblosen, organischen Phase abgetrennt und diese viermal mit je 80 ml Wasser gewaschen, über Natriumsulfat getrocknet und im Vakuum vollständig eingedampft. Man erhält ein schwach gelbliches Oel, das nach kurzer Zeit zum 2,4-Bis-isopropyloxy-6-(2,2,6,6-tetramethylpiperidin-1-yl)-1,3,5-triazin mit Schmelzbereich 70-98° erstarrt.

Beispiel 57: 2-Isopropyloxy-4-n-octoxy-6-(2,2,6,6-tetramethylpiperidin-1-yl)-1,3,5-triazin

Verwendet man anstelle von Isopropanol 5,7 g 1-Octanol und verfährt man wie in Beispiel 56 beschrieben, so erhält man das 2-Isopropyloxy-4-n-octoxy-6-(2,2,6,6-tetramethylpiperidin-1-yl)-1,3,5-triazin als schwach gelbliches Harz

| Analyse: | ber.: | 13,78 % N, | gef. | 13,87 % N |
|---|---|---|---|---|

Beispiel 58: 2-Isopropyloxy-4-dibutylamino-6-(2,2,6,6-tetramethylpiperidin-1-yl)-1,3,5-triazin

12,5 g 2-Chlor-4-isopropyloxy-6-(2,2,6,6-tetramethylpiperidin-1-yl)-1,3,5-triazin und 5,5 g Dibutylamin werden in 100 ml Xylol gelöst. Nach der Zugabe einer Lösung von 1,8 g Natriumhydroxid in 10 ml Wasser wird am Wasserabscheider unter schwachem Stickstoffstrom das Wasser langsam abdestilliert. Der Kolbeninhalt wird anschliessend ca. 16 Stunden bei 135° gerührt. Man lässt etwas abkühlen, gibt zum Kolbeninhalt 50 ml Wasser und rührt kräftig während 10 Minuten. Die wässrige Phase wird abgetrennt und die organische Schicht viermal mit je 50 ml Wasser gewaschen, über Natriumsulfat getrocknet und im Vakuum vollständig eingedampft. Das erhaltene 2-Isopropyloxy-4-dibutylamino-6-(2,2,6,6-tetramethylpiperidin-1-yl)-1,3,5-triazin ist ein schwach gelbliches Harz.

| Analyse N: | ber. | 17,27 %, | gef. | 17,29 % |
|---|---|---|---|---|

Beispiel 59: Verbindung der Formel

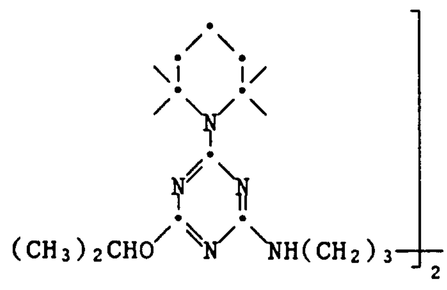

18,8 g 2-Chlor-4-isopropyloxy-6-(2,2,6,6-tetramethylpiperidin-1-yl)-1,3,5-triazin, 3,6 g 1,6-Diaminohexan und eine Lösung von 2,6 g Natriumhydroxid in 10 ml Wasser werden in 100 ml Xylol wie in Beispiel 58 beschrieben umgesetzt. Durch Kristallisation aus Xylol erhält man die Verbindung obiger Formel als farblose Kristalle mit einem Schmelzpunkt von 224-226°.

Beispiel 60: 2,4-Bis-(2-hydroxyethylamino)-6-(2,2,6,6-tetramethylpiperidin-1-yl)-1,3,5-triazin

Verwendet man anstelle von Dibutylamin 26,9 g Ethanolamin (1. Portion: 12,2 g; 2. Portion: 14,7 g) und verfährt man im übrigen wie in Beispiel 6 beschrieben, so erhält man, nach Kristallisation aus Toluol, das 2,4-Bis-(2-hydroxyethylamino)-6-(2,2,6,6-tetramethylpiperidin-1-yl)-1,3,5-triazin mit einem Schmelzpunkt von 147-148°.

Beispiel 61: Polymeres der Formel

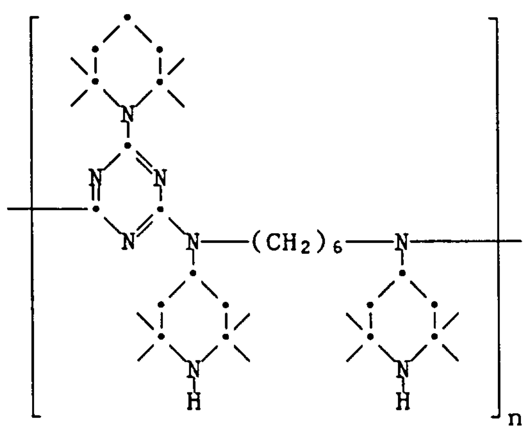

23,1 g 2,4-Dichlor-6-(2,2,6,6-tetramethylpiperidin-1-yl)-1,3,5-triazin und 31,4 g 1,6-Bis-(2,2,6,6-tetramethyl-4-piperidylamino)-hexan werden in Gegenwart von 6,8 g Natriumhydroxid in 200 ml Xylol wie in Beispiel 6 beschrieben umgesetzt. Das erhaltene, leicht gelbliche Harz weist ein Molekulargewicht von 1330 auf.

Beispiel 62: Verbindung der Formel

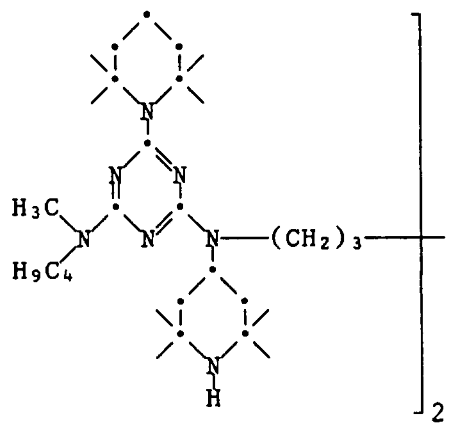

57,8 g 2,4-Dichlor-6-(2,2,6,6-tetramethylpiperidin-1-yl)-1,3,5-triazin werden in 200 ml Xylol vorgelegt. Dazu tropft man in 15 Minuten 17,4 g N-Methyl-butylamin. Die Temperatur steigt auf 50°. Nun tropft man in 10 Minuten eine Lösung von 8,8 g Natriumhydroxid in 30 ml Wasser zum Reaktionsgemisch und rührt 2 Stunden bei 60°. Man trennt dann die wässrige Phase ab, gibt 41,4 g 1,6-Bis-(2,2,6,6-tetramethyl-4-piperidylamino)-hexan hinzu, erwärmt auf 90° und fügt dann noch eine Lösung von 9,6 g Natriumhydroxid in 30 ml Wasser hinzu. Unter schwachem Stickstoffstrom wird nun das Wasser am Wasserabscheider langsam abdestilliert und der Kolbeninhalt dann 18 Stunden bei 135° gerührt. Man lässt etwas abkühlen, gibt 100 ml Wasser zum Reaktionsgemisch und rührt kräftig während 10 Minuten. Die wässrige Phase wird abgetrennt und die organische Lösung dreimal mit je 50 ml Wasser gewaschen, über Natriumsulfat getrocknet und im Vakuum eingedampft. Durch Kristallisation des Rückstandes aus Methylethylketon erhält man die Verbindung obiger Formel als farblose Kristalle mit einem Schmelzpunkt von 137-138°.

Beispiel 63: Verbindung der Formel

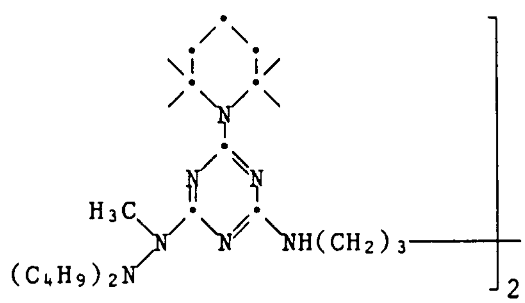

Setzt man 28,9 g 2,4-Dichlor-6-(2,2,6,6-tetramethylpiperidin-1-yl)-1,3,5-triazin mit 12,9 g Dibutylamin und dann mit 1,6 g Diaminohexan wie in Beispiel 62 beschrieben um, so erhält man nach Kristallisation aus Acetonitril die Verbindung obiger Formel als farblose Kristalle mit einem Schmelzpunkt von 93-94°.

Beispiel 64: Verbindung der Formel

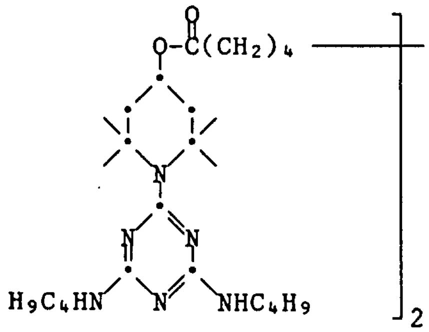

19,0 g 2,4-Bis-butylamino-6-(2,2,6,6-tetramethyl-4-hydroxypiperidin-1-yl)-1,3,5-triazin (hergestellt nach Beispiel 9) werden mit 5,8 g Dimethylsebacat in 150 ml Xylol nach der Zugabe von 0,2 g Lithiumamid unter schwachem Stickstoffstrom und Abdestillieren des gebildeten Methanols 12 Stunden am Rückfluss erhitzt. Man lässt das Reaktionsgemisch auf ca. 100° abkühlen, versetzt mit 5 g Tonsil optimum (Bleicherde), rührt 5 Minuten und filtriert. Durch Eindampfen des Lösungsmittels erhält man die Verbindung der obigen Formel als leicht gelbes Harz.

| | | | | |
|---|---|---|---|---|
| Analyse: | ber. | 18,20 % N, | gef. | 18,51 % N |

Beispiel 65: Verbindung der Formel

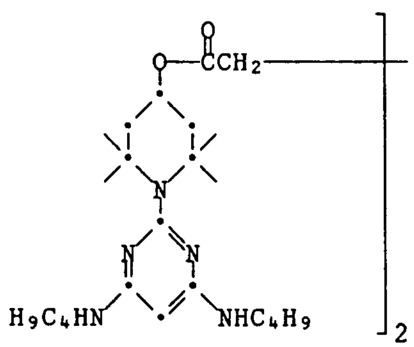

Verwendet man anstelle von Dimethylsebacat 3,6 g Dimethylsuccinat und verfährt wie in Beispiel 64 beschrieben, so erhält man nach Kristallisation aus Acetonitril die Verbindung obiger Formel als farblose Kristalle mit einem Schmelzpunkt von 131°.

Beispiel 66: Verbindung der Formel

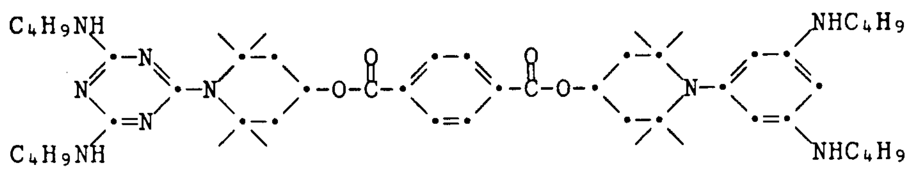

Verwendet man anstelle von Dimethylsebacat 4,8 g Dimethylterephthalat und verfährt wie in Beispiel 64 beschrieben, so erhält man nach Kristallisation aus Xylol die Verbindung obiger Formel als farblose Kristalle mit einem Schmelzpunkt von 181°.

Beispiel 67: 2,4-Bis-butylamino-6-{2,2,6,6-tetramethyl-4-[2-(3,5-di-tert.-butyl-4-hydroxyphenyl)-propionyloxy]-piperidin-1-yl}-1,3,5-triazin

Verwendet man anstelle von Dimethylsebacat 14,6 g 2-(3,5-Di-tert.-butyl-4-hydroxyphenyl)-propion-säuremethylester und verfährt wie in Beispiel 64 beschrieben, so erhält man das 2,4-Bis-butylamino-6-{2,2,6,6-tetramethyl-4-[2-(3,5-di-tert.-butyl-4-hydroxyphenyl)-propionyloxy]-piperidin-1-yl}-1,3,5-triazin als schwach gelbliches Harz.

| | | | | |
|---|---|---|---|---|
| Analyse: | ber. | 13,15 % N, | gef. | 13,36 % N |

Beispiel 68: Verbindung der Formel

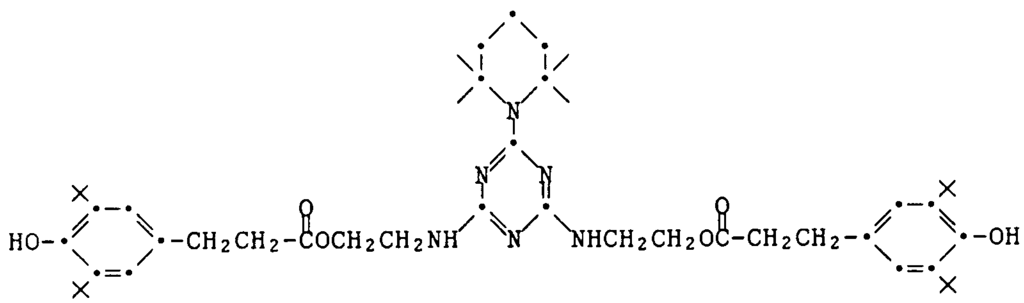

16,9 g 2,4-Bis-(2-hydroxyethylamino)-6-(2,2,6,6-tetramethylpiperidin-1-yl)-1,3,5-triazin (hergestellt nach Beispiel 60) werden mit 29,2 g β-(3,5-Di-tert.-butyl-4-hydroxyphenyl)-propionsäuremethylester in 100 ml Xylol nach der Zugabe von 0,2 g Lithiumamid unter schwachem Stickstoffstrom und Abdestillieren des gebildeten Methanols 12 Stunden am Rückfluss erhitzt. Man lässt das Reaktionsgemisch auf ca. 100° abkühlen, versetzt mit 5 g Tonsil Optimum (Bleicherde), rührt 5 Minuten und filtriert. Nach Eindampfen des Lösungsmittels erhält man die Verbindung der obigen Formel als gelbliches Harz

| | | | | |
|---|---|---|---|---|
| Analyse: | ber. | 9,78 % N, | gef. | 9,67 % N. |

Beispiel 69: Verbindung der Formel

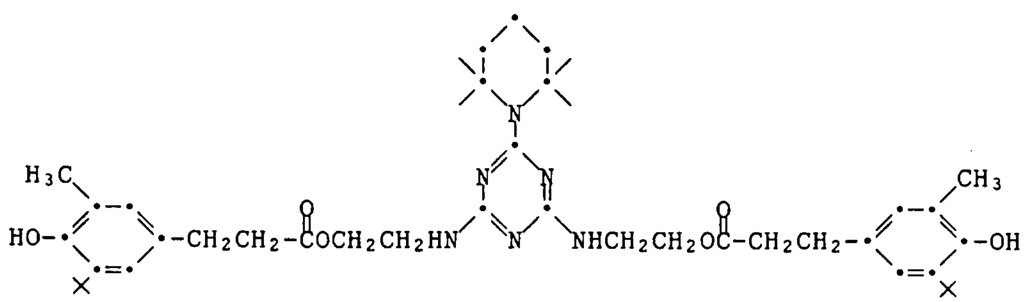

Verwendet man anstelle von β-(3,5-Di-tert.-butyl-4-hydroxyphenyl)-propionsäuremethylester 25,0 g β-(3-Methyl-4-hydroxy-5-tert.butylphenyl)-propionsäuremethylester und verfährt man wie in Beispiel 68 beschrieben, so erhält man die Verbindung der obigen Formel als gelbliches, pulverisierbares Harz.

| | | | | |
|---|---|---|---|---|
| Analyse: | ber. | 10,84 % N, | gef. | 10,57 % N. |

Beispiel 70: 2-Chlor-4-[N-(2,2,6,6-tetramethyl-4-piperidyl)-n-butylamino]-6-(2,2,6,6-tetramethyl-4-hexyloxypiperidin-1-yl)-1,3,5-triazin

Setzt man das Dichlorderivat aus Beispiel 10 mit einem Aequivalent 4-Butylamino-2,2,6,6-tetramethylpiperidin um und verfährt dabei wie im Beispiel 26 beschrieben, so erhält man die Titelverbindung als farbloses Oel.

| Analyse: | ber. | 14,87 % N, | gef. | 14,74 % N. |
|---|---|---|---|---|

Beispiel 71: N,N',N"-Tris{2-(2,2,6,6-tetramethyl-4-hexyloxypiperidin-1-yl)-4-[N-(2,2,6,6-tetramethylpiperidin-4-yl)-butylamino]-1,3,5-triazin-6-yl}-diethylentriamin
Zu 10,6 g des Monochlorderivates aus Beispiel 70, gelöst in 50 ml Toluol, werden 0,71 ml Diethylentriamin und 0,9 g NaOH, gelöst in 3 ml Wasser, zugegeben. Nach Erwärmen auf 100° beginnt das Lösungsmittel abzudestillieren. Man gibt weitere 50 ml Toluol sowie 1 g Polyethylenglykol 1000 und 0,9 g NaOH zu und erwärmt unter Rühren 14 h unter Rückfluss. Nach dem Abkühlen verdünnt man das Reaktionsgemisch mit 100 ml Ethylacetat und wäscht die Lösung viermal mit Wasser. Die organische Lösung wird über $Na_2SO_4$ getrocknet und eingedampft. Der Rückstand wird in 50 ml Methanol gelöst, über 200 g Silicagel filtriert und mit 500 ml Methanol gewaschen. Die Methanol-Lösung wird eingedampft, der Rückstand ist ein beinahe farbloses Harz.

| Analyse: | ber. | 17,4 % N, | gef. | 17,4 % N. |
|---|---|---|---|---|

Beispiel 72: Stabilisierung eines Zweischicht-Lackes
Es wird ein Klarlack bereitet durch Mischen der folgenden Komponenten:
58,3 Teile eines Acrylatharzes (Viacryl®VC 373, Vianova AG)
27,3 Teile eines Melaminharzes (Maprenal®MF 590, Hoechst AG)
4,0 Teile eines aromatischen Lösungsmittelgemisches (Solvesso®150)
5,4 Teile Xylol
4,0 Teile Butylglykolacetat
1,0 Teile eines Verlaufhilfsmittels (Baysilon®A, Bayer AG)
Diesem Lack werden die in Tabelle 1 aufgeführten Lichtschutzmittel zugegeben. Der Lack wird mit einem 1:1:1-Gemisch von Butylacetat und Xylol auf Spritzfähigkeit verdünnt und auf ein mit einem Silbermetallic Basislack lackierten Aluminiumblech gespritzt. Die Proben werden dann 30 Minuten bei 130°C gehärtet. Es resultiert eine Schichtdicke des Klarlackes von 40-45 µm.

Die so bereiteten Proben werden in einem UVCON®-Belichtungsgerät (Atlas Corp.) bewittert bei einem Zyklus von 8 h UV-Bestrahlung bei 70°C und 4 h Kondensation bei 50°C. Nach jeweils 400 h Bewitterung wird der 20°-Glanz der Proben nach DIN 67530 gemessen. Die Ergebnisse sind in Tabelle 1 aufgeführt.

Tabelle 1

| Lichtschutzmittel[1] | 20°-Glanz nach | | | |
|---|---|---|---|---|
| | 0 | 400 | 800 | 1200 h |
| keines | 85 | 83 | 19[2] | |
| 1 % Beispiel 5 | 86 | 84 | 67 | 28[2] |
| 1 % Beispiel 6 | 87 | 85 | 75 | 43 |
| 1 % Beispiel 7 | 88 | 86 | 74 | 40 |
| 1 % Beispiel 13 | 86 | 85 | 78 | 31 |
| 1 % Beispiel 24 | 87 | 86 | 80 | 35 |

[1] Mengenangabe bezogen auf den Feststoffgehalt des Lackes
[2] Rissbildung

Beispiel 73: - Freibewitterung eines Zweischicht-Lackes
Es werden Zweischicht-Lackierungen bereitet wie in Beispiel 70 beschrieben. Die Proben werden einer Freibewitterung in Florida von 54 Monaten ausgesetzt. Alle 12 Monate wird der 20°-Glanz nach DIN 67530 gemessen. Die Ergebnisse sind in Tabelle 2 aufgeführt.

Tabelle 2

| Lichtschutzmittel[1)] | 20°-Glanz nach | | | | | |
|---|---|---|---|---|---|---|
| | 0 | 12 | 24 | 36 | 48 | 54 Monaten |
| keines | 93 | 70 | 49 | 36[2)] | 16 | -- |
| 1 % Beispiel 4 | 94 | 73 | 76 | 69 | 57 | 53[2)] |
| 1 % Beispiel 5 | 95 | 72 | 73 | 69 | 64 | 67 |

[1)] Mengenangabe bezogen auf Feststoffgehalt des Lackes
[2)] Rissbildung

Beispiel 74: 0,087 g des Gelbkupplers der Formel

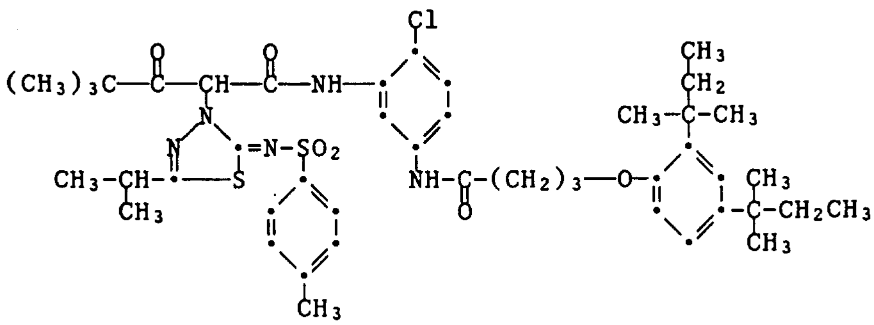

werden in 2,0 ml einer Lösung des in Tabelle 3 aufgeführten Stabilisators in Ethylacetat (2,25 g/100 ml) gelöst. Zu 1,0 ml dieser Lösung gibt man 9,0 ml einer 2,3%-igen wässrigen Gelatinelösung, die auf einem pH-Wert von 6,5 eingestellt ist, und 1,744 g/l des Netzmittels der Formel

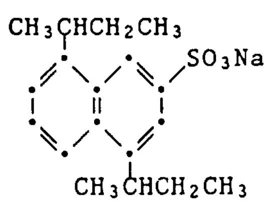

enthält.

Zu 5,0 ml der so erhaltenen Kuppleremulsion gibt man 2 ml einer Silberbromid-Emulsion mit einem Silbergehalt von 6,0 g/l sowie 1,0 ml einer 0,7%-igen wässrigen Lösung des Härters der Formel

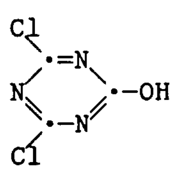

und vergiesst es auf ein 13 x 18 cm Kunststoff-beschichtetes Papier. Nach einer Härtungszeit von 7 Tagen werden die Proben hinter einem Silber-Stufenkeil mit 125 Lux•s belichtet und anschliessend im Ektaprint 2®-Prozess der Firma Kodak verarbeitet.

Die erhaltenen Gelbkeile werden in einem Atlas Weather-Ometer mit einer 2500 W-Xenonlampe hinter einem UV-Filter (Kodak 2C) mit total 60 k Joule/cm$^2$ bestrahlt.

Eine Probe ohne Stabilisator läuft als Standard mit.

Die folgende Tabelle 3 gibt den bei der Bestrahlung eingetretenen Farbdichteverlust beim Absorptionsmaximum des gelben Farbstoffes, gemessen mit einem Densitometer TR 924A der Fa. Macbeth,

wieder.

Der Lichtschutzeffekt ist aus dem Farbdichteverlust ersichtlich. Je kleiner der Dichteverlust, desto höher ist die Lichtschutzwirksamkeit.

Tabelle 3

| Stabilisator | Farbdichteverlust (in %) |
|---|---|
| keiner | 35 |
| Produkt von Bespiel 68 | 18 |
| Produkt von Beispiel 69 | 14 |

Beispiel 75: Je 0,033 g des Cyan-Kupplers der Formel

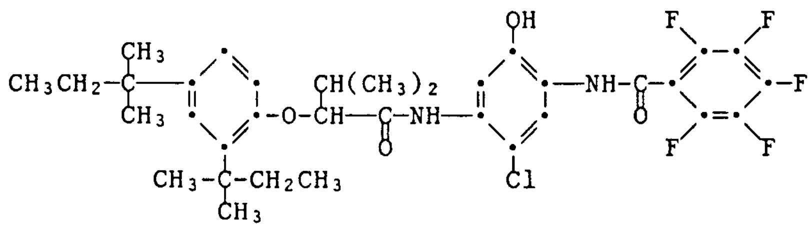

und des in Tabelle 4 angegebenen Stabilisators werden in 2,0 ml eines Gemisches von Dibutylphthalat/Ethylacetat (0,8 g/100 ml) gelöst.

Zu 1,0 ml dieser Lösung gibt man 9,0 ml einer 2,3%-igen wässrigen Gelatinelösung, die auf einen pH-Wert von 6,5 eingestellt ist, und 0,872 g/l des Netzmittels Natrium-dibutylnaphthalinsulfonat enthält.

Im weiteren verfährt man mit der Emulsion wie in Beispiel 72 beschrieben, jedoch mit dem Unterschied, dass die Silberbromid-Emulsion einen Silbergehalt von 3 g/l aufweist.

Die erhaltenen Farbstufenkeile werden in einem Atlas Weather-Ometer mit einer 2500 W-Xenonlampe hinter einem UV-Filter (Kodak 2c) mit insgesamt 60 k $Joule/cm^2$ bestrahlt und anschliessend der Farbdichteverlust, wie in Beispiel 72 beschrieben, bestimmt.

Die Ergebnisse sind in der nachfolgenden Tabelle 4 zusammengefasst.

Tabelle 4

| Stabilisator | Farbdichteverlust (in %) |
|---|---|
| keiner | 43 |
| Produkt von Beispiel 68 | 22 |
| Produkt von Beispiel 69 | 23 |

## Patentansprüche

**1.** Eine Verbindung der Formel II,

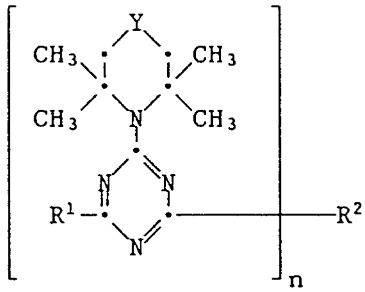

II

worin n eine ganze Zahl von 1 bis 6 bedeutet,
$R^1$ einen Rest der Formel

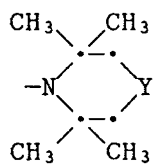

oder Cl, OH, $-OR^3$, $-SR^3$ oder $-NR^4R^5$ bedeutet, wobei $R^3$ $C_1$-$C_{18}$-Alkyl, Allyl, Cyclohexyl, Benzyl, Phenyl oder eine Gruppe der Formel A bedeutet,

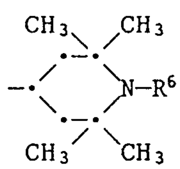

A

$R^4$ Wasserstoff, $C_1$-$C_{12}$-Alkyl, 2-Hydroxyethyl, Allyl, Cyclohexyl, Benzyl oder eine Gruppe der Formel A bedeutet,
$R^5$ $C_1$-$C_{12}$-Alkyl, 2-Hydroxyethyl, Allyl, Cyclohexyl, Benzyl, Phenyl, durch Halogen, $C_1$-$C_4$-Alkyl oder $C_1$-$C_4$-Alkoxy substituiertes Phenyl, eine Gruppe der Formel A oder eine Gruppe der Formel C bedeutet,

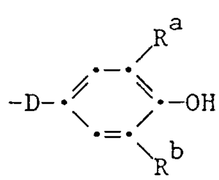

C

worin D durch

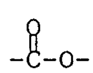

unterbrochenes $C_2$-$C_{20}$-Alkylen bedeutet, $R^a$ und $R^b$ $C_1$-$C_{12}$-Alkyl, $C_5$-$C_6$-Cycloalkyl, $C_6$-$C_{10}$-Aryl oder $C_7$-$C_9$-Phenylalkyl bedeuten und $R^a$ auch Wasserstoff bedeutet, oder
$R^4$ und $R^5$ zusammen $C_4$-$C_8$-Alkylen bedeuten, das durch -O- oder $-N(R^8)$-unterbrochen sein kann und worin $R^8$ Wasserstoff, $C_1$-$C_4$-Alkyl oder Acetyl bedeutet,
$R^6$ Wasserstoff, $C_1$-$C_{12}$-Alkyl, $C_7$-$C_9$-Phenylalkyl, $C_3$-$C_5$-Alkenyl, $C_2$-$C_4$-Alkanoyl, $C_3$-$C_5$-Alkenoyl, -O•,

-OH oder $-OR^7$ bedeutet und
$R^7$ $C_1$-$C_{18}$-Alkyl, $C_5$-$C_8$-Cycloalkyl, $C_7$-$C_9$-Phenylalkyl, Phenyl, $C_2$-$C_{18}$-Alkanoyl oder Benzoyl bedeutet,
$R^2$, wenn n = 1 ist, Cl, OH, $-OR^3$, $-SR^3$ oder $-NR^4R^5$ bedeutet,
wenn n = 2 ist, eine Gruppe $-O-R^9-O-$, $-S-R^9-S-$, $-N(R^{10})-R^9-N(R^{10})-$, $-O-R^9-N(R^{10})-$,

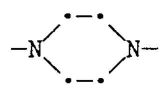

oder -NH-NH- bedeutet, wobei
$R^9$ $C_2$-$C_{20}$-Alkylen, das durch ein oder mehrere -O-, $-N(R^8)-$ oder $-OOC-R^{17}-COO-$ unterbrochen sein kann, $C_4$-$C_8$-Alkenylen, $C_5$-$C_8$-Cycloalkylen, Xylylen, Phenylen oder Tolylen bedeutet,
$R^{10}$ Wasserstoff, $C_1$-$C_{12}$-Alkyl, Allyl, 2-Hydroxyethyl, Benzyl, Phenyl oder eine Gruppe der Formel A bedeutet,
wenn n = 3 ist, eine Gruppe

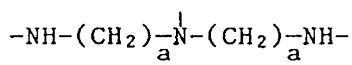 oder 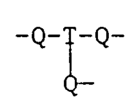

bedeutet, worin
a 2 oder 3 ist, Q -O-, -S- oder $-N(R^{10})-$ ist und T $C_3$-$C_{20}$-Alkantriyl oder eine Gruppe

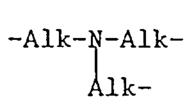 oder 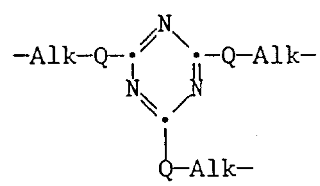

bedeutet, worin Alk eine $C_2$-$C_{12}$-Alkylengruppe ist,
wenn n = 4 ist, eine Gruppe $C(CH_2O-)_4$ oder

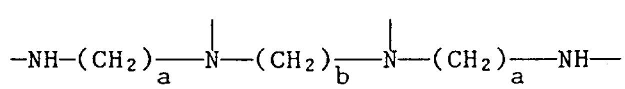

bedeutet, worin
a 2 oder 3 ist und b 2-12 ist,
wenn n = 5 ist, eine Gruppe

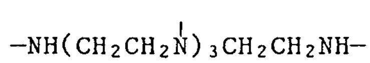

bedeutet, und wenn n = 6 ist, eine Gruppe

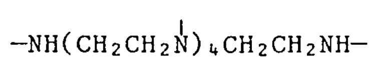

bedeutet,
Y eine Gruppe

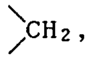, 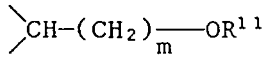, 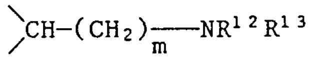, 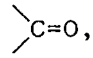,

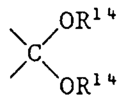, 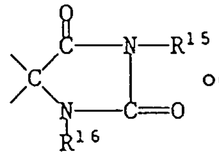 oder 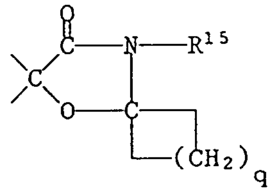

bedeutet,

worin m 0, 1 oder 2 ist, q eine ganze Zahl von 5 - 11 ist,

$R^{11}$ Wasserstoff, $C_1$-$C_{18}$-Alkyl, $C_3$-$C_7$-Alkenyl, $C_5$-$C_8$-Cycloalkyl, $C_7$-$C_{11}$-Aralkyl oder eine Gruppe -CO-$R^{18}$ bedeutet,

$R^{12}$ Wasserstoff, $C_1$-$C_{12}$-Alkyl, $C_3$-$C_7$-Alkenyl, $C_5$-$C_8$-Cycloalkyl, $C_7$-$C_{11}$-Aralkyl, $C_2$-$C_4$-Hydroxyalkyl, $C_3$-$C_8$-Alkoxyalkyl, $C_4$-$C_{20}$-Dialkylaminoalkyl, $C_3$-$C_{14}$-Alkoxycarbonylalkyl oder eine Gruppe der Formel A bedeutet,

$R^{13}$ $C_1$-$C_{12}$-Alkyl, $C_2$-$C_4$-Hydroxyalkyl, $C_3$-$C_7$-Alkenyl, $C_5$-$C_8$-Cycloalkyl, Phenyl, durch Halogen, $C_1$-$C_4$-Alkyl oder $C_1$-$C_4$-Alkoxy substituiertes Phenyl, $C_2$-$C_{20}$-Alkanoyl, $C_3$-$C_8$-Alkenoyl, Benzoyl, Phenylacetyl oder einen Triazinylrest der Formel B

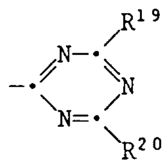 B

bedeutet, oder

$R^{12}$ und $R^{13}$ zusammen $C_4$-$C_8$-Alkylen bedeuten, das durch -O- oder -N($R^8$)-unterbrochen sein kann oder $R^{12}$ und $R^{13}$ zusammen einen Rest der Formel

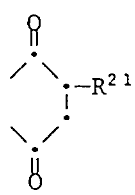

bedeuten, worin $R^{21}$ $C_1$-$C_{18}$-Alkyl bedeutet,

$R^{14}$ $C_1$-$C_{18}$-Alkyl, $C_5$-$C_8$-Cycloalkyl oder $C_7$-$C_9$-Phenylalkyl bedeutet oder beide Gruppen $R^{14}$ zusammen $C_2$-$C_6$-Alkylen, o-Phenylen oder o-Xylylen bedeuten,

$R^{15}$ Wasserstoff, $C_1$-$C_{12}$-Alkyl, $C_3$-$C_5$-Alkenyl, $C_7$-$C_9$-Phenylalkyl, $C_2$-$C_4$-Hydroxyalkyl, $C_3$-$C_8$-Alkoxyalkyl oder $C_3$-$C_{14}$-Alkoxycarbonylalkyl bedeutet,

$R^{16}$ Wasserstoff, $C_1$-$C_{12}$-Alkyl, Allyl oder Benzyl bedeutet,

$R^{17}$ $C_1$-$C_{12}$-Alkylen, Vinylen, Cyclohexylen, Xylylen oder $C_6$-$C_{12}$-Arylen bedeutet,

$R^{18}$ $C_1$-$C_{18}$-Alkyl, $C_2$-$C_6$-Alkenyl, $C_5$-$C_8$-Cycloalkyl, $C_7$-$C_9$-Phenylalkyl, Phenyl oder durch Halogen, Nitro, $C_1$-$C_4$-Alkyl, Hydroxy oder $C_1$-$C_4$-Alkoxy substituiertes Phenyl oder durch Hydroxy und $C_1$-$C_4$-Alkyl substituiertes $C_7$-$C_9$-Phenylalkyl bedeutet,

$R^{19}$ eine der für $R^1$ gegebenen Bedeutungen hat, und

$R^{20}$ Cl, -OH, -$OR^3$, -$SR^3$ oder -$NR^4R^5$ bedeutet, mit

Ausnahme von 2-(2,2,6,6-Tetramethyl-1-piperidino)-4,6-bis(2,4,4,-trimethyl-2-pentylamino)-triazin und 2,4-Dichlor-6-(2,2,6,6-tetramethyl-1-piperidino)-triazin.

**2.** Eine Verbindung der Formel III,

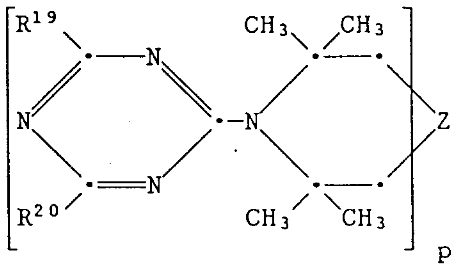

III

worin p 2, 3 oder 4 ist,
$R^{19}$ und $R^{20}$ die in Anspruch 1 gegebenen Bedeutungen haben und
Z, wenn p = 2 ist, eine der Gruppen

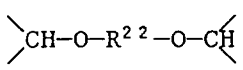 , 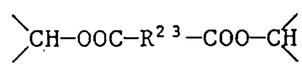 , 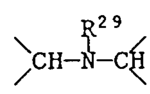 ,

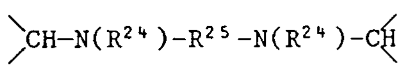 , 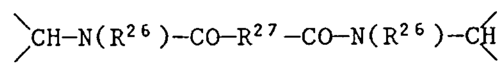 ,

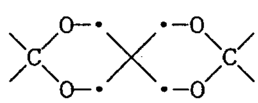 , 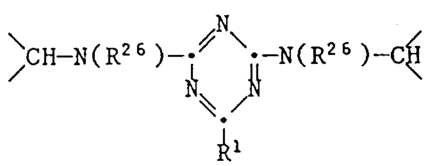 , 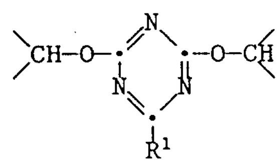 ,

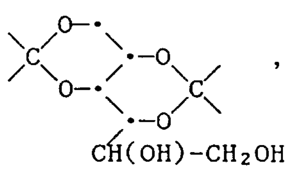 , 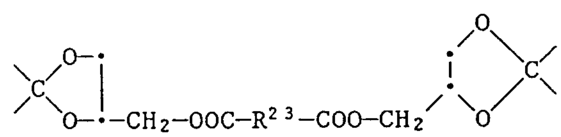

bedeutet, worin
$R^{22}$ $C_2$-$C_{12}$-Alkylen, $C_4$-$C_8$-Alkenylen, Xylylen oder -CO- bedeutet,
$R^{23}$ $C_1$-$C_{12}$-Alkylen, Vinylen, Cyclohexylen, Xylylen, $C_6$-$C_{12}$-Arylen oder durch Halogen, Nitro oder $C_1$-$C_4$-Alkyl substituiertes Phenylen oder eine direkte Bindung bedeutet,
$R^{24}$ Wasserstoff, $C_1$-$C_{12}$-Alkyl, $C_5$-$C_8$-Cycloalkyl, $C_7$-$C_9$-Phenylalkyl, $C_3$-$C_7$-Alkenyl, $C_2$-$C_{18}$-Alkanoyl, $C_3$-$C_7$-Alkenoyl, oder Benzoyl bedeutet, $R^{25}$ $C_2$-$C_{12}$-Alkylen, durch NH oder O umterbrochenes $C_4$-$C_{16}$-Alkylen, $C_4$-$C_8$-Alkenylen, Xylylen oder Cyclohexylen bedeutet
$R^{26}$ Wasserstoff, $C_1$-$C_{12}$-Alkyl, $C_5$-$C_8$-Cycloalkyl oder eine Gruppe der Formel A bedeutet,
$R^{27}$ eine der für $R^{23}$ gegebenen Bedeutungen hat oder eine Gruppe -NH-$R^{28}$-NH ist,
$R^{28}$ $C_2$-$C_{12}$-Alkylen oder $C_6$-$C_{12}$-Arylen bedeutet, das durch $C_1$-$C_4$-Alkyl substituiert sein kann,
$R^{29}$ Wasserstoff, $C_1$-$C_{12}$-Alkyl, $C_2$-$C_{18}$-Alkanoyl oder einen Triazinylrest der Formel B bedeutet, und $R^1$, A und B die in Anspruch 1 gegebenen Bedeutungen haben,
und wenn p = 3 ist, eine der Gruppen

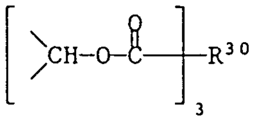 , 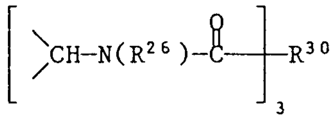 ,

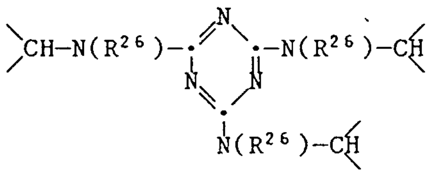

bedeutet, worin $R^{30}$ $C_3$-$C_{18}$-Alkantriyl oder $C_6$-$C_{12}$-Arentriyl bedeutet, und wenn p = 4 ist, eine Gruppe

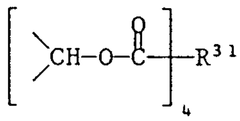 oder 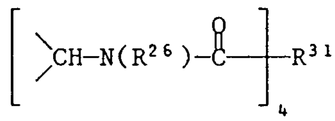

bedeutet, worin $R^{31}$ $C_4$-$C_{16}$-Alkantetrayl oder $C_6$-$C_{12}$-Arentetrayl bedeutet.

**3.** Eine Verbindung der Formel IV,

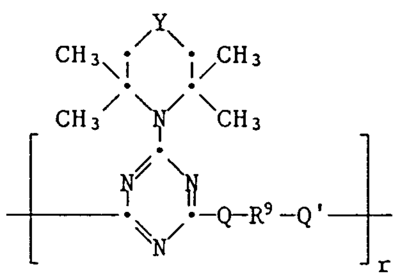

IV

worin r ein Wert von 3 bis 50 ist,
Q und Q' unabhängig voneinander -O-, -S- oder -N($R^{10}$)- bedeuten und Y, $R^9$ und $R^{10}$ die in Anspruch 1 gegebenen Bedeutungen haben, oder worin -Q-$R^9$-Q'- eine Gruppe -NHNH-,

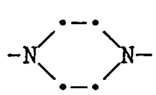 oder 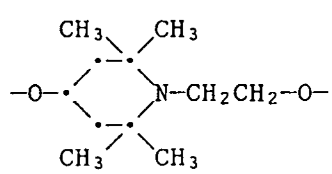

ist.

**4.** Eine Verbindung der Formel V,

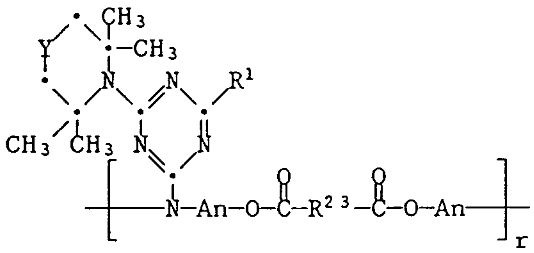

V

worin r ein Wert von 3 bis 50 ist, Y und $R^1$ die in Anspruch 1 gegebenen Bedeutungen haben, An eine $C_2$-$C_4$-Alkylengruppe ist und $R^{23}$ die in Anspruch 2 gegebene Bedeutung hat.

**5.** Eine Verbindung der Formel VI,

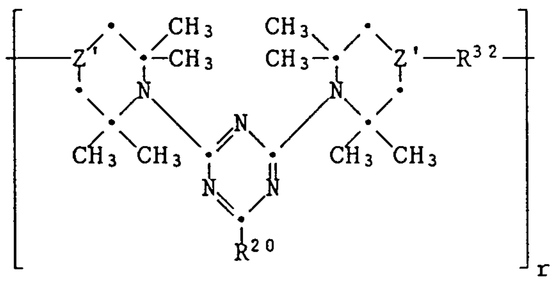

VI

worin r ein Wert von 3 bis 50 ist,
$R^{20}$ die in Anspruch 1 gegebene Bedeutung hat und
$>$ Z'-$R^{32}$-Z' $<$ eine der folgenden Gruppen bedeutet

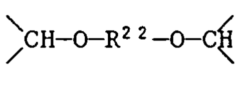 , 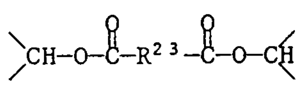 , 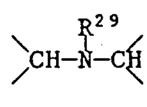 ,

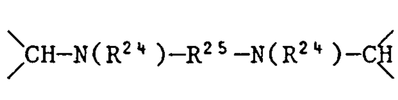 , 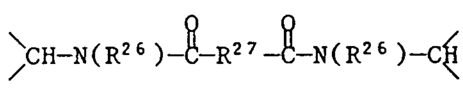

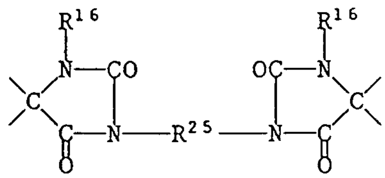

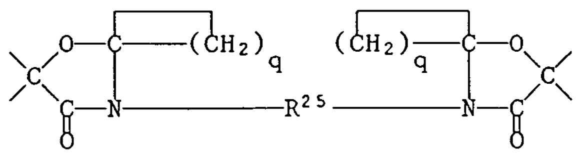

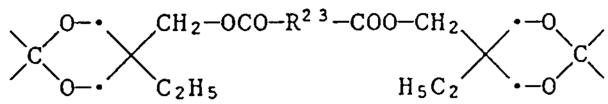

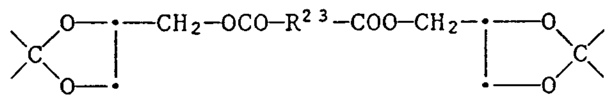

worin $R^{22}$, $R^{23}$, $R^{24}$, $R^{25}$, $R^{26}$, $R^{27}$ und $R^{29}$ die in Anspruch 2 gegebene Bedeutung haben und q und $R^{16}$ die in Anspruch 1 gegebene Bedeutung hat.

**6.** Eine Verbindung der Formel VII,

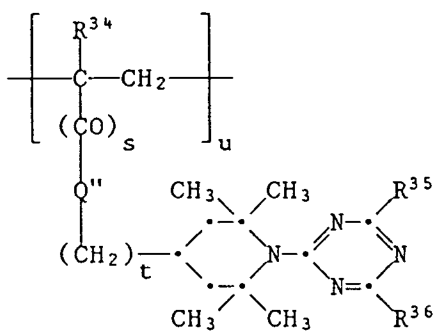

VII

worin s 0 oder 1, t 0 oder 2 und u einen Wert von 5 bis 100 bedeutet,
Q'' -O-, -NH- oder -N($C_1$-$C_4$-Alkyl)- bedeutet,
$R^{34}$ Wasserstoff oder Methyl ist,
$R^{35}$ und $R^{36}$ unabhängig voneinander -$OR^3$, -$SR^3$ oder -$NR^4R^5$ bedeuten, worin
$R^3$, $R^4$ und $R^5$ die in Anspruch 1 gegebenen Bedeutungen haben,
sowie Copolymere einer solchen Verbindung mit (Meth)acrylsäure, Alkyl(meth)acrylaten, Hydroxyalkyl(meth)acrylaten oder Maleinsäureanhydrid.

**7.** Eine Verbindung der Formel VIII,

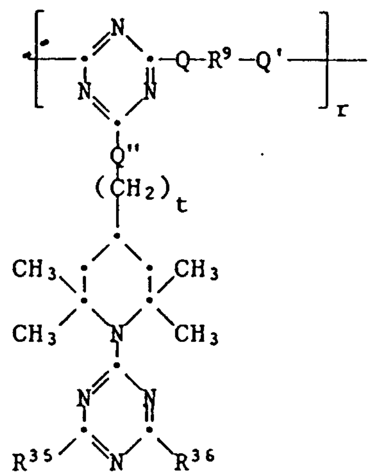

VIII

worin r ein Wert von 3 bis 50 ist, t 0 oder 2 ist,
$R^9$, Q und Q' die in Anspruch 3 gegebenen Bedeutungen haben,
Q'' -O-, -NH- oder -N($C_1$-$C_4$-Alkyl)- bedeutet und
$R^{35}$ und $R^{36}$ die in Anspruch 6 gegebenen Bedeutungen haben.

**8.** Eine Verbindung der Formel IX,

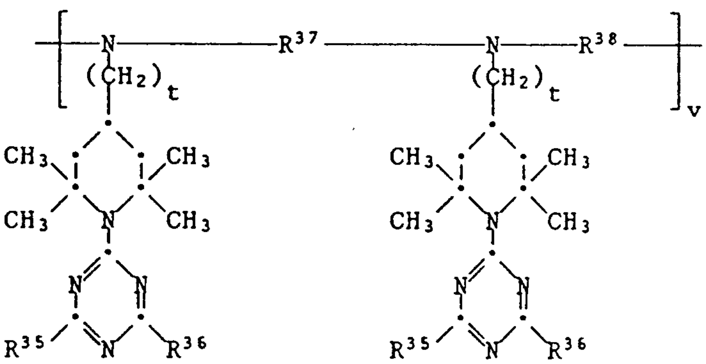

IX

worin v ein Wert von 2 bis 30 ist, t 0 oder 2 ist, $R^{35}$ und $R^{36}$ die in Anspruch 6 gegebenen Bedeutungen haben,
$R^{37}$ $C_2$-$C_8$-Alkylen, $C_4$-$C_8$-Alkenylen, Xylylen, $-CH_2-CH(OH)-CH_2-$ oder $-CH_2CH(OH)CH_2-O-R^{39}-O-CH_2CH(CH)CH_2-$ bedeutet,
$R^{38}$ eine der für $R^{37}$ gegebenen Bedeutungen hat oder

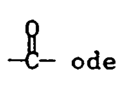 oder 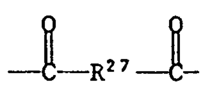

ist,
$R^{39}$ $C_2$-$C_8$-Alkylen, Phenylen oder

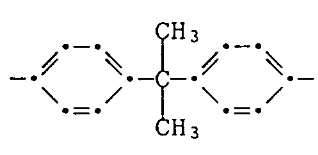

bedeutet und
$R^{27}$ die im Anspruch 2 genannte Bedeutung hat.

**9.** Eine Verbindung der Formel X,

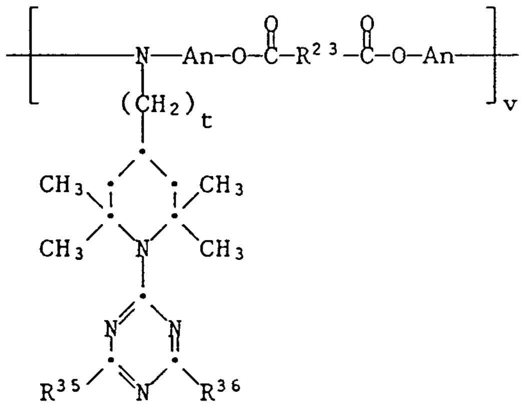

X

worin t 0 oder 2 ist, v ein Wert von 2 bis 30 ist,
An $C_2$-$C_4$-Alkylen bedeutet, $R^{35}$ und $R^{36}$ die in Anspruch 6 gegebenen Bedeutungen haben und $R^{23}$ die in Anspruch 2 gegebene Bedeutung hat.

**10.** Eine Verbindung der Formel XI,

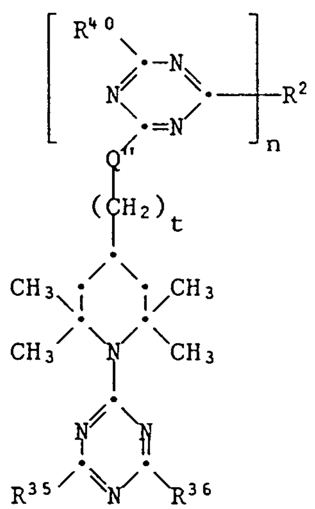

XI

worin n eine ganze Zahl von 1 bis 6 bedeutet, t 0 oder 2 ist, $R^2$ die in Anspruch 1 gegebene Bedeutung hat, Q'', $R^{35}$ und $R^{36}$ die in Anspruch 6 gegebenen Bedeutungen haben und $R^{40}$ entweder eine der für $R^1$ in Anspruch 1 gegebenen Bedeutungen hat oder eine Gruppe

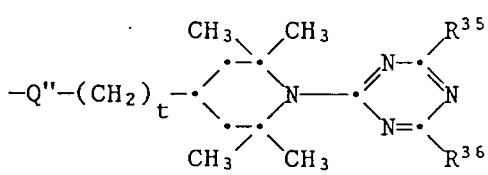

bedeutet.

**11.** Eine Verbindung der Formel XII,

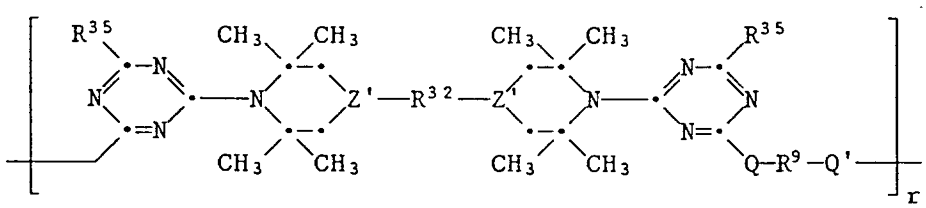

XII

worin r ein Wert von 3 bis 50 ist,
$R^9$ die in Anspruch 1 gegebene Bedeutung hat, die Gruppe

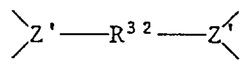

eine der in Anspruch 5 gegebenen Bedeutungen hat, Q und Q' unabhängig voneinander -O-, -S- oder -N($R^{10}$)- bedeuten und $R^{35}$ die in Anspruch 6 gegebene Bedeutung hat.

**12.** Eine Verbindung gemäss Anspruch 1 der Formel II,
worin n eine ganze Zahl von 1-4 ist,
$R^1$ ein Rest Cl, -$OR^3$ oder -$NR^4R^5$ ist, worin
$R^3$, $R^4$ und $R^5$ die in Anspruch 1 gegebenen Bedeutungen haben,
$R^2$, wenn n = 1 ist, Cl, -$OR^3$ oder -$NR^4R^5$ bedeutet,
wenn n = 2 ist, eine Gruppe -N($R^{10}$)-$R^9$-N($R^{10}$)- oder

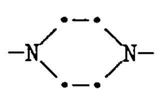

bedeutet, worin $R^9$ und $R^{10}$ die in Anspruch 1 gegebenen Bedeutungen haben,
wenn n = 3 ist, eine Gruppe

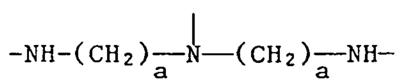

bedeutet und
wenn n = 4 ist, eine Gruppe

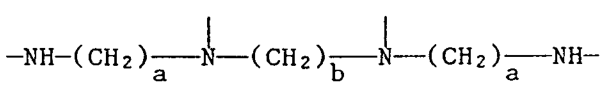

bedeutet, worin a 2 oder 3 und b 2 bis 8 bedeuten und
Y eine Gruppe
>CH—$OR^{11}$, >CH—$NR^{12}R^{13}$, >C=O oder

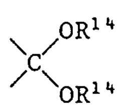

ist,
worin $R^{11}$, $R^{12}$, $R^{13}$ und $R^{14}$ die in Anspruch 1 gegebenen Bedeutungen haben.

**13.** Eine Verbindung gemäss Anspruch 1 der Formel II, worin n 1 oder 2 ist, $R^1$ einen Rest der Formel

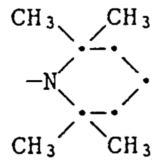

oder Cl, $-OR^3$ oder $-NR^4R^5$ bedeutet, wobei $R^3$ $C_1$-$C_{12}$-Alkyl, Allyl oder eine Gruppe der Formel A ist, $R^4$ Wasserstoff, $C_1$-$C_8$-Alkyl, 2-Hydroxyalkyl oder eine Gruppe der Formel A bedeutet,
$R^5$ $C_1$-$C_8$-Alkyl, 2-Hydroxyethyl, eine Gruppe der Formel A oder eine Gruppe der Formel

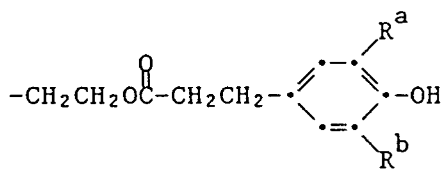

bedeutet, worin $R^a$ und $R^b$ $C_1$-$C_4$-Alkyl bedeuten, oder $R^4$ und $R^5$ zusammen 3-Oxapentamethylen bedeuten,
$R^2$ bei n = 1 Cl, $-OR^3$ oder $-NR^4R^5$ bedeutet, worin $R^3$, $R^4$ und $R^5$ die obigen Bedeutungen haben, und bei n = 2 $-NH(CH_2)_6-NH-$ bedeutet,
Y eine der Gruppen
$>CH_2$, $>CH-OR^{11}$, $>CH-NR^{12}R^{13}$, $>C=O$,

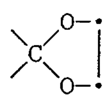 oder 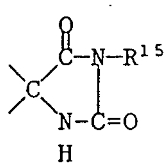

ist,
worin $R^{11}$ Wasserstoff, $C_1$-$C_6$-Alkyl, Acetyl, Acryloyl, Benzyl oder eine Gruppe der Formel

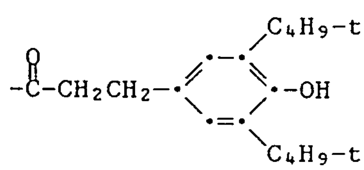

ist,
$R^{12}$ Wasserstoff oder $C_1$-$C_4$-Alkyl ist,
$R^{13}$ $C_1$-$C_4$-Alkyl oder ein Rest der Formel B ist, in der
$R^{19}$ und $R^{20}$ Cl oder $-NR^4R^5$ sind, und $R^{15}$ $C_1$-$C_{12}$-Alkyl ist.

**14.** Eine Verbindung gemäss Anspruch 2 der Formel III, worin p 2, 3 oder 4 ist, $R^{19}$ und $R^{20}$ unabhängig voneinander $-OR^3$ oder $-NR^4R^5$ bedeuten, worin $R^3$, $R^4$ und $R^5$ die in Anspruch 1 gegebenen Bedeutungen haben,
Z, wenn p = 2 ist, eine der Gruppen

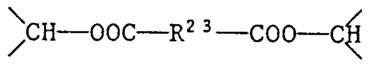 , 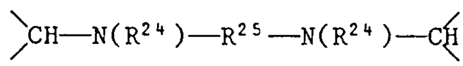 ,

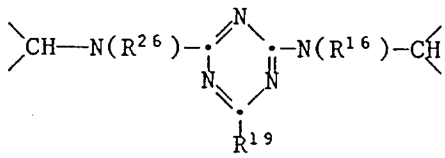 , 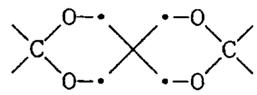 ,

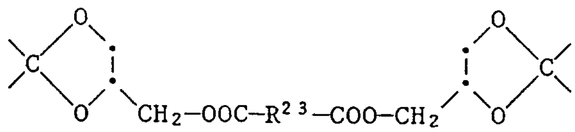

bedeutet, worin $R^{23}$, $R^{24}$, $R^{25}$ und $R^{26}$ die in Anspruch 2 gegebenen Bedeutungen haben, wenn p = 3 ist, eine der Gruppen

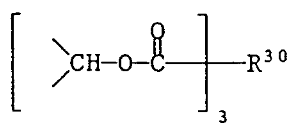 oder 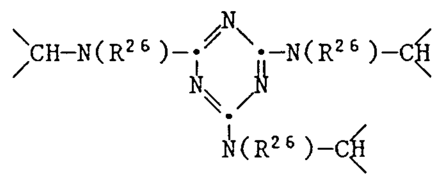

bedeutet, worin $R^{30}$ $C_3$-$C_8$-Alkantriyl oder $C_6$-$C_{12}$-Arentriyl bedeutet und $R^{26}$ die in Anspruch 2 gegebene Bedeutung hat, und wenn p = 4 ist, eine Gruppe

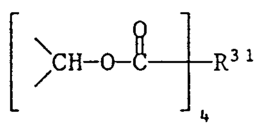

ist, worin $R^{31}$ $C_4$-$C_{12}$-Alkantetrayl oder $C_6$-$C_{12}$-Arentetrayl bedeuten.

**15.** Eine Verbindung gemäss Anspruch 2 der Formel III, worin p 2 ist, $R^{19}$ und $R^{20}$ Cl oder $-NR^4R^5$ bedeuten, worin $R^4$ und $R^5$ $C_1$-$C_4$-Alkyl sind, und Z eine der Gruppen

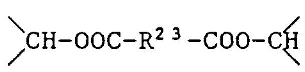 , 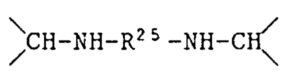 oder 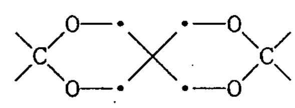

bedeutet, worin $R^{23}$ $C_2$-$C_8$-Alkylen oder Phenylen ist und $R^{25}$ $-(CH_2)_6-$ bedeutet.

**16.** Eine Verbindung gemäss Anspruch 3 der Formel IV, worin r ein Wert von 3 bis 25 ist, Q und Q' -O- oder $-N(R^{10})-$ bedeuten und Y, $R^9$ und $R^{10}$ die in Anspruch 1 gegebenen Bedeutungen haben.

**17.** Eine Verbindung gemäss Anspruch 3 der Formel IV, worin r ein Wert von 3 bis 50 ist, Y eine Gruppe $>CH_2$, $>C=O$,

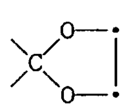

oder >CH-O$C_1$-$C_8$-Alkyl ist, Q und Q' -NH- bedeuten und $R^9$ -$(CH_2)_6$- bedeutet.

**18.** Eine Verbindung gemäss Anspruch 4 der Formel V, worin r ein Wert von 3 bis 50 ist, Y eine Gruppe der Formel

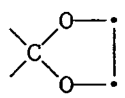

bedeutet, An -$CH_2CH_2$- ist und $R^{23}$ $C_2$-$C_8$-Alkylen ist.

**19.** Organisches Material, enthaltend als Stabilisator mindestens eine Verbindung der Formel II des Anspruches 1, der Formel III des Anspruches 2, der Formel IV des Anspruches 3, der Formel V des Anspruches 4, der Formel VI des Anspruches 5, der Formel VII des Anspruches 6, der Formel VIII des Anspruches 7, der Formel IX des Anspruches 8, der Formel X des Anspruches 9, der Formel XI des Anspruches 10 oder der Formel XII des Anspruches 11, mit Ausnahme von 2-(2,2,6,6-Tetramethyl-1-piperidino)-4,6-bis(2,4,4-trimethyl-2-pentylamino)-triazin und 2,4-Dichlor-6-(2,2,6,6-tetramethyl-1-piperidino)-triazin.

**20.** Organisches Material gemäss Anspruch 19, das ein organisches Polymer ist.

**21.** Organisches Material gemäss Anspruch 19, das ein Anstrichstoff ist.

**22.** Organisches Material gemäss Anspruch 19, das eine photographische Schicht ist.

**23.** Photographische Schicht gemäss Anspruch 22, enthaltend zusätzlich ein sterisch gehindertes Phenol.

**24.** Verwendung einer Verbindung gemäss einem der Ansprüche 1-11, als Stabilisator für organische Materialien gegen Schädigung durch Licht, Sauerstoff und Hitze.

**25.** Verwendung gemäss Anspruch 24 einer Verbindung der Formel

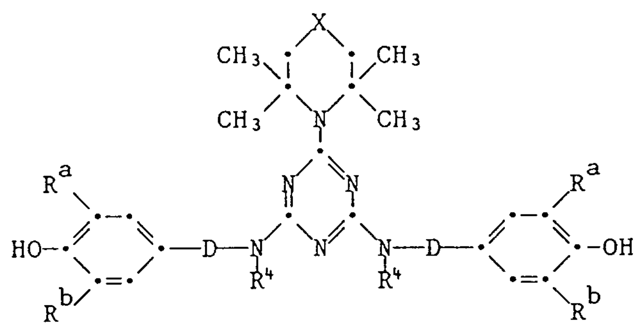

worin X eine Gruppe ist, die den Ring zu einem Piperidinring ergänzt,
D ein durch

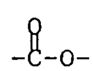

unterbrochener $C_2$-$C_{20}$-Alkylenrest ist,
$R^a$ und $R^b$ $C_1$-$C_{12}$-Alkyl, $C_5$-$C_6$-Cycloalkyl, $C_6$-$C_{10}$-Aryl oder $C_7$-$C_9$-Phenylalkyl bedeuten und $R^a$ auch Wasserstoff bedeutet, als Stabilisatoren für photographische Farbstoffe.

## Claims

**1.** A compound of the formula II

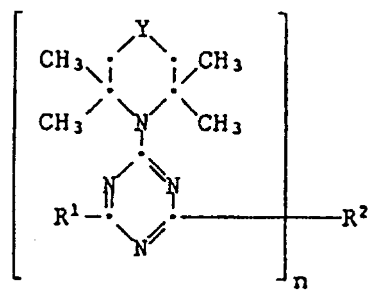

II

in which n is an integer from 1 to 6, $R^1$ is a radical of the formula

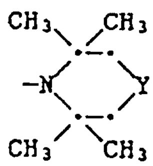

or Cl, OH, $-OR^3$, $-SR^3$ or $-NR^4R^5$, where $R^3$ is $C_1$-$C_{18}$alkyl, allyl, cyclohexyl, benzyl, phenyl or a group of the formula A

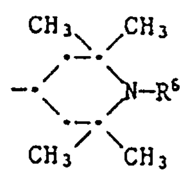

A

$R^4$ is hydrogen, $C_1$-$C_{12}$alkyl, 2-hydroxyethyl, allyl, cyclohexyl, benzyl or a group of the formula A, $R^5$ is $C_1$-$C_{12}$alkyl, 2-hydroxyethyl, allyl, cyclohexyl, benzyl, phenyl, phenyl which is substituted by halogen, $C_1$-$C_4$alkyl or $C_1$-$C_4$alkoxy, a group of the formula A or a group of the formula C

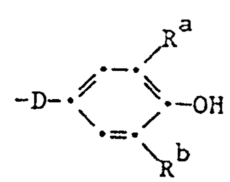

C

in which D is $C_2$-$C_{20}$alkylene interrupted by

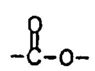 ,

$R^a$ and $R^b$ are $C_1$-$C_{12}$alkyl, $C_5$-$C_6$cycloalkyl, $C_6$-$C_{10}$aryl or $C_7$-$C_9$phenylalkyl and $R^a$ is also hydrogen, or $R^4$ and $R^5$ together are $C_4$-$C_8$alkylene which can be interrupted by -O- or $-N(R^8)-$ and in which $R^8$ is hydrogen, $C_1$-$C_4$alkyl or acetyl, $R^6$ is hydrogen, $C_1$-$C_{12}$alkyl, $C_7$-$C_9$phenylalkyl, $C_3$-$C_5$alkenyl, $C_2$-$C_4$alkanoyl, $C_3$-$C_5$alkenoyl, -O•, -OH or $-OR^7$ and $R^7$ is $C_1$-$C_{18}$alkyl, $C_5$-$C_8$cycloalkyl, $C_7$-$C_9$ phenylalkyl, phenyl, $C_2$-$C_{18}$alkanoyl or benzoyl, $R^2$, if n = 1, is Cl, OH, $-OR^3$, $-SR^3$ or $-NR^4R^5$, if n = 2, $R^2$ is a group $-O-R^9-O-$, $-S-R^9-S-$, $-N(R^{10})-R^9-N(R^{10})-$, $-O-R^9-N(R^{10})-$,

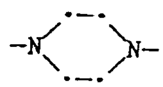

or -NH-NH-, where $R^9$ is $C_2$-$C_{20}$alkylene which can be interrupted by one or more -O-, -N($R^8$)- or -OOC-$R^{17}$-COO-, $C_4$-$C_8$alkenylene, $C_5$-$C_8$cycloalkylene, xylylene, phenylene or tolylene, $R^{10}$ is hydrogen, $C_1$-$C_{12}$alkyl, allyl, 2-hydroxyethyl, benzyl, phenyl or a group of the formula A, if n = 3, $R^2$ is a group

$$-NH-(CH_2)_a-N-(CH_2)_a-NH-$$

or

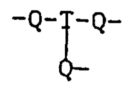

in which a is 2 or 3, Q is -O-, -S- or -N($R^{10}$)- and T is $C_3$-$C_{20}$alkanetriyl or a group

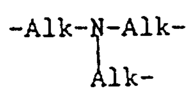

or

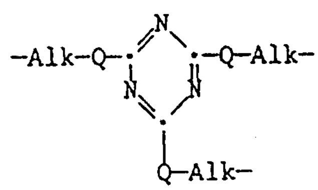

in which Alk is a $C_2$-$C_{12}$alkylene group, if n = 4, $R^2$ is a group $C(CH_2O-)_4$ or

$$-NH-(CH_2)_a-N-(CH_2)_b-N-(CH_2)_a-NH-$$

in which a is 2 or 3 and b is 2-12,
if n = 5, $R^2$ is a group

$$-NH(CH_2CH_2N)_3CH_2CH_2NH-$$

and if n = 6, $R^2$ is a group

$$-NH(CH_2CH_2N)_4CH_2CH_2NH-$$

Y is a group

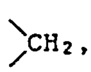,
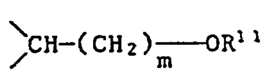,
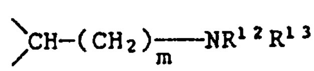,
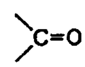,

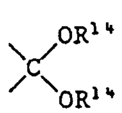,
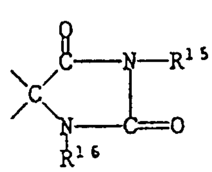
or
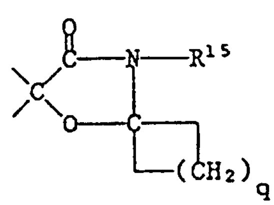

in which m is 0, 1 or 2, q is an integer from 5-11, $R^{11}$ is hydrogen, $C_1$-$C_{18}$alkyl, $C_3$-$C_7$alkenyl, $C_5$-$C_8$cycloalkyl, $C_7$-$C_{11}$aralkyl or a group -CO-$R^{18}$, $R^{12}$ is hydrogen, $C_1$-$C_{12}$alkyl, $C_3$-$C_7$alkenyl, $C_5$-$C_8$cycloalkyl, $C_7$-$C_{11}$aralkyl, $C_2$-$C_4$hydroxyalkyl, $C_3$-$C_8$alkoxyalkyl, $C_4$-$C_{20}$dialkylaminoalkyl, $C_3$-$C_{14}$alkoxycarbonylalkyl or a group of the formula A, $R^{13}$ is $C_1$-$C_{12}$alkyl, $C_2$-$C_4$hydroxyalkyl, $C_3$-$C_7$alkenyl, $C_5$-$C_8$cycloalkyl, phenyl, phenyl which is substituted by halogen, $C_1$-$C_4$alkyl or $C_1$-$C_4$alkoxy, $C_2$-$C_{20}$alkanoyl, $C_3$-$C_8$alkenoyl, benzoyl, phenylacetyl or a triazinyl radical of the formula B

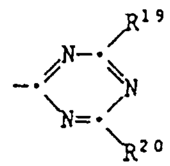
B

or $R^{12}$ and $R^{13}$ together are $C_4$-$C_8$alkylene which can be interrupted by -O- or -N($R^8$)- or $R^{12}$ and $R^{13}$ together are a radical of the formula

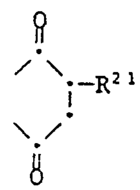

in which $R^{21}$ is $C_1$-$C_{18}$alkyl, $R^{14}$ is $C_1$-$C_{18}$alkyl, $C_5$-$C_8$cycloalkyl or $C_7$-$C_9$phenylalkyl or both groups $R^{14}$ together are $C_2$-$C_6$alkylene, o-phenylene or o-xylylene, $R^{15}$ is hydrogen, $C_1$-$C_{12}$alkyl, $C_3$-$C_5$alkenyl, $C_7$-$C_9$phenylalkyl, $C_2$-$C_4$hydroxyalkyl, $C_3$-$C_8$alkoxyalkyl or $C_3$-$C_{14}$alkoxycarbonylalkyl, $R^{16}$ is hydrogen, $C_1$-$C_{12}$alkyl, allyl or benzyl, $R^{17}$ is $C_1$-$C_{12}$alkylene, vinylene, cyclohexylene, xylylene or $C_6$-$C_{12}$arylene, $R^{18}$ is $C_1$-$C_{18}$alkyl, $C_2$-$C_6$alkenyl, $C_5$-$C_8$cycloalkyl, $C_7$-$C_9$phenylalkyl, phenyl or phenyl which is substituted by halogen, nitro, $C_1$-$C_4$alkyl, hydroxyl or $C_1$-$C_4$alkoxy or $C_7$-$C_9$phenylalkyl which is substituted by hydroxyl and $C_1$-$C_4$alkyl, $R^{19}$ is as defined for $R^1$, and $R^{20}$ is Cl, -OH, -$OR^3$, -$SR^3$ or -$NR^4R^5$, with the exception of 2-(2,2,6,6-tetramethyl-1-piperidino)-4,6-bis(2,4,4-trimethyl-2-pentylamino) triazine and 2,4-dichloro-6-(2,2,6,6-tetramethyl-1-piperidino) triazine.

**2.** A compound of the formula III

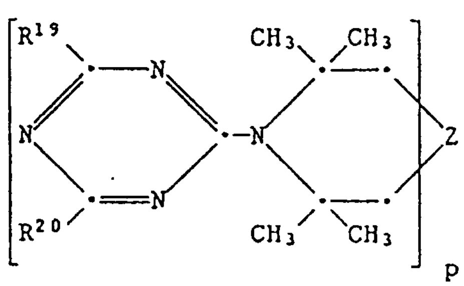
III

in which p is 2, 3 or 4, $R^{19}$ and $R^{20}$ are as defined in claim 1 and Z, if p = 2, is one of the groups

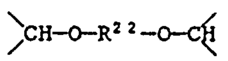 , 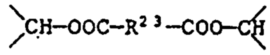 , 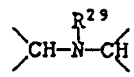 ,

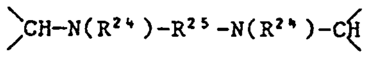 , 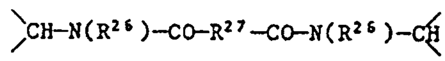 ,

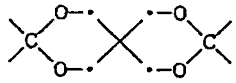 , 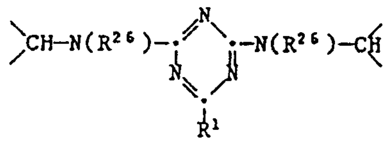 , 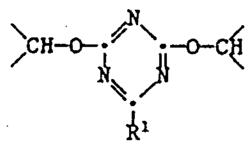 ,

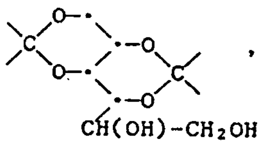 , 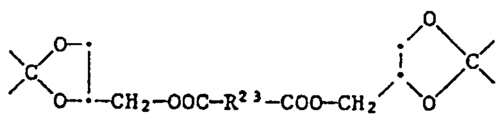

in which $R^{22}$ is $C_2$-$C_{12}$alkylene, $C_4$-$C_8$alkenylene, xylylene or -CO-, $R^{23}$ is $C_1$-$C_{12}$alkylene, vinylene, cyclohexylene, xylylene, $C_6$-$C_{12}$arylene or phenylene which is substituted by halogen, nitro or $C_1$-$C_4$alkyl, or a direct bond, $R^{24}$ is hydrogen, $C_1$-$C_{12}$alkyl, $C_5$-$C_8$cycloalkyl, $C_7$-$C_9$phenylalkyl, $C_3$-$C_7$alkenyl, $C_2$-$C_{18}$alkanoyl, $C_3$-$C_7$alkenoyl, or benzoyl, $R^{25}$ is $C_2$-$C_{12}$alkylene, $C_4$-$C_{16}$alkylene which is interrupted by NH or O, $C_4$-$C_8$alkenylene, xylylene or cyclohexylene, $R^{26}$ is hydrogen, $C_1$-$C_{12}$alkyl, $C_5$-$C_8$cycloalkyl or a group of the formula A, $R^{27}$ is as defined for $R^{23}$ or is a group -NH-$R^{28}$-NH, $R^{28}$ is $C_2$-$C_{12}$alkylene or $C_6$-$C_{12}$arylene which can be substituted by $C_1$-$C_4$alkyl, $R^{29}$ is hydrogen, $C_1$-$C_{12}$alkyl, $C_2$-$C_{18}$alkanoyl or a triazinyl radical of the formula B, and $R^1$, A and B are as defined in claim 1, and if p = 3, Z is one of the groups

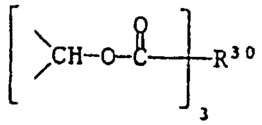 , 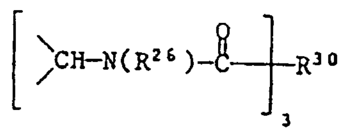 ,

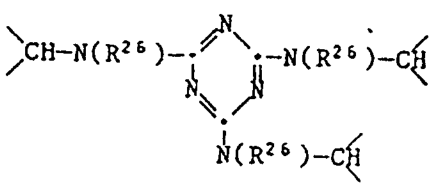

in which $R^{30}$ is $C_3$-$C_{18}$alkanetriyl or $C_6$-$C_{12}$arenetriyl, and if p = 4, Z is a group

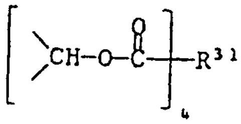 or 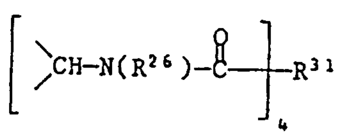

in which $R^{31}$ is $C_4$-$C_{16}$alkanetetrayl or $C_6$-$C_{12}$arenetetrayl.

**3.** A compound of the formula IV

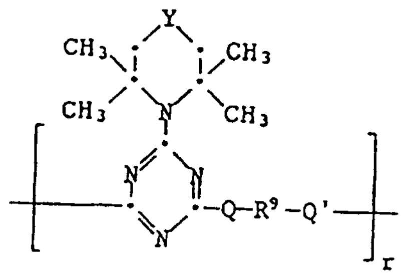

IV

in which r has a value from 3 to 50, Q and Q' independently of one another are -O-, -S- or -N($R^{10}$)- and Y, $R^9$ and $R^{10}$ are as defined in claim 1, or in which -Q-$R^9$-Q'- is a group -NHNH-,

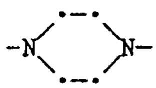 or 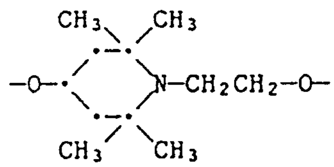 .

**4.** A compound of the formula V

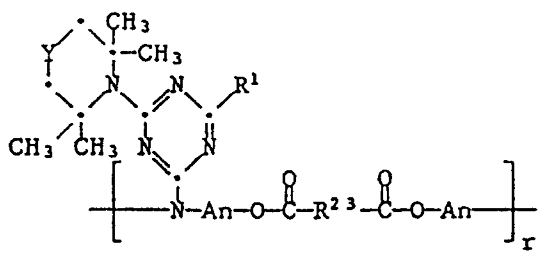

V

in which r has a value from 3 to 50, Y and $R^1$ are as defined in claim 1, An is a $C_2$-$C_4$alkylene group and $R^{23}$ is as defined in claim 2.

**5.** A compound of the formula VI

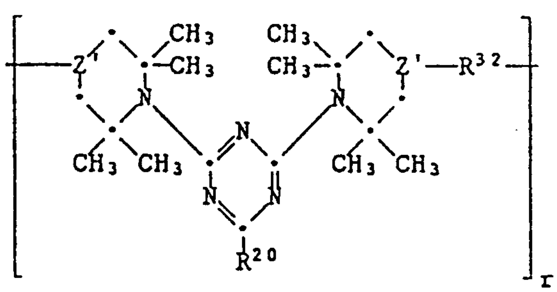

VI

in which r has a value from 3 to 50, $R^{20}$ is as defined in claim 1 and $>$Z'-$R^{32}$-Z'$<$ is one of the following groups

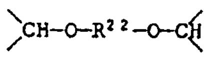 , 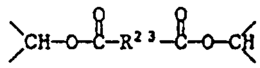 , 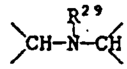 ,

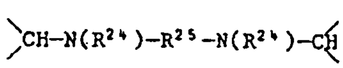 , 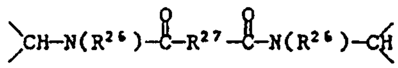

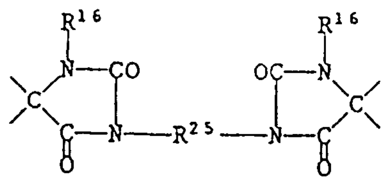

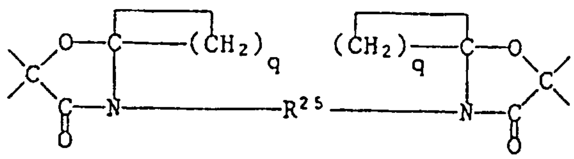

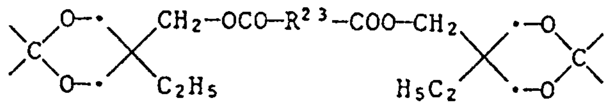

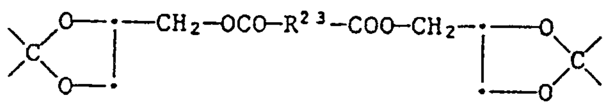

in which $R^{22}$, $R^{23}$, $R^{24}$, $R^{25}$, $R^{26}$, $R^{27}$ and $R^{29}$ are as defined in claim 2 and q and $R^{16}$ are as defined in claim 1.

**6.** A compound of the formula VII

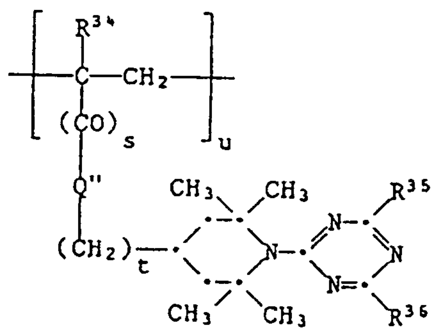 VII

in which s is 0 or 1, t is 0 or 2 and u has a value from 5 to 100, Q" is -O-, -NH- or -N($C_1$-$C_4$alkyl)-, $R^{34}$ is hydrogen or methyl, $R^{35}$ and $R^{36}$ independently of one another are -$OR^3$, -$SR^3$ or -$NR^4R^5$, in which $R^3$, $R^4$ and $R^5$ are as defined in claim 1, and copolymers of such a compound with (meth)acrylic acid, alkyl (meth)acrylates, hydroxyalkyl (meth)acrylates or maleic anhydride.

**7.** A compound of the formula VIII

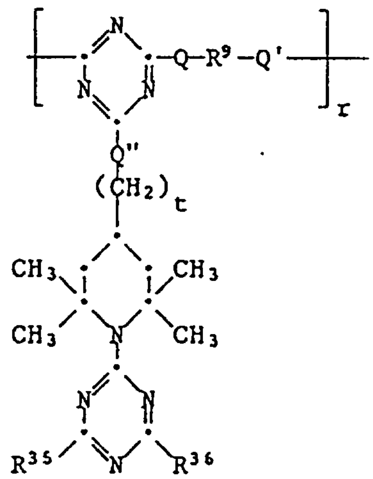

VIII

in which r has a value from 3 to 50, t is 0 or 2, $R^9$, Q and Q' are as defined in claim 3, Q'' is -O-, -NH- or -N($C_1$-$C_4$alkyl)- and $R^{35}$ and $R^{36}$ are as defined in claim 6.

**8.** A compound of the formula IX

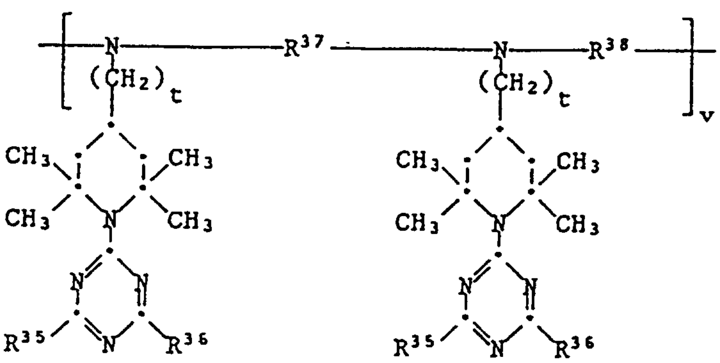

IX

in which v has a value from 2 to 30, t is 0 or 2, $R^{35}$ and $R^{36}$ are as defined in claim 6, $R^{37}$ is $C_2$-$C_8$alkylene, $C_4$-$C_8$alkenylene, xylylene, $-CH_2-CH(OH)-CH_2-$ or $-CH_2CH(OH)CH_2-O-R^{39}-O-CH_2CH(CH)-CH_2-$, $R^{38}$ is as defined for $R^{37}$ or is

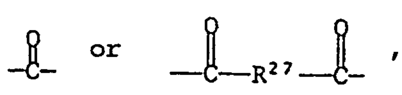

R[39] is $C_2$-$C_8$alkylene, phenylene or

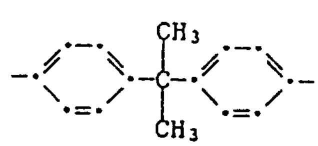

and $R^{27}$ is as defined in claim 2.

**9.** A compound of the formula X

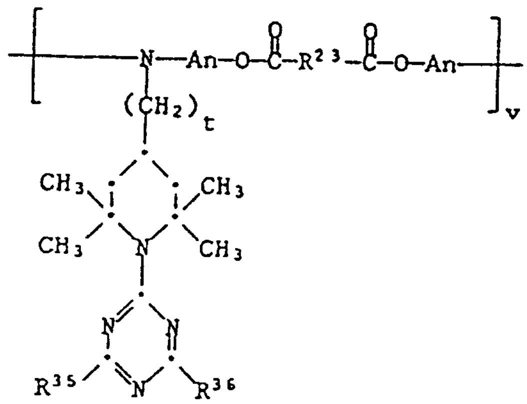

X

in which t is 0 or 2, v has a value from 2 to 30, An is $C_2$-$C_4$alkylene, $R^{35}$ and $R^{36}$ are as defined in claim 6 and $R^{23}$ is as defined in claim 2.

**10.** A compound of the formula XI

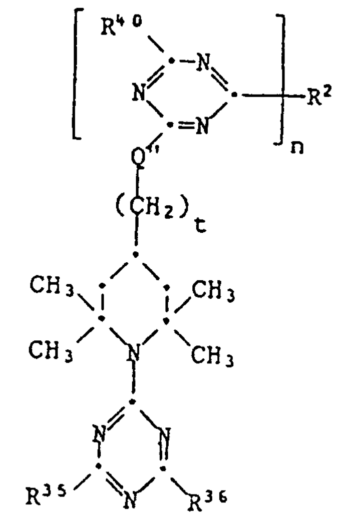

XI

in which n is an integer from 1 to 6, t is 0 or 2, $R^2$ is as defined in claim 1, Q'', $R^{35}$ and $R^{36}$ are as defined in claim 6 and $R^{40}$ is either as defined for $R^1$ in claim 1 or is a group

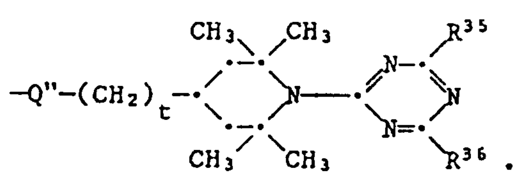

**11.** A compound of the formula XII

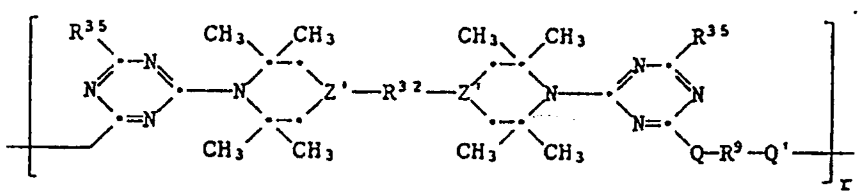

XII

in which r has a value from 3 to 50, $R^9$ is as defined in claim 1, the group

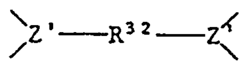

is as defined in claim 5, Q and Q' independently of one another are -O-, -S- or $-N(R^{10})-$ and $R^{35}$ is as defined in claim 6.

**12.** A compound according to claim 1 of the formula II, in which n is an integer from 1-4, $R^1$ is a radical Cl, $-OR^3$ or $-NR^4R^5$, in which $R^3$, $R^4$ and $R^5$ are as defined in claim 1, $R^2$, if n = 1, is Cl, $-OR^3$ or $-NR^4R^5$, if n = 2, $R^2$ is a group $-N(R^{10})-R^9-N(R^{10})-$ or

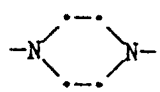

in which $R^9$ and $R^{10}$ are as defined in claim 1, if n = 3 $R^2$ is a group

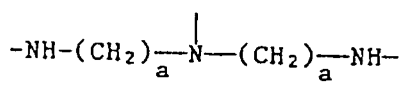

and if n = 4, $R^2$ is a group

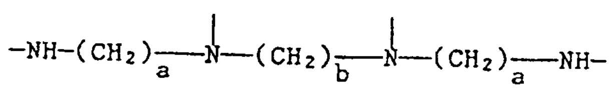

in which a is 2 or 3 and b is 2 to 8 and Y is a group $>CH-OR^{11}$, $>CH-NR^{12}R^{13}$, $>C=O$ or

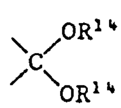

in which $R^{11}$, $R^{12}$, $R^{13}$ and $R^{14}$ are as defined in claim 1.

**13.** A compound according to claim 1 of the formula II, in which n is 1 or 2, $R^1$ is a radical of the formula

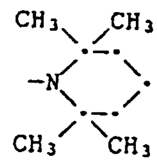

or Cl, $-OR^3$ or $-NR^4R^5$, where $R^3$ is $C_1$-$C_{12}$alkyl, allyl or a group of the formula A, $R^4$ is hydrogen, $C_1$-$C_8$alkyl, 2-hydroxyalkyl or a group of the formula A, $R^5$ is $C_1$-$C_8$alkyl, 2-hydroxyethyl, a group of the formula A or a group of the formula

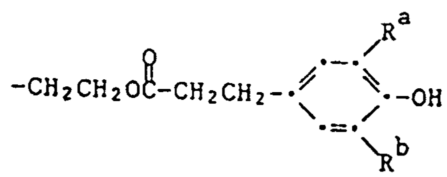

in which $R^a$ and $R^b$ are $C_1$-$C_4$alkyl, or $R^4$ and $R^5$ together are 3-oxapentamethylene, $R^2$, with n = 1, is Cl, $-OR^3$ or $NR^4R^5$, in which $R^3$, $R^4$ and $R^5$ are as defined above, and with n = 2, is $-NH(CH_2)_6-NH-$, Y is one of the groups

$>CH_2$, $>CH-OR^{11}$, $>CH-NR^{12}R^{13}$, $>C=O$,

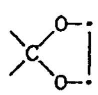 or 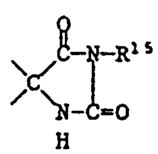

in which $R^{11}$ is hydrogen, $C_1$-$C_6$alkyl, acetyl, acryloyl, benzyl or a group of the formula

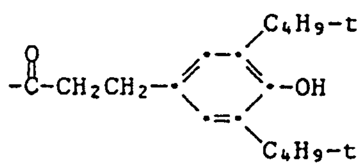

$R^{12}$ is hydrogen or $C_1$-$C_4$alkyl, $R^{13}$ is $C_1$-$C_4$alkyl or a radical of the formula B in which $R^{19}$ and $R^{20}$ are Cl or $-NR^4R^5$, and $R^{15}$ is $C_1$-$C_{12}$alkyl.

**14.** A compound according to claim 2 of the formula III, in which p is 2, 3 or 4, and $R^{19}$ and $R^{20}$ independently of one another are $-OR^3$ or $-NR^4R^5$, in which $R^3$, $R^4$ and $R^5$ are as defined in claim 1, Z, if p = 2, is one of the groups

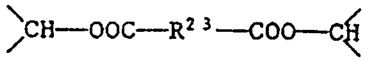 , 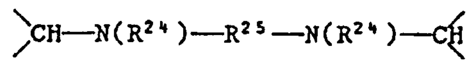 ,

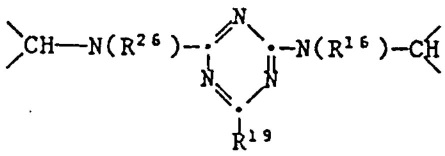 , 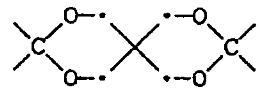 ,

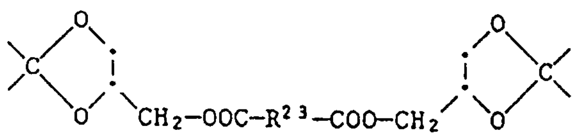

in which $R^{23}$, $R^{24}$, $R^{25}$ and $R^{26}$ are as defined in claim 2, if p = 3, Z is one of the groups

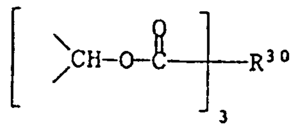 or 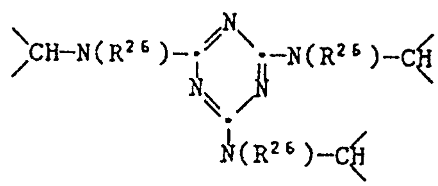

in which $R^{30}$ is $C_3$-$C_8$alkanetriyl or $C_6$-$C_{12}$arenetriyl and $R^{26}$ is as defined in claim 2, and if p = 4, Z is a group

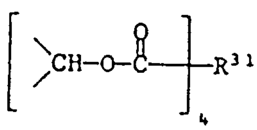

in which $R^{31}$ is $C_4$-$C_{12}$alkane tetrayl or $C_6$-$C_{12}$arenetetrayl.

**15.** A compound according to claim 2 of the formula III, in which p is 2, $R^{19}$ and $R^{20}$ are Cl or $-NR^4R^5$, in which $R^4$ and $R^5$ are $C_1$-$C_4$alkyl, and Z is one of the groups

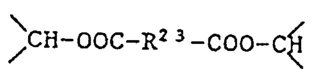 , 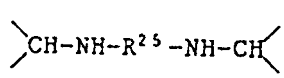 or 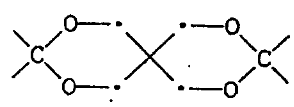

in which $R^{23}$ is $C_2$-$C_8$alkylene or phenylene and $R^{25}$ is $-(CH_2)_6-$.

**16.** A compound according to claim 3 of the formula IV, in which r has a value from 3 to 25, Q and Q' are -O- or $-N(R^{10})-$ and Y, $R^9$ and $R^{10}$ are as defined in claim 1.

**17.** A compound according to claim 3 of the formula IV in which r has a value from 3 to 50, Y is a group $>CH_2$, $>C=O$,

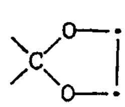

or $>CH-OC_1-C_8$alkyl
Q and Q' are -NH- and $R^9$ is $-(CH_2)_6-$.

**18.** A compound according to claim 4 of the formula V, in which r has a value from 3 to 50, Y is a group of the formula

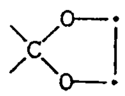 ,

An is $-CH_2CH_2-$ and $R^{23}$ is $C_2$-$C_8$alkylene.

**19.** An organic material which contains as a stabilizer at least one compound of the formula II of claim 1, of the formula III of claim 2, of the formula IV of claim 3, of the formula V of claim 4, of the formula VI of claim 5, of the formula VII of claim 6, of the formula VIII of claim 7, of the formula IX of claim 8, of the formula X of claim 9, of the formula XI of claim 10 or of the formula XII of claim 11, with the exception of 2,-(2,2,6,6-tetramethyl-1-piperidino)-4,6-bis(2,4,4-trimethyl-2-pentylamino)triazine and 2,4-dichloro-6-(2,2,6,6-tetramethyl-1-piperidino)triazine.

**20.** An organic material according to claim 19 which is an organic polymer.

**21.** An organic material according to claim 19 which is a paint.

**22.** An organic material according to claim 19 which is a photographic layer.

**23.** A photographic layer according to claim 22 additionally containing a sterically hindered phenol.

**24.** Use of a compound according to one of claims 1-11 as a stabilizer for organic materials against damage by light, oxygen and heat.

**25.** Use according to claim 24 of a compound of the formula

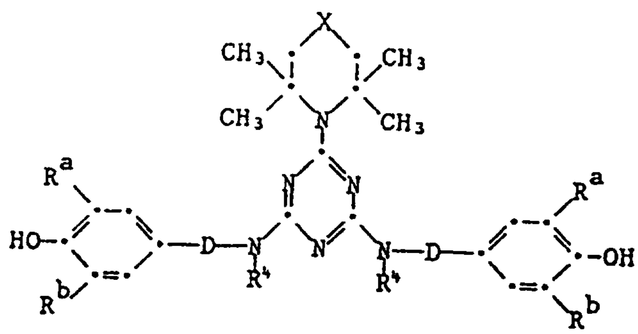

in which X is a group which completes the ring to form a piperidine ring,
D is a $C_2$-$C_{20}$alkylene radical interrupted by

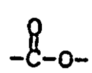 ,

$R^a$ and $R^b$ are $C_1$-$C_{12}$ alkyl, $C_5$-$C_6$cycloalkyl, $C_6$-$C_{10}$aryl or $C_7$-$C_9$phenylalkyl and $R^a$ is also hydrogen, as stabilizers for photographic dyes.

**Revendications**

**1.** Composé de formule II :

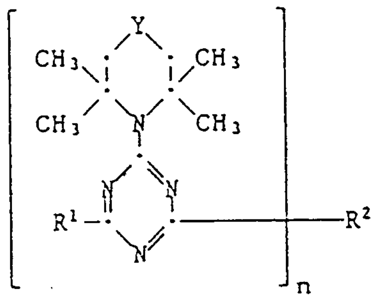

II

où n représente un nombre entier de 1 à 6,
$R^1$ représente un radical de formule :

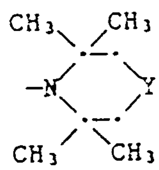

ou Cl, OH, $-OR^3$, $-SR^3$ ou $-NR^4R^5$, $R^3$ représentant les alkyle en $C_1$-$C_{18}$, allyle, cyclohexyle, benzyle, phényle ou un groupe de formule A :

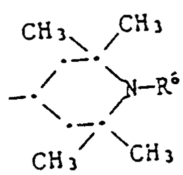

A

$R^4$ représente l'hydrogène, les alkyle en $C_1$-$C_{12}$, 2-hydroxyéthyle, allyle, cyclohexyle, benzyle ou un groupe de formule A,
$R^5$ représente les alkyle en $C_1$-$C_{12}$, 2-hydroxyéthyle, allyle, cyclohexyle, benzyle, phényle, phényle substitué par des halogènes,alkyle en $C_1$-$C_4$ ou alcoxy en $C_1$-$C_4$, ou représente un groupe de formule A ou un groupe de formule C :

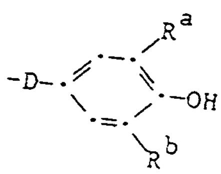

C

où D représente un alkylène en $C_2$-$C_{20}$ interrompu par

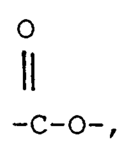

$R^a$ et $R^b$ représentent les alkyle en $C_1$-$C_{12}$, cycloalkyle en $C_5$-$C_6$, aryle en $C_6$-$C_{10}$ ou phénylalkyle en

$C_7$-$C_9$ et $R^a$ représente aussi l'hydrogène, ou
$R^4$ et $R^5$ ensemble représentent un alkylène en $C_4$-$C_8$ qui peut être interrompu par -O- ou -N($R^8$)- et dans lequel $R^8$ représente l'hydrogène, les alkyle en $C_1$-$C_4$ ou acétyle,
$R^6$ représente l'hydrogène, les alkyle en $C_1$-$C_{12}$,phénylalkyle en $C_7$-$C_9$, alcényle en $C_3$-$C_5$, alcanoyle en $C_2$-$C_4$, alcénoyle en $C_3$-$C_5$, -O• -OH ou -$OR^7$ et
$R^7$ représente les alkyle en $C_1$-$C_{18}$, cycloalkyle en $C_5$-$C_8$, phénylalkyle en $C_7$-$C_9$, phényle, alcanoyle en $C_2$-$C_{18}$ ou benzoyle,
$R^2$, lorsque n = 1, représente Cl, OH, -$OR^3$, -$SR^3$ ou -$NR^4R^5$,lorsque n = 2, un groupe -O-$R^9$-O-, -S-$R^9$-S-, -N($R^{10}$)-$R^9$-N($R^{10}$)-, -O-$R^9$-N($R^{10}$)-,

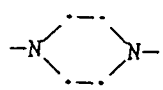

ou -NH-NH-
$R^9$ représente alors un alkylène en $C_2$-$C_{20}$ qui peut être interrompu par un ou plusieurs -O-, -N($R^8$)- ou -OOC-$R^{17}$-COO-, représente aussi les alcénylène en $C_4$-$C_8$, cycloalkylène en $C_5$-$C_8$, xylylène, phénylène ou tolylène,
$R^{10}$ représente l'hydrogène, les alkyle en $C_1$-$C_{12}$, allyle, 2-hydroxyéthyle, benzyle, phényle ou un groupe de formule A,
lorsque n = 3, un groupe

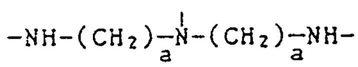

ou

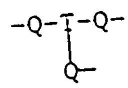

où a vaut 2 ou 3, Q représente -O-, -S- ou -N($R^{10}$)- et T représente un alcanetriyle en $C_3$-$C_{20}$ ou un groupe

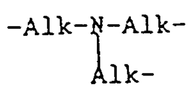

ou

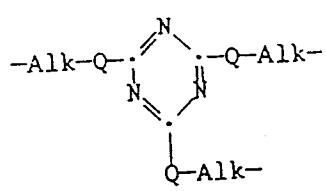

où Alk représente un groupe alkylène en $C_2$-$C_{12}$,
lorsque n = 4, un groupe $C(CH_2O-)_4$ ou

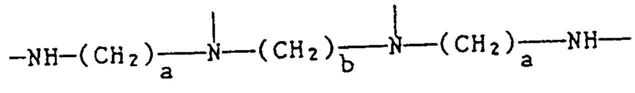

où a vaut 2 ou 3 et b va de 2 à 12,
lorsque n = 5, un groupe

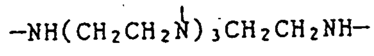

et lorsque n = 6, un groupe

$$-NH(CH_2CH_2\overset{|}{N})_4CH_2CH_2NH-$$

Y représente un groupe

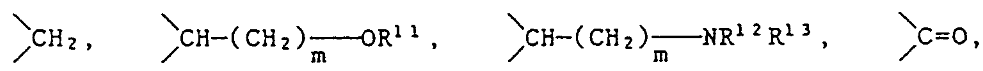

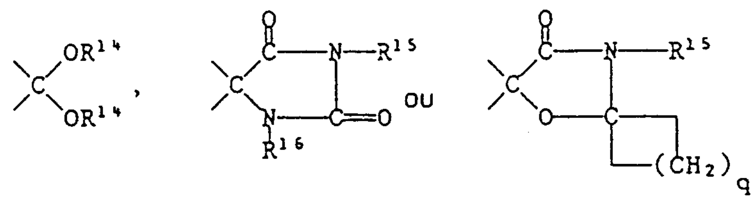

où m vaut 0, 1 ou 2, q représente un nombre entier de 5 à 11,

$R^{11}$ représente l'hydrogène, les alkyle en $C_1$-$C_{18}$, alcényle en $C_3$-$C_7$, cycloalkyle en $C_5$-$C_8$, aralkyle en $C_7$-$C_{11}$ ou un groupe -CO-$R^{18}$,

$R^{12}$ représente l'hydrogène, les alkyle en $C_1$-$C_{12}$, alcényle en $C_3$-$C_7$, cycloalkyle en $C_5$-$C_8$, aralkyle en $C_7$-$C_{11}$, hydroxyalkyle en $C_2$-$C_4$, alcoxyalkyle en $C_3$-$C_8$, dialkylaminoalkyle en $C_4$-$C_{20}$, alcoxycarbonylalkyle en $C_3$-$C_{14}$ ou un groupe de formule A,

$R^{13}$ représente les alkyle en $C_1$-$C_{12}$, hydroxyalkyle en $C_2$-$C_4$, alcényle en $C_3$-$C_7$, cycloalkyle en $C_5$-$C_8$, phényle, un phényle substitué par les halogènes, alkyle en $C_1$-$C_4$ ou alcoxy en $C_1$-$C_4$, les alcanoyle en $C_2$-$C_{20}$, alcénoyle en $C_3$-$C_8$, benzoyle, phénylacétyle ou un radical triazinyle de formule B :

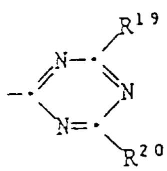

B

ou

$R^{12}$ et $R^{13}$ ensemble représentent un alkylène en $C_4$-$C_8$ qui peut être interrompu par -O- -N($R^8$)-, ou $R^{12}$ et $R^{13}$ ensemble représentent un radical de formule :

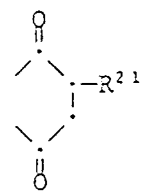

où $R^{21}$ représente un alkyle en $C_1$-$C_{18}$,

$R^{14}$ représente les alkyle en $C_1$-$C_{18}$, cycloalkyle en $C_5$-$C_8$ ou phénylalkyle en $C_7$-$C_9$, ou les deux groupes $R^{14}$ ensemble représentent les alkylène en $C_2$-$C_6$, o-phénylène ou o-xylylène,

$R^{15}$ représente l'hydrogène, les alkyle en $C_1$-$C_{12}$, alcényle en $C_3$-$C_5$, phénylalkyle en $C_7$-$C_9$, hydroxyalkyle en $C_2$-$C_4$, alcoxyalkyle en $C_3$-$C_8$ ou alcoxycarbonylalkyle en $C_3$-$C_{14}$,

$R^{16}$ représente l'hydrogène, les alkyle en $C_1$-$C_{12}$, allyle ou benzyle,

$R^{17}$ représente les alkylène en $C_1$-$C_{12}$, vinylène, cyclohexylène, xylylène ou arylène en $C_6$-$C_{12}$,

$R^{18}$ représente les alkyle en $C_1$-$C_{18}$, alcényle en $C_2$-$C_6$, cycloalkyle en $C_5$-$C_8$, phénylalkyle en $C_7$-$C_9$, phényle ou phényle substitué par les halogène, nitro, alkyle en $C_1$-$C_4$, hydroxy ou alcoxy en $C_1$-$C_4$, ou phénylalkyle en $C_7$-$C_9$ substitué par hydroxy et un alkyle en $C_1$-$C_4$,

$R^{19}$ a l'une des significations données pour $R^1$ et

$R^{20}$ représente Cl, -OH, -$OR^3$, -$SR^3$ ou -$NR^4R^5$, à l'exception de la 2-(2,2,6,6-tétraméthyl-1-pipéridino)-4,6-bis(2,4,4-triméthyl-2-pentylamino)-triazine et de la 2,4-dichloro-6-(2,2,6,6-tétraméthyl-1-pipéridino)-triazine.

**2.** Composé de formule III

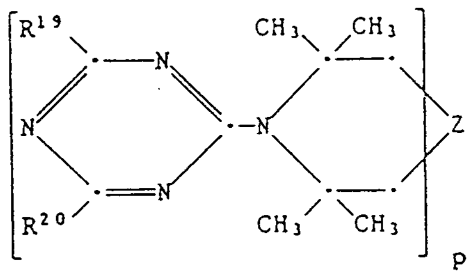

III

où p vaut 2, 3 ou 4,
$R^{19}$ et $R^{20}$ ont les significations données dans la revendication 1, et
Z, lorsque p = 2, représente l'un des groupes

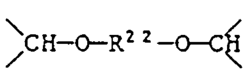 , 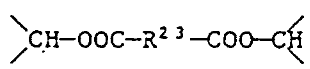 , 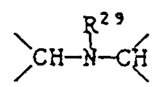 ,

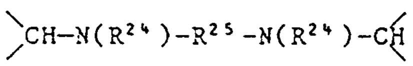 , 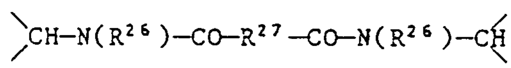 ,

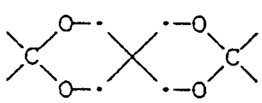 , 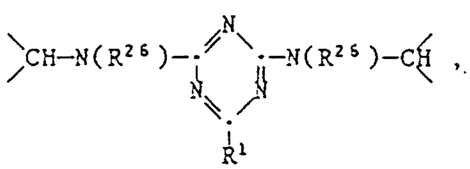 , 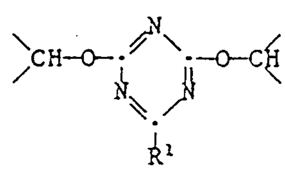 ,

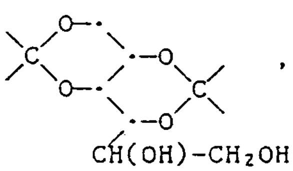 , 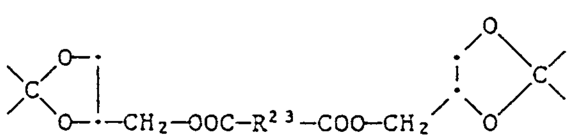

où
$R^{22}$ représente les alkylène en $C_2$-$C_{12}$, alcénylène en $C_4$-$C_8$, xylylène ou -CO-,
$R^{23}$ représente les alkylène en $C_1$-$C_{12}$, vinylène, cyclohexylène, xylylène, arylène en $C_6$-$C_{12}$ ou phénylène substitué par les halogènes, nitro ou alkyle en $C_1$-$C_4$ ou représente une liaison directe,
$R^{24}$ représente l'hydrogène, les alkyle en $C_1$-$C_{12}$, cycloalkyle en $C_5$-$C_8$, phénylalkyle en $C_7$-$C_9$, alcényle en $C_3$-$C_7$, alcanoyle en $C_2$-$C_{18}$, alcénoyle en $C_3$-$C_7$ ou benzoyle,
$R^{25}$ représente les alkylène en $C_2$-$C_{12}$, alkylène en $C_4$-$C_{16}$ interrompu par NH ou O, alcénylène en $C_4$-$C_8$, xylylène ou cyclohexylène,
$R^{26}$ représente l'hydrogène, les alkyle en $C_1$-$C_{12}$, cycloalkyle en $C_5$-$C_8$ ou un groupe de formule A,
$R^{27}$ a l'une des significations données pour $R^{23}$ ou représente un groupe -NH-$R^{28}$-NH-,
$R^{28}$ représente un alkylène en $C_2$-$C_{12}$ ou arylène en $C_6$-$C_{12}$ qui peut être substitué par un alkyle en $C_1$-$C_4$,
$R^{29}$ représente l'hydrogène, les alkyle en $C_1$-$C_{12}$, alcanoyle en $C_2$-$C_{18}$ ou un radical triazinyle de formule B, et $R^1$, A et B ont les significations données dans la revendication 1,
et lorsque p = 3, représente un des groupes

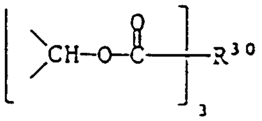 , 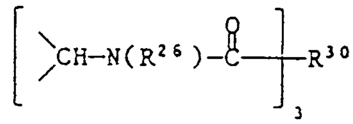 ,

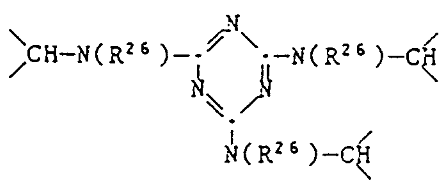

où $R^{30}$ représente un alcanetriyle en $C_3$-$C_{18}$ ou arènetriyle en $C_6$-$C_{12}$, et lorsque p = 4, un groupe

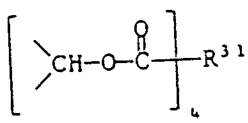 ou 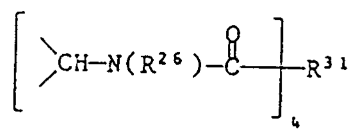

où $R^{31}$ représente un alcanetétrayle en $C_4$-$C_{16}$ ou arènetétrayle en $C_6$-$C_{12}$.

**3.** Composé de formule IV

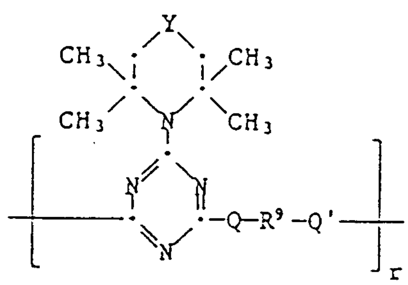

IV

où r va de 3 à 50,
Q et Q', indépendamment l'un de l'autre, représentent -O-, -S- ou -N($R^{10}$)- et Y, $R^9$ et $R^{10}$ ont les significations données dans la revendication 1, ou dans lesquelles -Q-$R^9$-Q' représente un groupe -NHNH-,

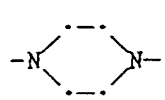 ou 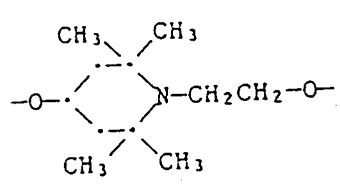

**4.** Composé de formule V

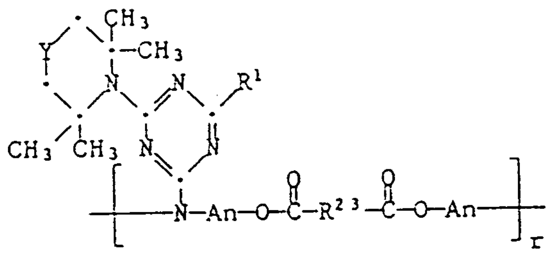

V

où r va de 3 à 50, Y et $R^1$ ont les significations données dans la revendication 1, An représente un groupe alkylène en $C_2$-$C_4$ et $R^{23}$ a la signification donnée dans la revendication 2.

**5.** Composé de formule VI

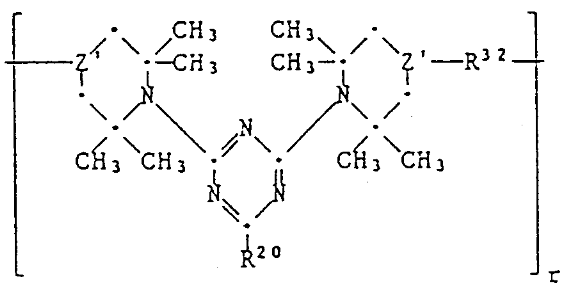

VI

où r va de 3 à 50,
$R^{20}$ a la signification donnée dans la revendication 1, et
$>$ Z'-$R^{32}$-Z' $<$ représente l'un des groupes suivants

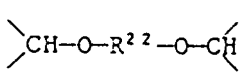 , 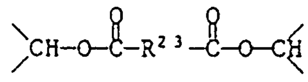 , 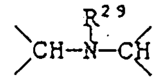 ,

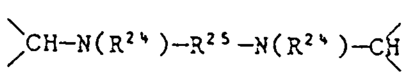 , 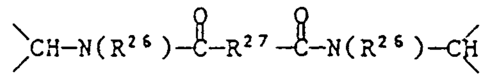

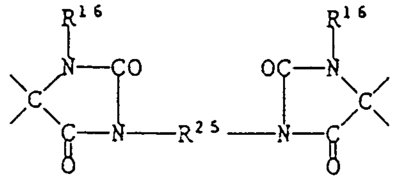

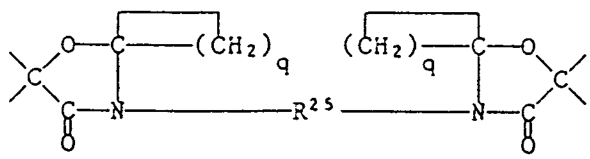

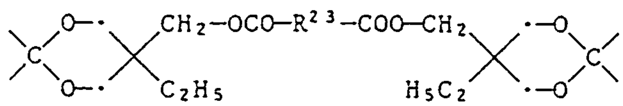

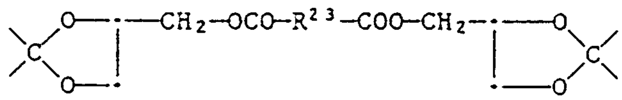

où $R^{22}$, $R^{23}$, $R^{24}$, $R^{25}$, $R^{26}$, $R^{27}$ et $R^{29}$ ont la signification donnée dans la revendication 2 et q et $R^{16}$ la signification donnée dans la revendication 1.

**6.** Composé de formule VII

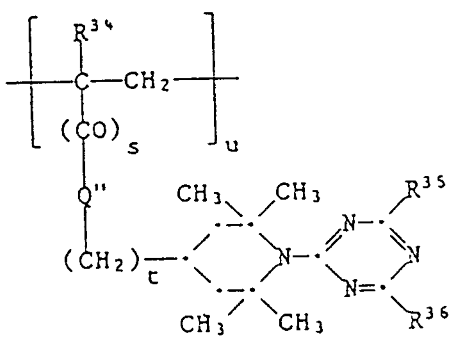

VII

où s vaut 0 ou 1, t vaut 0 ou 2 et u va de 5 à 100, Q'' représente -O-, -NH- ou -N(alkyle en $C_1$-$C_4$)-,
$R^{34}$ représente l'hydrogène ou le méthyle,
$R^{35}$ et $R^{36}$, indépendamment l'un de l'autre, représentent $-OR^3$, $-SR^3$ ou $-NR^4R^5$, où $R^3$, $R^4$ et $R^5$ ont les significations données dans la revendication 1, ainsi que des copolymères d'un tel composé avec les acide (méth)acrylique, (méth)acrylates d'alkyle, (méth)acrylates d'hydroxyalkyle ou anhydride maléique.

**7.** Composé de formule VIII :

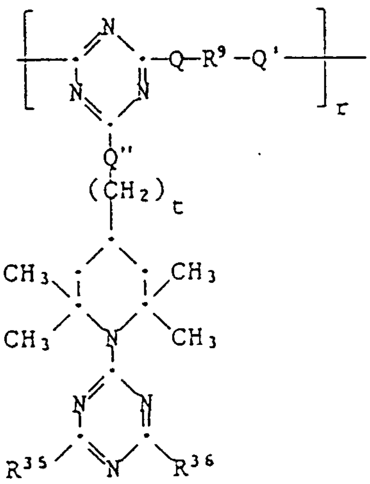

VIII

où r va de 3 à 50, t vaut 0 ou 2,
$R^9$, Q et Q' ont les significations données dans la revendication 3,
Q" représente -O-, -NH- ou -N(alkyle en $C_1$-$C_4$)- et
$R^{35}$ et $R^{36}$ ont les significations données dans la revendication 6.

**8.** Composé de formule IX

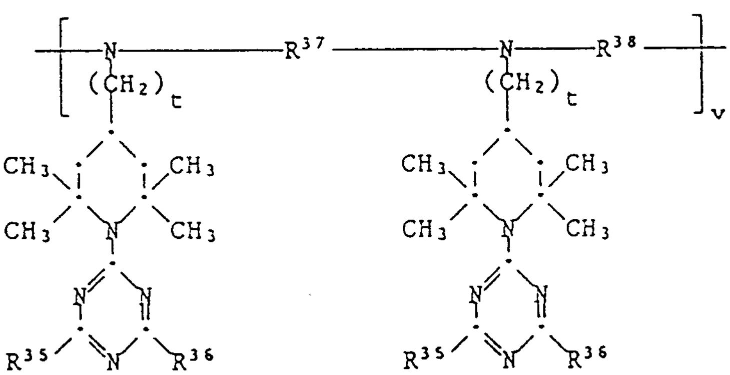

IX

où v représente une valeur de 2 à 30, t vaut 0 ou 2, $R^{35}$ et $R^{36}$ ont les significations données dans la revendication 6,
$R^{37}$ représente les alkylène en $C_2$-$C_8$, alcénylène en $C_4$-$C_8$, xylylène, $-CH_2-CH(OH)-CH_2-$ ou $-CH_2CH(OH)CH_2-O-R^{39}-O-CH_2CH(CH)CH_2-$,
$R^{38}$ a l'une des significations données pour $R^{37}$ ou représente

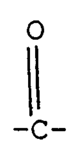

ou

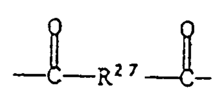

$R^{39}$ représente les alkylène en $C_2$-$C_8$, phénylène ou

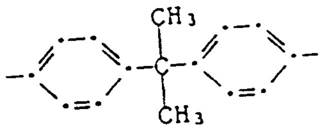

et $R^{27}$ a la signification donnée dans la revendication 2.

**9.** Composé de formule X

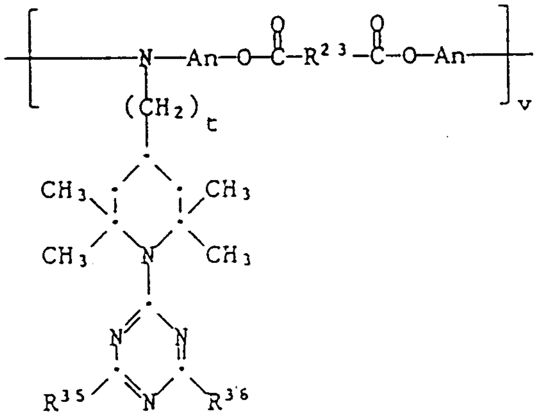

X

où t vaut 0 ou 2, v a une valeur de 2 à 30,
An représente un alkylène en $C_2$-$C_4$, $R^{35}$ et $R^{36}$ ont les significations données dans la revendication 6 et $R^{23}$ a la signification donnée dans la revendication 2.

**10.** Composé de formule XI

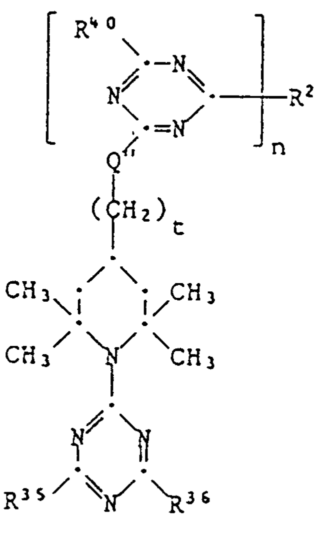

XI

où n représente un nombre entier de 1 à 6, t vaut 0 ou 2,
$R^2$ a la signification donnée dans la revendication 1, Q'',
$R^{35}$ et $R^{36}$ ont les significations données dans la revendication 6 et $R^{40}$ a soit l'une des significations données pour $R^1$ dans la revendication 1 soit représente un groupe

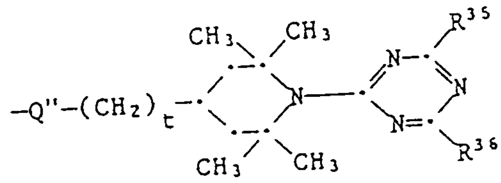

**11.** Composé de formule XII

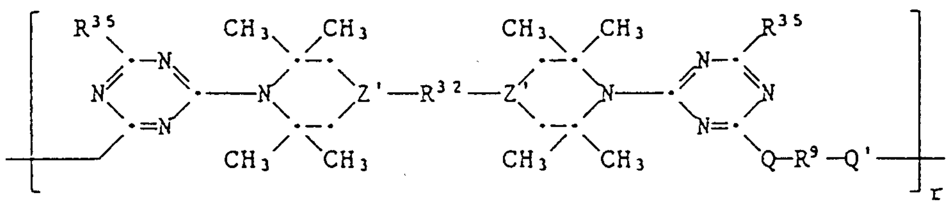

XII

où r représente une valeur de 3 à 50,
$R^9$ a la signification donnée dans la revendication 1, le groupe

$>Z'-R^{32}-Z'<$

a l'une des significations données dans la revendication 5, Q et Q', indépendamment l'un de l'autre, représentent -O-, -S- ou $-N(R^{10})-$, et $R^{35}$ a la signification donnée dans la revendication 6.

**12.** Composé selon la revendication 1 de formule II, où n représente un nombre entier de 1 à 4,
$R^1$ représente un radical Cl, $-OR^3$ ou $-NR^4R^5$, où
$R^3$, $R^R$ et $R^5$ ont les significations données dans la revendication 1,
$R^2$, lorsque n = 1, représente Cl, $-OR^3$ ou $-NR^4R^5$,
lorsque n = 2, représente un groupe $-N(R^{10})-R^9-N(R^{10})-$ ou

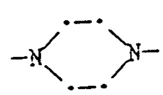

où $R^9$ et $R^{10}$ ont les significations données dans la revendication 1,
lorsque n = 3, un groupe

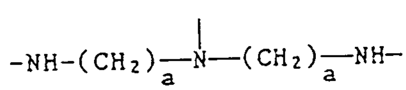

et lorsque n = 4, un groupe

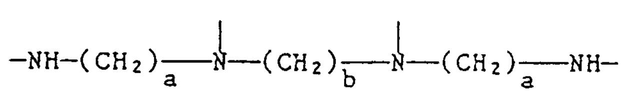

où a vaut 2 ou 3 et b va de 2 à 8 et
Y représente un groupe
$>CH-OR^{11}$, $>CH-NR^{12}R^{13}$, $>C=O$

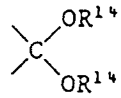

où $R^{11}$, $R^{12}$, $R^{13}$ et $R^{14}$ ont les significations données dans la revendication 1.

**13.** Composé selon la revendication 1 de formule II, où n vaut 1 ou 2, $R^1$ représente un radical de formule

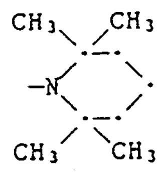

ou Cl, $-OR^3$ ou $-NR^4R^5$, $R^3$ représentant un alkyle en $C_1$-$C_{12}$, un allyle ou un groupe de formule A, $R^4$ représentant l'hydrogène, un alkyle en $C_1$-$C_8$, un 2-hydroxyalkyle ou un groupe de formule A,
$R^5$ représente un alkyle en $C_1$-$C_8$, un 2-hydroxyéthyle, un groupe de formule A ou un groupe de formule

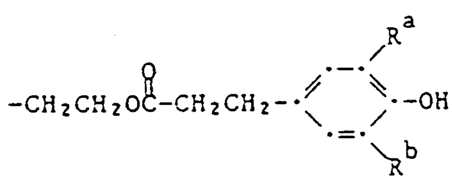

où $R^a$ et $R^b$ représentent un alkyle en $C_1$-$C_4$, ou $R^4$ et $R^5$ ensemble représentent le 3-oxapentaméthylène,
$R^2$ pour n = 1 représente Cl, $-OR^3$ ou $-NR^4R^5$, où $R^3$, $R^4$ et $R^5$ ont les significations données ci-dessus, et
pour n = 2, représente $-NH(CH_2)_6-NH-$,
Y représente un des groupes
$>CH_2$, $>CH—OR^{11}$, $>CH—NR^{12}R^{13}$, $>C=O$,

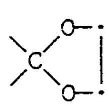 ou 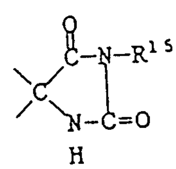

où $R^{11}$ représente l'hydrogène, les alkyle en $C_1$-$C_6$, acétyle, acryloyle, benzyle ou un groupe de formule

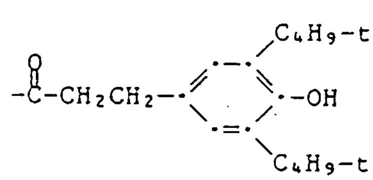

$R^{12}$ représente l'hydrogène ou un alkyle en $C_1$-$C_4$,
$R^{13}$ représente un alkyle en $C_1$-$C_4$ ou un radical de formule B, dans laquelle
$R^{19}$ et $R^{20}$ représentent Cl ou $-NR^4R^5$ et $R^{15}$ représente un alkyle en $C_1$-$C_{12}$.

**14.** Composé selon la revendication 2 de formule III, où p vaut 2, 3 ou 4, $R^{19}$ et $R^{20}$, indépendamment l'un de l'autre, représentent $-OR^3$ ou $-NR^4R^5$, où $R^3$, $R^4$ et $R^5$ ont les significations données dans la revendication 1,

Z, lorsque p = 2, représente un des groupes

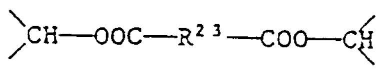 , 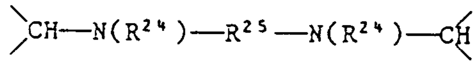 ,

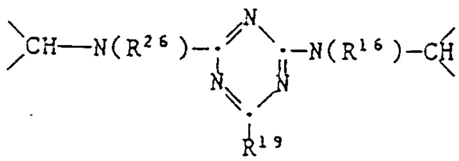 , 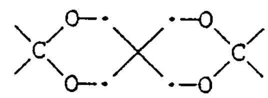 ,

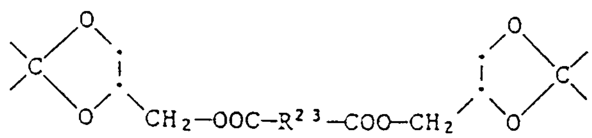

où $R^{23}$, $R^{24}$, $R^{25}$ et $R^{26}$ ont les significations données dans la revendication 2,

lorsque p = 3, un des groupes

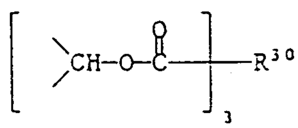 ou 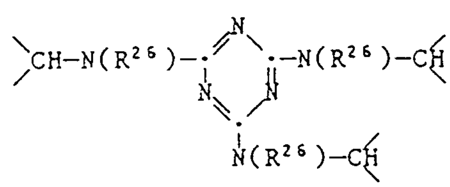

où $R^{30}$ représente les alcanetriyle en $C_3$-$C_8$ ou arènetriyle en $C_6$-$C_{12}$ et $R^{26}$ a la signification donnée dans la revendication 2, et

lorsque p = 4, un groupe

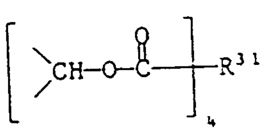

où $R^{31}$ représente les alcanetétrayle en $C_4$-$C_{12}$ ou arènetétrayle en $C_6$-$C_{12}$.

**15.** Composé selon la revendication 2 de formule III, où p vaut 2, $R^{19}$ et $R^{20}$ représentent Cl ou $-NR^4R^5$, où $R^4$ et $R^5$ représentent un alkyle en $C_1$-$C_4$ et Z un des groupes

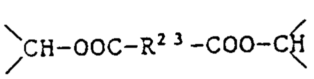 , 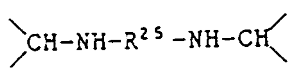 ou 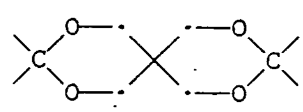

où $R^{23}$ représente les alkylène en $C_2$-$C_8$ ou phénylène et $R^{25}$ représente $-(CH_2)_6-$.

**16.** Composé selon la revendication 3 de formule IV, où r représente une valeur de 3 à 25, Q et Q' représentent -O- ou $-N(R^{10})-$ et Y, $R^9$ et $R^{10}$ ont les significations données dans la revendication 1.

**17.** Composé selon la revendication 3 de formule IV, où r représente une valeur de 3 à 50, Y un groupe $>CH_2$, $>C=O$,

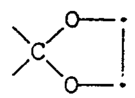

ou $>CH$-O(alkyle en $C_1$-$C_8$),
où Q et Q' représentent -NH- et $R^9$ représente $-(CH_2)_6-$.

**18.** Composé selon la revendication 4 de formule V, où r représente une valeur de 3 à 50, Y un groupe de formule

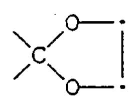

An représente $-CH_2CH_2-$ et $R^{23}$ représente un alkylène en $C_2$-$C_8$.

**19.** Matériau organique contenant en tant que stabilisant au moins un composé de formule II de la revendication 1, de formule III de la revendication 2, de formule IV de la revendication 3, de formule V de la revendication 4, de formule VI de la revendication 5, de formule VII de la revendication 6, de formule VIII de la revendication 7, de formule IX de la revendication 8, de formule X de la revendication 9, de formule XI de la revendication 10 ou de formule XII de la revendication 11, à l'exception de la 2-(2,2,6,6-tétraméthyl-1-pipéridino)-4,6-bis(2,4,4-triméthyl-2-pentylamino)-triazine et de la 2,4-dichloro-6-(2,2,6,6-tétraméthyl-1-pipéridino)-triazine.

**20.** Matériau organique selon la revendication 19, qui est un polymère organique.

**21.** Matériau organique selon la revendication 19, qui est un enduit.

**22.** Matériau organique selon la revendication 19, qui est une couche photographique.

**23.** Couche photographique selon la revendication 22, contenant de plus un phénol à empêchement stérique.

**24.** Utilisation d'un composé selon l'une des revendications 1 à 11, en tant que stabilisant pour matériaux organiques contre la dégradation induite par la lumière, par l'oxygène et par la chaleur.

**25.** Utilisation selon la revendication 24, d'un composé de formule

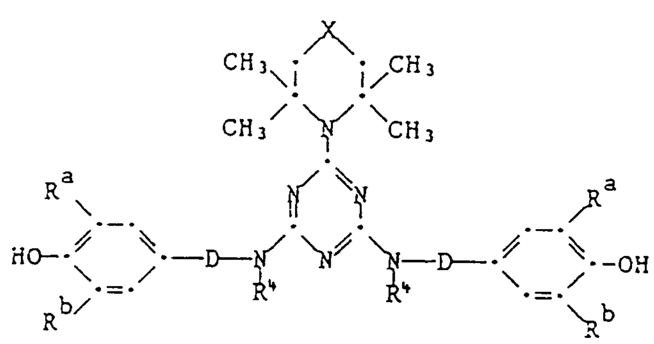

où X représente un groupe qui complète le cycle en un cycle de pipéridine,

D est un radical alkylène en $C_2$-$C_{20}$ interrompu par

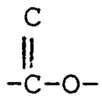

$R^a$ et $R^b$ représentent les alkyle en $C_1$-$C_{12}$, cycloalkyle en $C_5$-$C_6$, aryle en $C_6$-$C_{10}$ ou phénylalkyle en $C_7$-$C_9$ et $R^a$ représente aussi l'hydrogène, en tant que stabilisant pour les colorants photographiques.